(19) Europäisches Patentamt
European Patent Office
Office européen des brevets

(11) EP 2 230 319 B1

(12) **EUROPEAN PATENT SPECIFICATION**

(45) Date of publication and mention of the grant of the patent:
**21.10.2015 Bulletin 2015/43**

(51) Int Cl.:
***C12Q 1/68*** *(2006.01)*

(21) Application number: **10162868.3**

(22) Date of filing: **14.01.2004**

(54) **Gene expression markers for breast cancer prognosis**

Genexpressionsmarker für Brustkrebsprognose

Marqueurs d'expression génique pour pronostiquer le cancer du sein

(84) Designated Contracting States:
**AT BE BG CH CY CZ DE DK EE ES FI FR GB GR HU IE IT LI LU MC NL PT RO SE SI SK TR**

(30) Priority: **15.01.2003 US 440861 P**

(43) Date of publication of application:
**22.09.2010 Bulletin 2010/38**

(62) Document number(s) of the earlier application(s) in accordance with Art. 76 EPC:
**04702177.9 / 1 587 957**

(73) Proprietor: **Genomic Health, Inc.**
**Redwood City, CA 94063 (US)**

(72) Inventors:
- **Cobleigh, Melody A.**
  **Riverside, IL 60546 (US)**
- **Shak, Steven**
  **Hillsborough, CA 94010 (US)**
- **Baker, Joffre B.**
  **Montara, CA 94937 (US)**
- **Cronin, Maureen T.**
  **Los Altos, CA 94024 (US)**

(74) Representative: **Harding, Andrew Philip**
**Forresters**
**Sherborne House**
**119-121 Cannon Street**
**London EC4N 5AT (GB)**

(56) References cited:
**WO-A-02/10436** **WO-A-02/46467**
**US-B2- 7 056 674**

- **VEER VAN 'T L J ET AL: "Gene expression profiling predicts clinical outcome of breast cancer" NATURE, MACMILLAN JOURNALS LTD. LONDON, GB, vol. 415, no. 6871, 31 January 2002 (2002-01-31), pages 530-536, XP002259781 ISSN: 0028-0836**
- **SENS MARY ANN ET AL: "Metallothionein isoform 3 overexpression is associated with breast cancers having a poor prognosis" AMERICAN JOURNAL OF PATHOLOGY, vol. 159, no. 1, July 2001 (2001-07), pages 21-26, XP002285193 ISSN: 0002-9440**
- **RASCHELLA GIUSEPPE ET AL: "Expression of B-myb in neuroblastoma tumors is a poor prognostic factor independent from MYCN amplification" CANCER RESEARCH, vol. 59, no. 14, 15 July 1999 (1999-07-15), pages 3365-3368, XP002304932 ISSN: 0008-5472**
- **FOROZAN FARAHNAZ ET AL: "Comparative genomic hybridization analysis of 38 breast cancer cell lines: A basis for interpreting complementary DNA microarray data" CANCER RESEARCH, vol. 60, no. 16, 15 August 2000 (2000-08-15), pages 4519-4525, XP002304931 ISSN: 0008-5472**
- **PAIK SOONMYUNG ET AL: "A multigene assay to predict recurrence of tamoxifen-treated, node-negative breast cancer.", THE NEW ENGLAND JOURNAL OF MEDICINE 30 DEC 2004, vol. 351, no. 27, 30 December 2004 (2004-12-30), pages 2817-2826, ISSN: 1533-4406**
- **HABEL LAUREL A ET AL: "A population-based study of tumor gene expression and risk of breast cancer death among lymph node-negative patients.", BREAST CANCER RESEARCH : BCR 2006, vol. 8, no. 3, 2006, page R25, ISSN: 1465-542X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 230 319 B1

- SORLIE T ET AL: "Gene expression patterns of breast carcinomas distinguish tumor subclasses with clinical implications.", PROCEEDINGS OF THE NATIONAL ACADEMY OF SCIENCES OF THE UNITED STATES OF AMERICA 11 SEP 2001, vol. 98, no. 19, 11 September 2001 (2001-09-11), pages 10869-10874, ISSN: 0027-8424
- PEROU C M ET AL: "Molecular portraits of human breast tumours.", NATURE 17 AUG 2000, vol. 406, no. 6797, 17 August 2000 (2000-08-17), pages 747-752, ISSN: 0028-0836
- WINTERS Z E ET AL: "Subcellular localisation of cyclin B, Cdc2 and p21(WAF1/CIP1) in breast cancer. association with prognosis.", EUROPEAN JOURNAL OF CANCER (OXFORD, ENGLAND : 1990) DEC 2001, vol. 37, no. 18, December 2001 (2001-12), pages 2405-2412, ISSN: 0959-8049
- KEYOMARSI K ET AL: "Redundant cyclin overexpression and gene amplification in breast cancer cells.", PROCEEDINGS OF THE NATIONAL ACADEMY OF SCIENCES OF THE UNITED STATES OF AMERICA 1 FEB 1993, vol. 90, no. 3, 1 February 1993 (1993-02-01), pages 1112-1116, ISSN: 0027-8424

SEQUENCE LISTING

<110> GENOMIC HEALTH, INC.
RUSH UNIVERSITY MEDICAL CENTER
COBLEIGH, Melody
SHAK, Steven
BAKER, Joffre
CRONIN, Maureen

<120> GENE EXPRESSION MARKERS FOR BREAST CANCER PROGNOSIS

<130> 39740-0008 PCT

<140> Unassigned

<141> 2004-01-14

<150> US 60/440,861
<151> 2003-01-15

<160> 440

<170> FastSEQ for Windows Version 4.0

<210> 1
<211> 81
<212> DNA
<213> Artificial Sequence

<220>
<223> Amplicon

<400> 1

```
gcggcgagtt tccgatttaa agctgagctg cgaggaaaat ggcggcggga ggatcaaaat 60
acttgctgga tggtggactc a                                           81
```

<210> 2
<211> 71
<212> DNA
<213> Artificial Sequence

<220>
<223> Amplicon

<400> 2

```
cgcttctatg gcgctgagat tgtgtcagcc ctggactacc tgcactcgga gaagaacgtg 60
gtgtaccggg a                                                      71
```

<210> 3
<211> 71
<212> DNA
<213> Artificial Sequence

<220>
<223> Amplicon

<400> 3

```
tcctgccacc cttcaaacct caggtcacgt ccgaggtcga cacaaggtac ttcgatgatg 60
aatttaccgc c                                                      71
```

<210> 4
<211> 69
<212> DNA
<213> Artificial Sequence

<220>
<223> Amplicon

<400> 4

ggacagcagg aatgtgtttc tccatacagg tcacggggag ccaatggttc agaaacaaat 60
cgagtgggt 69

<210> 5
<211> 82
<212> DNA
<213> Artificial Sequence

<220>
<223> Amplicon

<400> 5

tgtgagtgaa atgccttcta gtagtgaacc gtcctcggga gccgactatg actactcaga 60
agagtatgat aacgaaccac aa 82

<210> 6
<211> 66
<212> DNA
<213> Artificial Sequence

<220>
<223> amplicon

<400> 6

cagcagatgt ggatcagcaa gcaggagtat gacgagtccg gcccctccat cgtccaccgc 60
aaatgc 66

<210> 7
<211> 80
<212> DNA
<213> Artificial Sequence

<220>
<223> Amplicon

<400> 7

ggctcttgtg cgtactgtcc ttcgggctgg tgacagggaa gacatcactg agcctgccat 60
ctgtgctctt cgtcatctga 80

<210> 8
<211> 73
<212> DNA
<213> Artificial Sequence

<220>
<223> Amplicon

<400> 8

gggtcaggtg cctcgagatc gggcttgggc ccagagcatg ttccagatcc cagagtttga 60
gccgagtgag cag 73

<210> 9
<211> 81
<212> DNA
<213> Artificial sequence

<220>
<223> Amplicon

<400> 9

cgttgtcagc acttggaata caagatggtt gccgggtcat gttaattggg aaaaagaaca 60
gtccacagga agaggttgaa c 81

<210> 10
<211> 83
<212> DNA
<213> Artificial Sequence

<220>
<223> Amplicon

<400> 10

cctggagggt cctgtacaat ctcatcatgg gactcctgcc cttacccagg ggccacagag 60
cccccgagat ggagcccaat tag 83

<210> 11
<211> 73
<212> DNA
<213> Artificial Sequence

<220>
<223> Amplicon

<400> 11

cagatggacc tagtacccac tgagatttcc acgccgaagg acagcgatgg gaaaaatgcc 60
cttaaatcat agg 73

<210> 12
<211> 72
<212> DNA
<213> Artificial Sequence

<220>
<223> Amplicon

<400> 12

```
atcctagccc tggtttttgg cctccttttt gctgtcacca gcgtcgcgtt ccttgtgcag 60
atgagaaggc ag                                                     72
```

<210> 13
<211> 84
<212> DNA
<213> Artificial Sequence

<220>
<223> Amplicon

<400> 13

```
ttcaggttgt tgcaggagac catgtacatg actgtctcca ttattgatcg gttcatgcag 60
aataattgtg tgcccaagaa gatg                                        84
```

<210> 14
<211> 69
<212> DNA
<213> Artificial sequence

<220>
<223> Amplicon

<400> 14

```
gcatgttcgt ggcctctaag atgaaggaga ccatccccct gacggccgag aagctgtgca 60
tctacaccg                                                         69
```

<210> 15
<211> 71
<212> DNA
<213> Artificial Sequence

<220>
<223> Amplicon

<400> 15

```
aaagaagatg atgaccgggt ttacccaaac tcaacgtgca agcctcggat tattgcacca 60
tccagaggct c                                                      71
```

<210> 16
<211> 82
<212> DNA
<213> Artificial Sequence

<220>
<223> Amplicon

<400> 16

atgctgtggc tccttcctaa ctggggcttt cttgacatgt aggttgcttg gtaataacct 60
ttttgtatat cacaatttgg gt 82

<210> 17
<211> 65
<212> DNA
<213> Artificial Sequence

<220>
<223> Amplicon

<400> 17

agatgaagtg gaaggcgctt ttcaccgcgg ccatcctgca ggcacagttg ccgattacag 60
aggca 65

<210> 18
<211> 74
<212> DNA
<213> Artificial Sequence

<220>
<223> Amplicon

<400> 18

tggttcccag ccctgtgtcc acctccaagc ccagattcag attcgagtca tgtacacaac 60
ccagggtgga ggag 74

<210> 19
<211> 64
<212> DNA
<213> Artificial Sequence

<220>
<223> Amplicon

<400> 19

gggcgtggaa cagtttatct cagacatctg ccccaagaag gacgtactcg aaaccttcac 60
cgtg 64

<210> 20
<211> 81
<212> DNA
<213> Artificial Sequence

<220>
<223> Amplicon

<400> 20

tgagtgtccc ccggtatctt ccccgccctg ccaatcccga tgaaattgga aattttattg 60
atgaaaatct gaaagcggct g 81

<210> 21
<211> 77
<212> DNA
<213> Artificial Sequence

<220>
<223> Amplicon

<400> 21

tgacaatcag cacacctgca ttcaccgctc ggaagagggc ctgagctgca tgaataagga 60
tcacggctgt agtcaca 77

<210> 22
<211> 82
<212> DNA
<213> Artificial Sequence

<220>
<223> Amplicon

<400> 22

gataaattgg tacaagggat cagcttttcc cagcccacat gtcctgatca tatgcttttg 60
aatagtcagt tacttggcac cc 82

<210> 23
<211> 72
<212> DNA
<213> Artificial sequence

<220>
<223> Amplicon

<400> 23

tgcctgtggt gggaagctca gtaactggga accaaaggat gatgctatgt cagaacaccg 60
gaggcatttt cc 72

<210> 24
<211> 86
<212> DNA
<213> Artificial Sequence

<220>
<223> Amplicon

<400> 24

ggatatttcc gtggctctta ttcaaactct ccatcaaatc ctgtaaactc cagagcaaat 60
caagattttt ctgccttgat gagaag 86

<210> 25
<211> 86
<212> DNA
<213> Artificial Sequence

<220>
<223> Amplicon

<400> 25

gacatttcca gtcctgcagt caatgcctct ctgccccacc ctttgttcag tgtggctggt 60
gccacgacaa atgtgtgcga tcggag 86

<210> 26
<211> 75
<212> DNA
<213> Artificial Sequence

<220>
<223> Amplicon

<400> 26

ggcatcctgg cccaaagttt cccaaatcca ggcggctaga ggcccactgc ttcccaacta 60

ccagctgagg gggtc 75

<210> 27
<211> 79
<212> DNA
<213> Artificial Sequence

<220>
<223> Amplicon

<400> 27

tctgcagagt tggaagcact ctatggtgac atcgatgctg tggagctgta tcctgccctt 60
ctggtagaaa agcctcggc 79

<210> 28
<211> 74
<212> DNA
<213> Artificial sequence

<220>
<223> Amplicon

<400> 28

gggaggctta tctcactgag tgagcagaat ctggtagact gctctgggcc tcaaggcaat 60
gaaggctgca atgg 74

<210> 29
<211> 67
<212> DNA
<213> Artificial Sequence

<220>
<223> Amplicon

<400> 29

tgtctcactg agcgagcaga atctggtgga ctgttcgcgt cctcaaggca atcagggctg 60
caatggt 67

<210> 30
<211> 77
<212> DNA
<213> Artificial Sequence

<220>
<223> Amplicon

<400> 30

cgctgacatc atgaatgttc ctcgaccggc tggaggcgag tttggatatg acaaagacac 60
atcgttgctg aaagaga 77

<210> 31
<211> 73
<212> DNA
<213> Artificial Sequence

<220>
<223> Amplicon

<400> 31

cacaatggcg gctctgaaga gttggctgtc gcgcagcgta acttcattct tcaggtacag 60
acagtgtttg tgt 73

<210> 32
<211> 84
<212> DNA
<213> Artificial Sequence

<220>
<223> Amplicon

<400> 32

ctctgagaca gtgcttcgat gactttgcag acttggtgcc ctttgactcc tgggagccgc 60
tcatgaggaa gttgggcctc atgg 84

<210> 33
<211> 62
<212> DNA
<213> Artificial Sequence

<220>
<223> Amplicon

<400> 33

tgtcgatgga cttccagaac cacctgggca gctgccaaaa gtgtgatcca agctgtccca 60
at 62

<210> 34
<211> 82
<212> DNA
<213> Artificial sequence

<220>
<223> Amplicon

<400> 34

gatctaagat ggcgactgtc gaaccggaaa ccacccctac tcctaatccc ccgactacag 60
aagaggagaa aacggaatct aa 82

<210> 35
<211> 68
<212> DNA
<213> Artificial Sequence

<220>
<223> Amplicon

<400> 35

ggcagtgtca ctgagtcctt gaaatcctcc cctgccccgc gggtctctgg attgggacgc 60
acagtgca 68

<210> 36
<211> 75
<212> DNA
<213> Artificial sequence

<220>
<223> Amplicon

<400> 36

```
gggccctcca gaacaatgat gggctttatg atcctgactg cgatgagagc gggctcttta 60
aggccaagca gtgca                                                  75
```

<210> 37
<211> 76
<212> DNA
<213> Artificial Sequence

<220>
<223> Amplicon

<400> 37

```
accgtaggct ctgctctgaa tgactctcct gtgggtctgg ctgcctatat tctagagaag 60
ttttccacct ggacca                                                 76
```

<210> 38
<211> 81
<212> DNA
<213> Artificial Sequence

<220>
<223> Amplicon

<400> 38

```
cggttatgtc atgccagata cacacctcaa aggtactccc tcctcccggg aaggcaccct 60
ttcttcagtg ggtctcagtt c                                           81
```

<210> 39
<211> 68
<212> DNA
<213> Artificial Sequence

<220>
<223> Amplicon

<400> 39

```
cgtggtgccc ctctatgacc tgctgctgga gatgctggac gcccaccgcc tacatgcgcc 60
cactagcc                                                          68
```

<210> 40
<211> 90
<212> DNA
<213> Artificial Sequence

<220>
<223> Amplicon

<400> 40

ggctattcct cattttctct acaaagtggc ctcagtgaac atgaagaagg tagcctcctg 60
gaggagaatt tcggtgacag tctacaatcc 90

<210> 41
<211> 68
<212> DNA
<213> Artificial Sequence

<220>
<223> Amplicon

<400> 41

cggtagtcaa gtccggatca agggcaagga gacggaattc tacctgtgca tgaaccgcaa 60
aggcaagc 68

<210> 42
<211> 74
<212> DNA
<213> Artificial Sequence

<220>
<223> Amplicon

<400> 42

cacgggacat tcaccacatc gactactata aaaagacaac caacggccga ctgcctgtga 60
agtggatggc accc 74

<210> 43
<211> 67
<212> DNA
<213> Artificial Sequence

<220>
<223> Amplicon

<400> 43

ccagtggagc gcttccatga cctgcgtcct gatgaagtgg ccgatttgtt tcagacgacc 60
cagagag 67

<210> 44
<211> 75
<212> DNA
<213> Artificial sequence

<220>
<223> Amplicon

<400> 44

ttggtacctg tgggttagca tcaagttctc cccagggtag aattcaatca gagctccagt 60
ttgcatttgg atgtg 75

<210> 45
<211> 68
<212> DNA
<213> Artificial sequence

<220>
<223> Amplicon

<400> 45

tcagcagcaa gggcatcatg gaggaggatg aggcctgcgg gcgccagtac acgctcaaga 60
aaaccacc 68

<210> 46
<211> 74
<212> DNA
<213> Artificial Sequence

<220>
<223> Amplicon

<400> 46

attccaccca tggcaaattc catggcaccg tcaaggctga gaacgggaag cttgtcatca 60
atggaaatcc catc 74

<210> 47
<211> 75
<212> DNA
<213> Artificial Sequence

<220>
<223> Amplicon

<400> 47

caaaggagct cactgtggtg tctgtgttcc aaccactgaa tctggacccc atctgtgaat 60
aagccattct gactc 75

<210> 48
<211> 67
<212> DNA
<213> Artificial Sequence

<220>
<223> Amplicon

<400> 48

ccatctgcat ccatcttgtt tgggctcccc acccttgaga agtgcctcag ataataccct 60
ggtggcc 67

<210> 49
<211> 73
<212> DNA
<213> Aritificial sequence

<220>
<223> Amplicon

<400> 49

cgaaaagatg ctgaacagtg acaaatccaa ctgaccagaa gggaggagga agctcactgg 60
tggctgttcc tga 73

<210> 50
<211> 86
<212> DNA
<213> Artificial Sequence

<220>
<223> Amplicon

<400> 50

aagctatgag gaaaagaagt acacgatggg ggacgctcct gattatgaca gaagccagtg 60
gctgaatgaa aaattcaagc tgggcc 86

<210> 51
<211> 73
<212> DNA
<213> Artificial Sequence

<220>
<223> Amplicon

<400> 51

cccactcagt agccaagtca caatgtttgg aaaacagccc gtttacttga gcaagactga 60
taccacctgc gtg 73

<210> 52
<211> 70
<212> DNA
<213> Artificial sequence

<220>
<223> Amplicon

<400> 52

cggtgtgaga agtgcagcaa gccctgtgcc cgagtgtgct atggtctggg catggagcac 60
ttgcgagagg 70

<210> 53
<211> 82

<212> DNA
<213> Artificial sequence

<220>
<223> Amplicon

<400> 53

```
tgaacataaa gtctgcaaca tggaaggtat tgcactgcac aggccacatt cacgtatatg 60
ataccaacag taaccaacct ca                                          82
```

<210> 54
<211> 73
<212> DNA
<213> Artificial Sequence

<220>
<223> Amplicon

<400> 54

```
tccaggatgt taggaactgt gaagatggaa gggcatgaaa ccagcgactg gaacagctac 60
tacgcagaca cgc                                                    73
```

<210> 55
<211> 70
<212> DNA
<213> Artificial Sequence

<220>
<223> Amplicon

<400> 55

```
agaaccgcaa ggtgagcaag gtggagattc tccagcacgt catcgactac atcagggacc 60
ttcagttgga                                                        70
```

<210> 56
<211> 76
<212> DNA
<213> Artificial Sequence

<220>
<223> Amplicon

<400> 56

```
tccggagctg tgatctaagg aggctggaga tgtattgcgc acccctcaag cctgccaagt 60
cagctcgctc tgtccg                                                 76
```

<210> 57
<211> 83
<212> DNA
<213> Artificial Sequence

<220>
<223> Amplicon

<400> 57

gcatggtagc cgaagatttc acagtcaaaa tcggagattt tggtatgacg cgagatatct 60
atgagacaga ctattaccgg aaa 83

<210> 58
<211> 73
<212> DNA
<213> Artificial Sequence

<220>
<223> Amplicon

<400> 58

gtggacagca ccatgaacat gttgggcggg ggaggcagtg ctggccggaa gcccctcaag 60
tcgggtatga agg 73

<210> 59
<211> 72
<212> DNA
<213> Artificial Sequence

<220>
<223> Amplicon

<400> 59

cctgaacctt ccaaagatgg ctgaaaaaga tggatgcttc caatctggat tcaatgagga 60
gacttgcctg gt 72

<210> 60
<211> 74
<212> DNA
<213> Artificial sequence

<220>
<223> Amplicon

<400> 60

ccacagctca ccttctgtca ggtgtccatc ccagctccag ccagctccca gagaggaaga 60
gactggcact gagg 74

<210> 61
<211> 80
<212> DNA
<213> Artificial Sequence

<220>
<223> Amplicon

<400> 61

cggactttgg gtgcgacttg acgagcggtg gttcgacaag tggccttgcg ggccggatcg 60
tcccagtgga agagttgtaa 80

<210> 62
<211> 78
<212> DNA
<213> Artificial Sequence

<220>
<223> Amplicon

<400> 62

gcccagaggc tccatcgtcc atcctcttcc tccccagtcg gctgaactct ccccttgtct 60
gcactgttca aacctctg 78

<210> 63
<211> 83
<212> DNA
<213> Artificial Sequence

<220>
<223> Amplicon

<400> 63

ggcctgctga gatcaaagac tacagtccct acttcaagac cattgaggac ctgaggaaca 60
agattctcac agccacagtg gac 83

<210> 64
<211> 73
<212> DNA
<213> Artificial Sequence

<220>
<223> Amplicon

<400> 64

cgaggattgg ttcttcagca agacagagga actgaaccgc gaggtggcca ccaacagtga 60
gctggtgcag agt 73

<210> 65
<211> 68
<212> DNA
<213> Artificial Sequence

<220>
<223> Amplicon

<400> 65

agagatcgag gctctcaagg aggagctgct cttcatgaag aagaaccacg aagaggaagt 60
aaaaggcc 68

<210> 66
<211> 77
<212> DNA
<213> Artificial Sequence

<220>
<223> Amplicon

<400> 66

tgagcggcag aatcaggagt accagcggct catggacatc aagtcgcggc tggagcagga 60
gattgccacc taccgca 77

<210> 67
<211> 69
<212> DNA
<213> Artificial Sequence

<220>
<223> Amplicon

<400> 67

tcagtggaga aggagttgga ccagtcaaca tctctgttgt cacaagcagt gtttcctctg 60
gatatggca 69

<210> 68
<211> 86
<212> DNA
<213> Artificial sequence

<220>
<223> Amplicon

<400> 68

ggatgaagct tacatgaaca aggtagagct ggagtctcgc ctggaagggc tgaccgacga 60
gatcaacttc ctcaggcagc tatatg 86

<210> 69
<211> 83
<212> DNA
<213> Artificial Sequence

<220>
<223> Amplicon

<400> 69

ggaaagacca cctgaaaaac cacctccaga cccacgaccc caacaaaatg gcctttgggt 60
gtgaggagtg tgggaagaag tac 83

<210> 70
<211> 77
<212> DNA
<213> Artificial sequence

<220>
<223> Amplicon

<400> 70

cagatggcca ctttgagaac attttagctg acaacagtgt gaacgaccag accaaaatcc 60
ttgtggttaa tgctgcc 77

<210> 71
<211> 75
<212> DNA
<213> Artificial Sequence

<220>
<223> Amplicon

<400> 71

gacttttgcc cgctaccttt cattccggcg tgacaacaat gagctgttgc tcttcatact 60
gaagcagtta gtggc 75

<210> 72
<211> 75
<212> DNA
<213> Artificial Sequence

<220>
<223> Amplicon

<400> 72

ggagaacaat ccccttgaga cagaatatgg cctttctgtc tacaaggatc accagaccat 60
caccatccag gagat 75

<210> 73
<211> 82
<212> DNA
<213> Artificial Sequence

<220>
<223> Amplicon

<400> 73

tgatggtcct atgtgtcaca ttcatcacag gtttcatacc aacacaggct tcagcacttc 60
ctttggtgtg tttcctgtcc ca 82

<210> 74
<211> 68
<212> DNA
<213> Artificial sequence

<220>
<223> Amplicon

<400> 74

ctacagggac gccatcgaat ccggatcttg atgctggtgt aagtgaacat tcaggtgatt 60
ggttggat 68

<210> 75
<211> 67
<212> DNA
<213> Artificial Sequence

<220>
<223> Amplicon

<400> 75

gagaaccaat ctcaccgaca ggcagctggc agaggaatac ctgtaccgct atggttacac 60
tcgggtg 67

<210> 76
<211> 77
<212> DNA
<213> Artificial Sequence

<220>
<223> Amplicon

<400> 76

ccgccctcac ctgaagagaa acgcgctcct tggcggacac tgggggagga gaggaagaag 60
cgcggctaac ttattcc 77

<210> 77
<211> 74
<212> DNA
<213> Artificial Sequence

<220>
<223> Amplicon

<400> 77

gccgagatcg ccaagatgtt gccagggagg acagacaatg ctgtgaagaa tcactggaac 60
tctaccatca aaag 74

<210> 78
<211> 72
<212> DNA
<213> Artificial Sequence

<220>
<223> Amplicon

<400> 78

ccctcgtgct gatgctactg aggagccagc gtctagggca gcagccgctt cctagaagac 60
caggtcatga tg 72

<210> 79
<211> 66
<212> DNA
<213> Artificial Sequence

<220>
<223> Amplicon

<400> 79

cggtggacca cgaagagtta acccgggact tggagaagca ctgcagagac atggaagagg 60
cgagcc 66

<210> 80
<211> 68
<212> DNA
<213> Artificial Sequence

<220>
<223> Amplicon

<400> 80

ctttgaaccc ttgcttgcaa taggtgtgcg tcagaagcac ccaggacttc catttgcttt 60
gtcccggg 68

<210> 81
<211> 81
<212> DNA
<213> Artificial Sequence

<220>
<223> Amplicon

<400> 81

```
ccgcaacgtg gttttctcac cctatggggt ggcctcggtg ttggccatgc tccagctgac 60
aacaggagga gaaacccagc a                                           81
```

<210> 82
<211> 66
<212> DNA
<213> Artificial sequence

<220>
<223> Amplicon

<400> 82

```
ccagctctcc ttccagctac agatcaatgt ccctgtccga gtgctggagc taagtgagag 60
ccaccc                                                            66
```

<210> 83
<211> 83
<212> DNA
<213> Artificial Sequence

<220>
<223> Amplicon

<400> 83

```
ataccaatca ccgcacaaac ccaggctatt tgttaagtcc agtcacagcg caaagaaaca 60
tatgcggaga aaatgctagt gtg                                         83
```

<210> 84
<211> 62
<212> DNA
<213> Artificial Sequence

<220>
<223> Amplicon

<400> 84

```
gccatccgca aaggctttct cgcttgtcac cttgccatgt ggaagaaact ggcggaatgg 60
cc                                                                62
```

<210> 85
<211> 85
<212> DNA
<213> Artificial Sequence

<220>
<223> Amplicon

<400> 85

```
gcatcaggct gtcattatgg tgtccttacc tgtgggagct gtaaggtctt ctttaagagg 60
gcaatggaag ggcagcacaa ctact                                       85
```

<210> 86
<211> 66
<212> DNA
<213> Artificial Sequence

<220>
<223> Amplicon

<400> 86

```
tctccatatc tgccttgcag agtctcctgc agcacctcat cgggctgagc aatctgaccc 60
acgtgc                                                            66
```

<210> 87
<211> 86
<212> DNA
<213> Artificial sequence

<220>
<223> Amplicon

<400> 87

```
gccctcccag tgtgcaaata agggctgctg tttcgacgac accgttcgtg gggtcccctg 60
gtgcttctat cctaatacca tcgacg                                      86
```

<210> 88
<211> 78
<212> DNA
<213> Artificial Sequence

<220>
<223> Amplicon

<400> 88

```
gaacttcttg agcaggagca tacccagggc ttcataatca ccttctgttc agcactagat 60
gatattcttg ggggtgga                                               78
```

<210> 89
<211> 77
<212> DNA
<213> Artificial Sequence

<220>
<223> Amplicon

<400> 89

```
cgaagccctt acaagtttcc tagttcaccc ttacggattc ctggagggaa catctatatt 60
tcacccctga agagtcc                                                77
```

<210> 90
<211> 74

<212> DNA
<213> Artificial Sequence

<220>
<223> Amplicon

<400> 90

```
ccagacgagc gattagaagc ggcagcttgt gaggtgaatg atttggggga agaggaggag 60
gaggaagagg agga                                                   74
```

<210> 91
<211> 69
<212> DNA
<213> Artificial Sequence

<220>
<223> Amplicon

<400> 91

```
catcttccag gaggaccact ctctgtggca ccctggacta cctgccccct gaaatgattg 60
aaggtcgga                                                         69
```

<210> 92
<211> 90
<212> DNA
<213> Artificial Sequence

<220>
<223> Amplicon

<400> 92

```
cctggaggct gcaacatacc tcaatcctgt cccaggccgg atcctcctga agccctttc 60
gcagcactgc tatcctccaa agccattgta                                  90
```

<210> 93
<211> 80
<212> DNA
<213> Artificial Sequence

<220>
<223> Amplicon

<400> 93

```
tgttttgatt cccgggctta ccaggtgaga agtgagggag gaagaaggca gtgtcccttt 60

tgctagagct gacagctttg                                             80
```

<210> 94
<211> 65

<212> DNA
<213> Artificial Sequence

<220>
<223> Amplicon

<400> 94

```
gcccgaaacg ccgaatataa tcccaagcgg tttgctgcgg taatcatgag gataagagag 60
ccacg                                                             65
```

<210> 95
<211> 83
<212> DNA
<213> Artificial Sequence

<220>
<223> Amplicon

<400> 95

```
ggtgtgccac agaccttcct acttggcctg taatcacctg tgcagccttt tgtgggcctt 60
caaaactctg tcaagaactc cgt                                         83
```

<210> 96
<211> 75
<212> DNA
<213> Artificial sequence

<220>
<223> Amplicon

<400> 96

```
tccctgcggt cccagatagc ctgaatcctg cccggagtgg aactgaagcc tgcacagtgt 60
ccaccctgtt cccac                                                  75
```

<210> 97
<211> 72
<212> DNA
<213> Artificial Sequence

<220>
<223> Amplicon

<400> 97

```
aatccaaggg ggagagtgat gacttccata tggactttga ctcagctgtg gctcctcggg 60
caaaatctgt ac                                                     72
```

<210> 98
<211> 66
<212> DNA
<213> Artificial Sequence

<220>
<223> Amplicon

<400> 98

tgtggacatc ttcccctcag acttccctac tgagccacct tctctgccac gaaccggtcg 60
ggctag 66

<210> 99
<211> 82
<212> DNA
<213> Artificial Sequence

<220>
<223> Amplicon

<400> 99

ctatatgcag ccagagatgt gacagccacc gtggacagcc tgccactcat cacagcctcc 60
attctcagta agaaactcgt gg 82

<210> 100
<211> 81
<212> DNA
<213> Artificial Sequence

<220>
<223> Amplicon

<400> 100

gggccaaata ttcagaagct tttatatcag aggaccacca tagcggccat ggagaccatc 60
tctgtcccat catacccatc c 81

<210> 101
<211> 73
<212> DNA
<213> Artificial Sequence

<220>
<223> Amplicon

<400> 101

cttcacagtg ctcctgcagt ctctctgtgt ggctgtaact tacgtgtact ttaccaacga 60
gctgaagcag atg 73

<210> 102
<211> 65
<212> DNA
<213> Artificial Sequence

<220>
<223> Amplicon

<400> 102

gcctcggtgt gcctttcaac atcgccagct acgccctgct cacgtacatg attgcgcaca 60
tcacg 65

<210> 103
<211> 70
<212> DNA
<213> Artificial Sequence

<220>
<223> Amplicon

<400> 103

gtggatgtgc cctgaaggac aagccaggcg tctacacgag agtctcacac ttcttaccct 60
ggatccgcag 70

<210> 104
<211> 67
<212> DNA
<213> Artificial Sequence

<220>
<223> Amplicon

<400> 104

gccctggatt tcagaaagag ccaagtctgg atctgggacc ctttccttcc ttccctggct 60
tgtaact 67

<210> 105
<211> 71
<212> DNA
<213> Artificial Sequence

<220>
<223> Amplicon

<400> 105

ctgctgtctt gggtgcattg gagccttgcc ttgctgctct acctccacca tgccaagtgg 60
tcccaggctg c 71

<210> 106
<211> 71
<212> DNA
<213> Artificial sequence

<220>
<223> Amplicon

<400> 106

tgacgatggc ctggagtgtg tgcccactgg gcagcaccaa gtccggatgc agatcctcat 60
gatccggtac c 71

<210> 107
<211> 75
<212> DNA
<213> Artificial Sequence

<220>
<223> Amplicon

<400> 107

agaggcatcc atgaacttca cacttgcggg ctgcatcagc acacgctcct atcaacccaa 60
gtactgtgga gtttg 75

<210> 108
<211> 77
<212> DNA
<213> Artificial Sequence

<220>
<223> Amplicon

<400> 108

gcagttggaa gacacaggaa agtatcccca aattgcagat ttatcaacgg cttttatctt 60
gaaaatagtg ccacgca 77

<210> 109
<211> 76
<212> DNA
<213> Artificial Sequence

<220>
<223> Amplicon

<400> 109

agactgtgga gtttgatgtt gttgaaggag aaaagggtgc ggaggcagca aatgttacag 60
gtcctggtgg tgttcc 76

<210> 110
<211> 70
<212> DNA
<213> Artificial Sequence

<220>
<223> Amplicon

<400> 110

acccagtagc aaggagaagc ccactcactg ctccgagtgc ggcaaagctt tcagaaccta 60
ccaccagctg 70

<210> 111
<211> 19
<212> DNA
<213> Artificial Sequence

<220>
<223> forward primer

<400> 111
gcggcgagtt tccgattta 19

<210> 112
<211> 21
<212> DNA
<213> Artificial Sequence

<220>
<223> reverse primer

<400> 112
tgagtccacc atccagcaag t 21

<210> 113
<211> 21
<212> DNA
<213> Artificial sequence

<220>
<223> probe

<400> 113
atggcggcgg gaggatcaaa a 21

<210> 114
<211> 20
<212> DNA
<213> Artificial Sequence

<220>
<223> forward primer

<400> 114
cgcttctatg gcgctgagat 20

<210> 115
<211> 20
<212> DNA
<213> Artificial Sequence

<220>
<223> reverse primer

<400> 115
tcccggtaca ccacgttctt 20

<210> 116
<211> 24
<212> DNA
<213> Artificial Sequence

<220>
<223> probe

<400> 116
cagccctgga ctacctgcac tcgg 24

<210> 117
<211> 19
<212> DNA
<213> Artificial sequence

<220>
<223> forward primer

<400> 117
tcctgccacc cttcaaacc 19

<210> 118
<211> 21
<212> DNA
<213> Artificial Sequence

<220>
<223> reverse primer

<400> 118
ggcggtaaat tcatcatcga a 21

<210> 119
<211> 24
<212> DNA
<213> Artificial Sequence

<220>
<223> probe

<400> 119
caggtcacgt ccgaggtcga caca 24

<210> 120
<211> 20
<212> DNA
<213> Artificial Sequence

<220>
<223> forward primer

<400> 120
ggacagcagg aatgtgtttc 20

<210> 121
<211> 20
<212> DNA

<213> Artificial Sequence

<220>
<223> reverse primer

<400> 121
acccactcga tttgtttctg 20

<210> 122
<211> 22
<212> DNA
<213> Artificial Sequence

<220>
<223> probe

<400> 122
cattggctcc ccgtgacctg ta 22

<210> 123
<211> 27
<212> DNA
<213> Artificial Sequence

<220>
<223> forward primer

<400> 123
tgtgagtgaa atgccttcta gtagtga 27

<210> 124
<211> 23
<212> DNA
<213> Artificial Sequence

<220>
<223> probe

<400> 124
ccgtcctcgg gagccgacta tga 23

<210> 125
<211> 27
<212> DNA
<213> Artificial Sequence

<220>
<223> reverse primer

<400> 125
ttgtggttcg ttatcatact cttctga 27

<210> 126
<211> 21
<212> DNA
<213> Artificial Sequence

<220>

<223> forward primer

<400> 126
cagcagatgt ggatcagcaa g 21

<210> 127
<211> 18
<212> DNA
<213> Artificial Sequence

<220>
<223> reverse primer

<400> 127
gcatttgcgg tggacgat 18

<210> 128
<211> 23
<212> DNA
<213> Artificial Sequence

<220>
<223> probe

<400> 128
aggagtatga cgagtccggc ccc 23

<210> 129
<211> 22
<212> DNA
<213> Artificial Sequence

<220>
<223> forward primer

<400> 129
ggctcttgtg cgtactgtcc tt 22

<210> 130
<211> 23
<212> DNA
<213> Artificial Sequence

<220>
<223> reverse primer

<400> 130
tcagatgacg aagagcacag atg 23

<210> 131
<211> 29
<212> DNA
<213> Artificial Sequence

<220>
<223> probe

<400> 131

aggctcagtg atgtcttccc tgtcaccag 29

<210> 132
<211> 19
<212> DNA
<213> Artificial Sequence

<220>
<223> forward primer

<400> 132
gggtcaggtg cctcgagat 19

<210> 133
<211> 21
<212> DNA
<213> Artificial Sequence

<220>
<223> reverse primer

<400> 133
ctgctcactc ggctcaaact c 21

<210> 134
<211> 24
<212> DNA
<213> Artificial Sequence

<220>
<223> probe

<400> 134
tgggcccaga gcatgttcca gatc 24

<210> 135
<211> 23
<212> DNA
<213> Artificial sequence

<220>
<223> forward primer

<400> 135
cgttgtcagc acttggaata caa 23

<210> 136
<211> 24
<212> DNA
<213> Artificial Sequence

<220>
<223> reverse primer

<400> 136
gttcaacctc ttcctgtgga ctgt 24

<210> 137

<211> 26
<212> DNA
<213> Artificial Sequence

<220>
<223> probe

<400> 137
cccaattaac atgacccggc aaccat 26

<210> 138
<211> 20
<212> DNA
<213> Artificial Sequence

<220>
<223> forward primer

<400> 138
cctggagggt cctgtacaat 20

<210> 139
<211> 19
<212> DNA
<213> Artificial sequence

<220>
<223> reverse primer

<400> 139
ctaattgggc tccatctcg 19

<210> 140
<211> 24
<212> DNA
<213> Artificial Sequence

<220>
<223> probe

<400> 140
catcatggga ctcctgccct tacc 24

<210> 141
<211> 25
<212> DNA
<213> Artificial Sequence

<220>
<223> forward primer

<400> 141
cagatggacc tagtacccac tgaga 25

<210> 142
<211> 22
<212> DNA
<213> Artificial Sequence

<220>
<223> probe

<400> 142
ttccacgccg aaggacagcg at 22

<210> 143
<211> 24
<212> DNA
<213> Artificial Sequence

<220>
<223> reverse primer

<400> 143
cctatgattt aagggcattt ttcc 24

<210> 144
<211> 20
<212> DNA
<213> Artificial Sequence

<220>
<223> forward primer

<400> 144
atcctagccc tggtttttgg 20

<210> 145
<211> 20
<212> DNA
<213> Artificial sequence

<220>
<223> reverse primer

<400> 145
ctgccttctc atctgcacaa 20

<210> 146
<211> 20
<212> DNA
<213> Artificial Sequence

<220>
<223> probe

<400> 146
tttgctgtca ccagcgtcgc 20

<210> 147
<211> 20
<212> DNA
<213> Artificial Sequence

<220>
<223> forward primer

<400> 147
ttcaggttgt tgcaggagac 20

<210> 148
<211> 20
<212> DNA
<213> Artificial Sequence

<220>
<223> reverse primer

<400> 148
catcttcttg ggcacacaat 20

<210> 149
<211> 27
<212> DNA
<213> Artificial Sequence

<220>
<223> probe

<400> 149
tgtctccatt attgatcggt tcatgca 27

<210> 150
<211> 21
<212> DNA
<213> Artificial Sequence

<220>
<223> forward primer

<400> 150
gcatgttcgt ggcctctaag a 21

<210> 151
<211> 22
<212> DNA
<213> Artificial Sequence

<220>
<223> reverse primer

<400> 151
cggtgtagat gcacagcttc tc 22

<210> 152
<211> 23
<212> DNA
<213> Artificial Sequence

<220>
<223> probe

<400> 152
aaggagacca tccccctgac ggc 23

<210> 153
<211> 24
<212> DNA
<213> Artificial sequence

<220>
<223> forward primer

<400> 153
aaagaagatg atgaccgggt ttac 24

<210> 154
<211> 20
<212> DNA
<213> Artificial sequence

<220>
<223> reverse primer

<400> 154
gagcctctgg atggtgcaat 20

<210> 155
<211> 24
<212> DNA
<213> Artificial Sequence

<220>
<223> probe

<400> 155
caaactcaac gtgcaagcct cgga 24

<210> 156
<211> 22
<212> DNA
<213> Artificial Sequence

<220>
<223> forward primer

<400> 156
atgctgtggc tccttcctaa ct 22

<210> 157
<211> 27
<212> DNA
<213> Artificial Sequence

<220>
<223> reverse primer

<400> 157
acccaaattg tgatatacaa aaaggtt 27

<210> 158
<211> 30
<212> DNA

<213> Artificial Sequence

<220>
<223> probe

<400> 158
taccaagcaa cctacatgtc aagaaagccc 30

<210> 159
<211> 20
<212> DNA
<213> Artificial Sequence

<220>
<223> forward primer

<400> 159
agatgaagtg gaaggcgctt 20

<210> 160
<211> 18
<212> DNA
<213> Artificial Sequence

<220>
<223> probe

<400> 160
caccgcggcc atcctgca 18

<210> 161
<211> 21
<212> DNA
<213> Artificial sequence

<220>
<223> reverse primer

<400> 161
tgcctctgta atcggcaact g 21

<210> 162
<211> 18
<212> DNA
<213> Artificial Sequence

<220>
<223> forward primer

<400> 162
tggttcccag ccctgtgt 18

<210> 163
<211> 28
<212> DNA
<213> Artificial Sequence

<220>

<223> probe

<400> 163
ctccaagccc agattcagat tcgagtca 28

<210> 164
<211> 19
<212> DNA
<213> Artificial Sequence

<220>
<223> reverse primer

<400> 164
ctcctccacc ctgggttgt 19

<210> 165
<211> 20
<212> DNA
<213> Artificial Sequence

<220>
<223> forward primer

<400> 165
gggcgtggaa cagtttatct 20

<210> 166
<211> 19
<212> DNA
<213> Artificial sequence

<220>
<223> reverse primer

<400> 166
cacggtgaag gtttcgagt 19

<210> 167
<211> 24
<212> DNA
<213> Artificial Sequence

<220>
<223> probe

<400> 167
agacatctgc cccaagaagg acgt 24

<210> 168
<211> 21
<212> DNA
<213> Artificial Sequence

<220>
<223> forward primer

<400> 168

tgagtgtccc ccggtatctt c 21

<210> 169
<211> 21
<212> DNA
<213> Artificial Sequence

<220>
<223> reverse primer

<400> 169
cagccgcttt cagattttca t 21

<210> 170
<211> 27
<212> DNA
<213> Artificial Sequence

<220>
<223> probe

<400> 170
tgccaatccc gatgaaattg gaaattt 27

<210> 171
<211> 21
<212> DNA
<213> Artificial Sequence

<220>
<223> forward primer

<400> 171
tgacaatcag cacacctgca t 21

<210> 172
<211> 23
<212> DNA
<213> Artificial sequence

<220>
<223> reverse primer

<400> 172
tgtgactaca gccgtgatcc tta 23

<210> 173
<211> 20
<212> DNA
<213> Artificial Sequence

<220>
<223> probe

<400> 173
caggccctct tccgagcggt 20

<210> 174

<211> 26
<212> DNA
<213> Artificial Sequence

<220>
<223> forward primer

<400> 174
gataaattgg tacaagggat cagctt 26

<210> 175
<211> 24
<212> DNA
<213> Artificial Sequence

<220>
<223> reverse primer

<400> 175
gggtgccaag taactgacta ttca 24

<210> 176
<211> 26
<212> DNA
<213> Artificial sequence

<220>
<223> probe

<400> 176
ccagcccaca tgtcctgatc atatgc 26

<210> 177
<211> 18
<212> DNA
<213> Artificial Sequence

<220>
<223> forward primer

<400> 177
tgcctgtggt gggaagct 18

<210> 178
<211> 19
<212> DNA
<213> Artificial Sequence

<220>
<223> reverse primer

<400> 178
ggaaaatgcc tccggtgtt 19

<210> 179
<211> 30
<212> DNA
<213> Artificial sequence

<220>
<223> probe

<400> 179
tgacatagca tcatcctttg gttcccagtt 30

<210> 180
<211> 24
<212> DNA
<213> Artificial Sequence

<220>
<223> forward primer

<400> 180
ggatatttcc gtggctctta ttca 24

<210> 181
<211> 30
<212> DNA
<213> Artificial sequence

<220>
<223> probe

<400> 181
tctccatcaa atcctgtaaa ctccagagca 30

<210> 182
<211> 25
<212> DNA
<213> Artificial Sequence

<220>
<223> reverse primer

<400> 182
cttctcatca aggcagaaaa atctt 25

<210> 183
<211> 22
<212> DNA
<213> Artificial Sequence

<220>
<223> forward primer

<400> 183
gacatttcca gtcctgcagt ca 22

<210> 184
<211> 23
<212> DNA
<213> Artificial Sequence

<220>
<223> probe

<400> 184
tgcctctctg ccccaccctt tgt 23

<210> 185
<211> 20
<212> DNA
<213> Artificial Sequence

<220>
<223> reverse primer

<400> 185
ctccgatcgc acacatttgt 20

<210> 186
<211> 18
<212> DNA
<213> Artificial Sequence

<220>
<223> forward primer

<400> 186
ggcatcctgg cccaaagt 18

<210> 187
<211> 21
<212> DNA
<213> Artificial Sequence

<220>
<223> reverse primer

<400> 187
gaccccctca gctggtagtt g 21

<210> 188
<211> 23
<212> DNA
<213> Artificial Sequence

<220>
<223> probe

<400> 188
cccaaatcca ggcggctaga ggc 23

<210> 189
<211> 23
<212> DNA
<213> Artificial Sequence

<220>
<223> forward primer

<400> 189
tctgcagagt tggaagcact cta 23

<210> 190
<211> 28
<212> DNA
<213> Artificial sequence

<220>
<223> probe

<400> 190
caggatacag ctccacagca tcgatgtc 28

<210> 191
<211> 21
<212> DNA
<213> Artificial Sequence

<220>
<223> reverse primer

<400> 191
gccgaggctt ttctaccaga a 21

<210> 192
<211> 23
<212> DNA
<213> Artificial Sequence

<220>
<223> forward primer

<400> 192
gggaggctta tctcactgag tga 23

<210> 193
<211> 19
<212> DNA
<213> Artificial Sequence

<220>
<223> reverse primer

<400> 193
ccattgcagc cttcattgc 19

<210> 194
<211> 29
<212> DNA
<213> Artificial Sequence

<220>
<223> probe

<400> 194
ttgaggccca gagcagtcta ccagattct 29

<210> 195
<211> 21
<212> DNA

<213> Artificial Sequence

<220>
<223> forward primer

<400> 195
tgtctcactg agcgagcaga a 21

<210> 196
<211> 19
<212> DNA
<213> Artificial Sequence

<220>
<223> reverse primer

<400> 196
accattgcag ccctgattg 19

<210> 197
<211> 24
<212> DNA
<213> Artificial Sequence

<220>
<223> probe

<400> 197
cttgaggacg cgaacagtcc acca 24

<210> 198
<211> 22
<212> DNA
<213> Artificial Sequence

<220>
<223> forward primer

<400> 198
cgctgacatc atgaatgttc ct 22

<210> 199
<211> 24
<212> DNA
<213> Artificial Sequence

<220>
<223> reverse primer

<400> 199
tctctttcag caacgatgtg tctt 24

<210> 200
<211> 25
<212> DNA
<213> Artificial Sequence

<220>

<223> probe

<400> 200
tcatatccaa actcgcctcc agccg 25

<210> 201
<211> 19
<212> DNA
<213> Artificial sequence

<220>
<223> forward primer

<400> 201
cacaatggcg gctctgaag 19

<210> 202
<211> 26
<212> DNA
<213> Artificial Sequence

<220>
<223> reverse primer

<400> 202
acacaaacac tgtctgtacc tgaaga 26

<210> 203
<211> 23
<212> DNA
<213> Artificial Sequence

<220>
<223> probe

<400> 203
aagttacgct gcgcgacagc caa 23

<210> 204
<211> 24
<212> DNA
<213> Artificial Sequence

<220>
<223> forward primer

<400> 204
ctctgagaca gtgcttcgat gact 24

<210> 205
<211> 19
<212> DNA
<213> Artificial sequence

<220>
<223> reverse primer

<400> 205

ccatgaggcc caacttcct 19

<210> 206
<211> 23
<212> DNA
<213> Artificial Sequence

<220>
<223> probe

<400> 206
cagacttggt gccctttgac tcc 23

<210> 207
<211> 20
<212> DNA
<213> Artificial sequence

<220>
<223> forward primer

<400> 207
tgtcgatgga cttccagaac 20

<210> 208
<211> 18
<212> DNA
<213> Artificial Sequence

<220>
<223> probe

<400> 208
cacctgggca gctgccaa 18

<210> 209
<211> 19
<212> DNA
<213> Artificial Sequence

<220>
<223> reverse primer

<400> 209
attgggacag cttggatca 19

<210> 210
<211> 23
<212> DNA
<213> Artificial Sequence

<220>
<223> forward primer

<400> 210
gatctaagat ggcgactgtc gaa 23

<210> 211

<211> 25
<212> DNA
<213> Artificial Sequence

<220>
<223> reverse primer

<400> 211
ttagattccg ttttctcctc ttctg 25

<210> 212
<211> 27
<212> DNA
<213> Artificial Sequence

<220>
<223> probe

<400> 212
accaccccta ctcctaatcc cccgact 27

<210> 213
<211> 22
<212> DNA
<213> Artificial Sequence

<220>
<223> forward primer

<400> 213
ggcagtgtca ctgagtcctt ga 22

<210> 214
<211> 18
<212> DNA
<213> Artificial Sequence

<220>
<223> reverse primer

<400> 214
tgcactgtgc gtcccaat 18

<210> 215
<211> 18
<212> DNA
<213> Artificial Sequence

<220>
<223> probe

<400> 215
atcctcccct gccccgcg 18

<210> 216
<211> 20
<212> DNA
<213> Artificial Sequence

<220>
<223> forward primer

<400> 216
gggccctcca gaacaatgat 20

<210> 217
<211> 21
<212> DNA
<213> Artificial Sequence

<220>
<223> reverse primer

<400> 217
tgcactgctt ggccttaaag a 21

<210> 218
<211> 25
<212> DNA
<213> Artificial Sequence

<220>
<223> probe

<400> 218
ccgctctcat cgcagtcagg atcat 25

<210> 219
<211> 20
<212> DNA
<213> Artificial Sequence

<220>
<223> forward primer

<400> 219
accgtaggct ctgctctgaa 20

<210> 220
<211> 20
<212> DNA
<213> Artificial Sequence

<220>
<223> reverse primer

<400> 220
tggtccaggt ggaaaacttc 20

<210> 221
<211> 20
<212> DNA
<213> Artificial Sequence

<220>
<223> probe

<400> 221
aggcagccag acccacagga 20

<210> 222
<211> 23
<212> DNA
<213> Artificial Sequence

<220>
<223> forward primer

<400> 222
cggttatgtc atgccagata cac 23

<210> 223
<211> 25
<212> DNA
<213> Artificial Sequence

<220>
<223> probe

<400> 223
cctcaaaggt actccctcct cccgg 25

<210> 224
<211> 24
<212> DNA
<213> Artificial Sequence

<220>
<223> reverse primer

<400> 224
gaactgagac ccactgaaga aagg 24

<210> 225
<211> 19
<212> DNA
<213> Artificial Sequence

<220>
<223> forward primer

<400> 225
cgtggtgccc ctctatgac 19

<210> 226
<211> 19
<212> DNA
<213> Artificial Sequence

<220>
<223> probe

<400> 226
ctggagatgc tggacgccc 19

<210> 227
<211> 19
<212> DNA
<213> Artificial sequence

<220>
<223> reverse primer

<400> 227
ggctagtggg cgcatgtag 19

<210> 228
<211> 25
<212> DNA
<213> Artificial Sequence

<220>
<223> reverse primer

<400> 228
ggattgtaga ctgtcaccga aattc 25

<210> 229
<211> 28
<212> DNA
<213> Artificial Sequence

<220>
<223> forward primer

<400> 229
ggctattcct cattttctct acaaagtg 28

<210> 230
<211> 30
<212> DNA
<213> Artificial sequence

<220>
<223> probe

<400> 230
cctccaggag gctaccttct tcatgttcac 30

<210> 231
<211> 21
<212> DNA
<213> Artificial Sequence

<220>
<223> forward primer

<400> 231
cggtagtcaa gtccggatca a 21

<210> 232
<211> 18
<212> DNA

<213> Artificial Sequence

<220>
<223> reverse primer

<400> 232
gcttgccttt gcggttca 18

<210> 233
<211> 25
<212> DNA
<213> Artificial Sequence

<220>
<223> probe

<400> 233
caaggagacg gaattctacc tgtgc 25

<210> 234
<211> 20
<212> DNA
<213> Artificial Sequence

<220>
<223> forward primer

<400> 234
cacgggacat tcaccacatc 20

<210> 235
<211> 19
<212> DNA
<213> Artificial Sequence

<220>
<223> reverse primer

<400> 235
gggtgccatc cacttcaca 19

<210> 236
<211> 27
<212> DNA
<213> Artificial Sequence

<220>
<223> probe

<400> 236
ataaaaagac aaccaacggc cgactgc 27

<210> 237
<211> 18
<212> DNA
<213> Artificial Sequence

<220>

<223> forward primer

<400> 237
ccagtggagc gcttccat 18

<210> 238
<211> 22
<212> DNA
<213> Artificial Sequence

<220>
<223> reverse primer

<400> 238
ctctctgggt cgtctgaaac aa 22

<210> 239
<211> 23
<212> DNA
<213> Artificial Sequence

<220>
<223> probe

<400> 239
tcggccactt catcaggacg cag 23

<210> 240
<211> 20
<212> DNA
<213> Artificial sequence

<220>
<223> forward primer

<400> 240
ttggtacctg tgggttagca 20

<210> 241
<211> 20
<212> DNA
<213> Artificial Sequence

<220>
<223> reverse primer

<400> 241
cacatccaaa tgcaaactgg 20

<210> 242
<211> 26
<212> DNA
<213> Artificial Sequence

<220>
<223> probe

<400> 242

tccccagggt agaattcaat cagagc 26

<210> 243
<211> 19
<212> DNA
<213> Artificial sequence

<220>
<223> forward primer

<400> 243
tcagcagcaa gggcatcat 19

<210> 244
<211> 23
<212> DNA
<213> Artificial Sequence

<220>
<223> reverse primer

<400> 244
ggtggttttc ttgagcgtgt act 23

<210> 245
<211> 19
<212> DNA
<213> Artificial Sequence

<220>
<223> probe

<400> 245
cgcccgcagg cctcatcct 19

<210> 246
<211> 20
<212> DNA
<213> Artificial Sequence

<220>
<223> forward primer

<400> 246
attccaccca tggcaaattc 20

<210> 247
<211> 22
<212> DNA
<213> Artificial sequence

<220>
<223> reverse primer

<400> 247
gatgggattt ccattgatga ca 22

<210> 248

<211> 22
<212> DNA
<213> Artificial sequence

<220>
<223> probe

<400> 248
ccgttctcag ccttgacggt gc 22

<210> 249
<211> 23
<212> DNA
<213> Artificial Sequence

<220>
<223> forward primer

<400> 249
caaaggagct cactgtggtg tct 23

<210> 250
<211> 24
<212> DNA
<213> Artificial Sequence

<220>
<223> probe

<400> 250
tgttccaacc actgaatctg gacc 24

<210> 251
<211> 26
<212> DNA
<213> Artificial Sequence

<220>
<223> reverse primer

<400> 251
gagtcagaat ggcttattca cagatg 26

<210> 252
<211> 20
<212> DNA
<213> Artificial Sequence

<220>
<223> forward primer

<400> 252
ccatctgcat ccatcttgtt 20

<210> 253
<211> 23
<212> DNA
<213> Artificial Sequence

<220>
<223> probe

<400> 253
ctccccaccc ttgagaagtg cct 23

<210> 254
<211> 20
<212> DNA
<213> Artificial Sequence

<220>
<223> reverse primer

<400> 254
ggccaccagg gtattatctg 20

<210> 255
<211> 23
<212> DNA
<213> Artificial Sequence

<220>
<223> forward primer

<400> 255
cgaaaagatg ctgaacagtg aca 23

<210> 256
<211> 20
<212> DNA
<213> Artificial Sequence

<220>
<223> reverse primer

<400> 256
tcaggaacag ccaccagtga 20

<210> 257
<211> 28
<212> DNA
<213> Artificial Sequence

<220>
<223> probe

<400> 257
cttcctcctc ccttctggtc agttggat 28

<210> 258
<211> 20
<212> DNA
<213> Artificial sequence

<220>
<223> reverse primer

<400> 258
ggcccagctt gaatttttca 20

<210> 259
<211> 27
<212> DNA
<213> Artificial Sequence

<220>
<223> forward primer

<400> 259
aagctatgag gaaaagaagt acacgat 27

<210> 260
<211> 30
<212> DNA
<213> Artificial sequence

<220>
<223> probe

<400> 260
tcagccactg gcttctgtca taatcaggag 30

<210> 261
<211> 20
<212> DNA
<213> Artificial Sequence

<220>
<223> forward primer

<400> 261
cccactcagt agccaagtca 20

<210> 262
<211> 27
<212> DNA
<213> Artificial Sequence

<220>
<223> probe

<400> 262
tcaagtaaac gggctgtttt ccaaaca 27

<210> 263
<211> 20
<212> DNA
<213> Artificial sequence

<220>
<223> reverse primer

<400> 263
cacgcaggtg gtatcagtct 20

<210> 264
<211> 20
<212> DNA
<213> Artificial sequence

<220>
<223> forward primer

<400> 264
cggtgtgaga agtgcagcaa 20

<210> 265
<211> 24
<212> DNA
<213> Artificial Sequence

<220>
<223> probe

<400> 265
ccagaccata gcacactcgg gcac 24

<210> 266
<211> 19
<212> DNA
<213> Artificial Sequence

<220>
<223> reverse primer

<400> 266
cctctcgcaa gtgctccat 19

<210> 267
<211> 24
<212> DNA
<213> Artificial Sequence

<220>
<223> forward primer

<400> 267
tgaacataaa gtctgcaaca tgga 24

<210> 268
<211> 28
<212> DNA
<213> Artificial sequence

<220>
<223> reverse primer

<400> 268
tgaggttggt tactgttggt atcatata 28

<210> 269
<211> 24
<212> DNA

<213> Artificial Sequence

<220>
<223> probe

<400> 269
ttgcactgca caggccacat tcac 24

<210> 270
<211> 24
<212> DNA
<213> Artificial Sequence

<220>
<223> forward primer

<400> 270
tccaggatgt taggaactgt gaag 24

<210> 271
<211> 22
<212> DNA
<213> Artificial Sequence

<220>
<223> reverse primer

<400> 271
gcgtgtctgc gtagtagctg tt 22

<210> 272
<211> 24
<212> DNA
<213> Artificial Sequence

<220>
<223> probe

<400> 272
agtcgctggt ttcatgccct tcca 24

<210> 273
<211> 19
<212> DNA
<213> Artificial Sequence

<220>
<223> forward primer

<400> 273
agaaccgcaa ggtgagcaa 19

<210> 274
<211> 21
<212> DNA
<213> Artificial Sequence

<220>

<223> reverse primer

<400> 274
tccaactgaa ggtccctgat g 21

<210> 275
<211> 26
<212> DNA
<213> Artificial Sequence

<220>
<223> probe

<400> 275
tggagattct ccagcacgtc atcgac 26

<210> 276
<211> 21
<212> DNA
<213> Artificial sequence

<220>
<223> forward primer

<400> 276
tccggagctg tgatctaagg a 21

<210> 277
<211> 24
<212> DNA
<213> Artificial Sequence

<220>
<223> probe

<400> 277
tgtattgcgc acccctcaag cctg 24

<210> 278
<211> 20
<212> DNA
<213> Artificial Sequence

<220>
<223> reverse primer

<400> 278
cggacagagc gagctgactt 20

<210> 279
<211> 21
<212> DNA
<213> Artificial Sequence

<220>
<223> forward primer

<400> 279

gcatggtagc cgaagatttc a 21

<210> 280
<211> 30
<212> DNA
<213> Artificial Sequence

<220>
<223> reverse primer

<400> 280
tttccggtaa tagtctgtct catagatatc 30

<210> 281
<211> 28
<212> DNA
<213> Artificial Sequence

<220>
<223> probe

<400> 281
cgcgtcatac caaaatctcc gattttga 28

<210> 282
<211> 19
<212> DNA
<213> Artificial sequence

<220>
<223> forward primer

<400> 282
gtggacagca ccatgaaca 19

<210> 283
<211> 20
<212> DNA
<213> Artificial sequence

<220>
<223> reverse primer

<400> 283
ccttcatacc cgacttgagg 20

<210> 284
<211> 20
<212> DNA
<213> Artificial sequence

<220>
<223> probe

<400> 284
cttccggcca gcactgcctc 20

<210> 285

<211> 20
<212> DNA
<213> Artificial Sequence

<220>
<223> forward primer

<400> 285
cctgaacctt ccaaagatgg 20

<210> 286
<211> 20
<212> DNA
<213> Artificial Sequence

<220>
<223> reverse primer

<400> 286
accaggcaag tctcctcatt 20

<210> 287
<211> 27
<212> DNA
<213> Artificial Sequence

<220>
<223> probe

<400> 287
ccagattgga agcatccatc tttttca 27

<210> 288
<211> 20
<212> DNA
<213> Artificial Sequence

<220>
<223> forward primer

<400> 288
ccacagctca ccttctgtca 20

<210> 289
<211> 20
<212> DNA
<213> Artificial Sequence

<220>
<223> reverse primer

<400> 289
cctcagtgcc agtctcttcc 20

<210> 290
<211> 20
<212> DNA
<213> Artificial Sequence

<220>
<223> probe

<400> 290
tccatcccag ctccagccag 20

<210> 291
<211> 23
<212> DNA
<213> Artificial Sequence

<220>
<223> probe

<400> 291
ccacttgtcg aaccaccgct cgt 23

<210> 292
<211> 19
<212> DNA
<213> Artificial Sequence

<220>
<223> forward primer

<400> 292
cggactttgg gtgcgactt 19

<210> 293
<211> 24
<212> DNA
<213> Artificial Sequence

<220>
<223> reverse primer

<400> 293
ttacaactct tccactggga cgat 24

<210> 294
<211> 18
<212> DNA
<213> Artificial Sequence

<220>
<223> forward primer

<400> 294
gcccagaggc tccatcgt 18

<210> 295
<211> 23
<212> DNA
<213> Artificial Sequence

<220>
<223> reverse primer

<400> 295
cagaggtttg aacagtgcag aca 23

<210> 296
<211> 23
<212> DNA
<213> Artificial Sequence

<220>
<223> probe

<400> 296
cctcttcctc cccagtcggc tga 23

<210> 297
<211> 20
<212> DNA
<213> Artificial Sequence

<220>
<223> forward primer

<400> 297
ggcctgctga gatcaaagac 20

<210> 298
<211> 20
<212> DNA
<213> Artificial Sequence

<220>
<223> reverse primer

<400> 298
gtccactgtg gctgtgagaa 20

<210> 299
<211> 26
<212> DNA
<213> Artificial Sequence

<220>
<223> probe

<400> 299
tgttcctcag gtcctcaatg gtcttg 26

<210> 300
<211> 21
<212> DNA
<213> Artificial Sequence

<220>
<223> forward primer

<400> 300
cgaggattgg ttcttcagca a 21

<210> 301
<211> 22
<212> DNA
<213> Artificial Sequence

<220>
<223> reverse primer

<400> 301
actctgcacc agctcactgt tg 22

<210> 302
<211> 24
<212> DNA
<213> Artificial sequence

<220>
<223> probe

<400> 302
cacctcgcgg ttcagttcct ctgt 24

<210> 303
<211> 20
<212> DNA
<213> Artificial Sequence

<220>
<223> forward primer

<400> 303
agagatcgag gctctcaagg 20

<210> 304
<211> 20
<212> DNA
<213> Artificial Sequence

<220>
<223> reverse primer

<400> 304
ggccttttac ttcctcttcg 20

<210> 305
<211> 27
<212> DNA
<213> Artificial sequence

<220>
<223> probe

<400> 305
tggttcttct tcatgaagag cagctcc 27

<210> 306
<211> 21
<212> DNA

<213> Artificial sequence

<220>
<223> forward primer

<400> 306
tgagcggcag aatcaggagt a 21

<210> 307
<211> 19
<212> DNA
<213> Artificial Sequence

<220>
<223> reverse primer

<400> 307
tgcggtaggt ggcaatctc 19

<210> 308
<211> 24
<212> DNA
<213> Artificial Sequence

<220>
<223> probe

<400> 308
ctcatggaca tcaagtcgcg gctg 24

<210> 309
<211> 20
<212> DNA
<213> Artificial Sequence

<220>
<223> forward primer

<400> 309
tcagtggaga aggagttgga 20

<210> 310
<211> 28
<212> DNA
<213> Artificial Sequence

<220>
<223> probe

<400> 310
ccagtcaaca tctctgttgt cacaagca 28

<210> 311
<211> 20
<212> DNA
<213> Artificial Sequence

<220>

<223> reverse primer

<400> 311
tgccatatcc agaggaaaca 20

<210> 312
<211> 27
<212> DNA
<213> Artificial sequence

<220>
<223> forward primer

<400> 312
ggatgaagct tacatgaaca aggtaga 27

<210> 313
<211> 25
<212> DNA
<213> Artificial Sequence

<220>
<223> reverse primer

<400> 313
catatagctg cctgaggaag ttgat 25

<210> 314
<211> 21
<212> DNA
<213> Artificial sequence

<220>
<223> probe

<400> 314
cgtcggtcag cccttccagg c 21

<210> 315
<211> 22
<212> DNA
<213> Artificial sequence

<220>
<223> forward primer

<400> 315
ggaaagacca cctgaaaaac ca 22

<210> 316
<211> 24
<212> DNA
<213> Artificial sequence

<220>
<223> reverse primer

<400> 316

gtacttcttc ccacactcct caca 24

<210> 317
<211> 23
<212> DNA
<213> Artificial sequence

<220>
<223> probe

<400> 317
acccacgacc ccaacaaaat ggc 23

<210> 318
<211> 23
<212> DNA
<213> Artificial Sequence

<220>
<223> forward primer

<400> 318
cagatggcca ctttgagaac att 23

<210> 319
<211> 22
<212> DNA
<213> Artificial Sequence

<220>
<223> reverse primer

<400> 319
ggcagcatta accacaagga tt 22

<210> 320
<211> 28
<212> DNA
<213> Artificial sequence

<220>
<223> probe

<400> 320
agctgacaac agtgtgaacg accagacc 28

<210> 321
<211> 21
<212> DNA
<213> Artificial Sequence

<220>
<223> forward primer

<400> 321
gacttttgcc cgctaccttt c 21

<210> 322

<211> 26
<212> DNA
<213> Artificial Sequence

<220>
<223> reverse primer

<400> 322
gccactaact gcttcagtat gaagag 26

<210> 323
<211> 24
<212> DNA
<213> Artificial Sequence

<220>
<223> probe

<400> 323
acagctcatt gttgtcacgc cgga 24

<210> 324
<211> 20
<212> DNA
<213> Artificial Sequence

<220>
<223> forward primer

<400> 324
ggagaacaat ccccttgaga 20

<210> 325
<211> 20
<212> DNA
<213> Artificial Sequence

<220>
<223> reverse primer

<400> 325
atctcctgga tggtgatggt 20

<210> 326
<211> 27
<212> DNA
<213> Artificial Sequence

<220>
<223> probe

<400> 326
tggcctttct gtctacaagg atcacca 27

<210> 327
<211> 24
<212> DNA
<213> Artificial Sequence

<220>
<223> forward primer

<400> 327
tgatggtcct atgtgtcaca ttca 24

<210> 328
<211> 20
<212> DNA
<213> Artificial Sequence

<220>
<223> reverse primer

<400> 328
tgggacagga aacacaccaa 20

<210> 329
<211> 30
<212> DNA
<213> Artificial Sequence

<220>
<223> probe

<400> 329
caggtttcat accaacacag gcttcag 30

<210> 330
<211> 19
<212> DNA
<213> Artificial Sequence

<220>
<223> forward primer

<400> 330
ctacagggac gccatcgaa 19

<210> 331
<211> 23
<212> DNA
<213> Artificial Sequence

<220>
<223> reverse primer

<400> 331
atccaaccaa tcacctgaat gtt 23

<210> 332
<211> 23
<212> DNA
<213> Artificial Sequence

<220>
<223> probe

<400> 332
cttacaccag catcaagatc cgg 23

<210> 333
<211> 20
<212> DNA
<213> Artificial Sequence

<220>
<223> forward primer

<400> 333
gagaaccaat ctcaccgaca 20

<210> 334
<211> 20
<212> DNA
<213> Artificial Sequence

<220>
<223> reverse primer

<400> 334
cacccgagtg taaccatagc 20

<210> 335
<211> 24
<212> DNA
<213> Artificial Sequence

<220>
<223> probe

<400> 335
acaggtattc ctctgccagc tgcc 24

<210> 336
<211> 19
<212> DNA
<213> Artificial Sequence

<220>
<223> forward primer

<400> 336
ccgccctcac ctgaagaga 19

<210> 337
<211> 22
<212> DNA
<213> Artificial Sequence

<220>
<223> reverse primer

<400> 337
ggaataagtt agccgcgctt ct 22

<210> 338
<211> 21
<212> DNA
<213> Artificial sequence

<220>
<223> probe

<400> 338
cccagtgtcc gccaaggagc g 21

<210> 339
<211> 18
<212> DNA
<213> Artificial Sequence

<220>
<223> forward primer

<400> 339
gccgagatcg ccaagatg 18

<210> 340
<211> 27
<212> DNA
<213> Artificial Sequence

<220>
<223> reverse primer

<400> 340
cttttgatgg tagagttcca gtgattc 27

<210> 341
<211> 24
<212> DNA
<213> Artificial Sequence

<220>
<223> probe

<400> 341
cagcattgtc tgtcctccct ggca 24

<210> 342
<211> 19
<212> DNA
<213> Artificial Sequence

<220>
<223> forward primer

<400> 342
ccctcgtgct gatgctact 19

<210> 343
<211> 22
<212> DNA

<213> Artificial Sequence

<220>
<223> reverse primer

<400> 343
catcatgacc tggtcttcta gg 22

<210> 344
<211> 22
<212> DNA
<213> Artificial sequence

<220>
<223> probe

<400> 344
ctgccctaga cgctggctcc tc 22

<210> 345
<211> 21
<212> DNA
<213> Artificial Sequence

<220>
<223> forward primer

<400> 345
cggtggacca cgaagagtta a 21

<210> 346
<211> 23
<212> DNA
<213> Artificial Sequence

<220>
<223> probe

<400> 346
ccgggacttg gagaagcact gca 23

<210> 347
<211> 19
<212> DNA
<213> Artificial Sequence

<220>
<223> reverse primer

<400> 347
ggctcgcctc ttccatgtc 19

<210> 348
<211> 20
<212> DNA
<213> Artificial Sequence

<220>

<223> forward primer

<400> 348
ctttgaaccc ttgcttgcaa 20

<210> 349
<211> 25
<212> DNA
<213> Artificial Sequence

<220>
<223> probe

<400> 349
aagtcctggg tgcttctgac gcaca 25

<210> 350
<211> 18
<212> DNA
<213> Artificial Sequence

<220>
<223> reverse primer

<400> 350
cccgggacaa agcaaatg 18

<210> 351
<211> 19
<212> DNA
<213> Artificial Sequence

<220>
<223> forward primer

<400> 351
ccgcaacgtg gttttctca 19

<210> 352
<211> 22
<212> DNA
<213> Artificial Sequence

<220>
<223> probe

<400> 352
ctcggtgttg gccatgctcc ag 22

<210> 353
<211> 21
<212> DNA
<213> Artificial Sequence

<220>
<223> reverse primer

<400> 353

tgctgggttt ctcctcctgt t 21

<210> 354
<211> 20
<212> DNA
<213> Artificial Sequence

<220>
<223> forward primer

<400> 354
ccagctctcc ttccagctac 20

<210> 355
<212> 20
<212> DNA
<213> Artificial Sequence

<220>
<223> reverse primer

<400> 355
gggtggctct cacttagctc 20

<210> 356
<211> 24
<212> DNA
<213> Artificial sequence

<220>
<223> probe

<400> 356
atcaatgtcc ctgtccgagt gctg 24

<210> 357
<211> 23
<212> DNA
<213> Artificial Sequence

<220>
<223> reverse primer

<400> 357
cacactagca ttttctccgc ata 23

<210> 358
<211> 21
<212> DNA
<213> Artificial Sequence

<220>
<223> forward primer

<400> 358
ataccaatca ccgcacaaac c 21

<210> 359

<211> 30
<212> DNA
<213> Artificial Sequence

<220>
<223> probe

<400> 359
tgcgctgtga ctggacttaa caaatagcct 30

<210> 360
<211> 18
<212> DNA
<213> Artificial Sequence

<220>
<223> forward primer

<400> 360
gccatccgca aaggcttt 18

<210> 361
<211> 18
<212> DNA
<213> Artificial Sequence

<220>
<223> reverse primer

<400> 361
ggccattccg ccagtttc 18

<210> 362
<211> 23
<212> DNA
<213> Artificial Sequence

<220>
<223> probe

<400> 362
tcgcttgtca ccttgccatg tgg 23

<210> 363
<211> 20
<212> DNA
<213> Artificial Sequence

<220>
<223> forward primer

<400> 363
gcatcaggct gtcattatgg 20

<210> 364
<211> 20
<212> DNA
<213> Artificial Sequence

<220>
<223> reverse primer

<400> 364
agtagttgtg ctgcccttcc 20

<210> 365
<211> 28
<212> DNA
<213> Artificial Sequence

<220>
<223> probe

<400> 365
tgtccttacc tgtgggagct gtaaggtc 28

<210> 366
<211> 23
<212> DNA
<213> Artificial Sequence

<220>
<223> forward primer

<400> 366
tctccatatc tgccttgcag agt 23

<210> 367
<211> 19
<212> DNA
<213> Artificial Sequence

<220>
<223> reverse primer

<400> 367
gcacgtgggt cagattgct 19

<210> 368
<211> 22
<212> DNA
<213> Artificial Sequence

<220>
<223> probe

<400> 368
tcctgcagca cctcatcggg ct 22

<210> 369
<211> 19
<212> DNA
<213> Artificial Sequence

<220>
<223> forward primer

<400> 369
gccctcccag tgtgcaaat 19

<210> 370
<211> 23
<212> DNA
<213> Artificial Sequence

<220>
<223> probe

<400> 370
tgctgtttcg acgacaccgt tcg 23

<210> 371
<211> 25
<212> DNA
<213> Artificial sequence

<220>
<223> reverse primer

<400> 371
cgtcgatggt attaggatag aagca 25

<210> 372
<211> 24
<212> DNA
<213> Artificial Sequence

<220>
<223> forward primer

<400> 372
gaacttcttg agcaggagca tacc 24

<210> 373
<211> 25
<212> DNA
<213> Artificial Sequence

<220>
<223> reverse primer

<400> 373
tccacccccca agaatatcat ctagt 25

<210> 374
<211> 25
<212> DNA
<213> Artificial Sequence

<220>
<223> probe

<400> 374
agggcttcat aatcaccttc tgttc 25

<210> 375
<211> 20
<212> DNA
<213> Artificial Sequence

<220>
<223> forward primer

<400> 375
cgaagccctt acaagtttcc 20

<210> 376
<211> 20
<212> DNA
<213> Artificial Sequence

<220>
<223> reverse primer

<400> 376
ggactcttca ggggtgaaat 20

<210> 377
<211> 24
<212> DNA
<213> Artificial sequence

<220>
<223> probe

<400> 377
cccttacgga ttcctggagg gaac 24

<210> 378
<211> 20
<212> DNA
<213> Artificial Sequence

<220>
<223> forward primer

<400> 378
ccagacgagc gattagaagc 20

<210> 379
<211> 20
<212> DNA
<213> Artificial Sequence

<220>
<223> reverse primer

<400> 379
tcctcctctt cctcctcctc 20

<210> 380
<211> 23
<212> DNA

<213> Artificial Sequence

<220>
<223> probe

<400> 380
tgtgaggtga atgatttggg gga 23

<210> 381
<211> 20
<212> DNA
<213> Artificial Sequence

<220>
<223> forward primer

<400> 381
catcttccag gaggaccact 20

<210> 382
<211> 20
<212> DNA
<213> Artificial Sequence

<220>
<223> reverse primer

<400> 382
tccgaccttc aatcatttca 20

<210> 383
<211> 24
<212> DNA
<213> Artificial Sequence

<220>
<223> probe

<400> 383
ctctgtggca ccctggacta cctg 24

<210> 384
<211> 20
<212> DNA
<213> Artificial Sequence

<220>
<223> forward primer

<400> 384
cctggaggct gcaacatacc 20

<210> 385
<211> 23
<212> DNA
<213> Artificial Sequence

<220>

<223> reverse primer

<400> 385
tacaatggct ttggaggata gca 23

<210> 386
<211> 25
<212> DNA
<213> Artificial Sequence

<220>
<223> probe

<400> 386
atcctcctga agcccttttc gcagc 25

<210> 387
<211> 20
<212> DNA
<213> Artificial sequence

<220>
<223> forward primer

<400> 387
tgttttgatt cccgggctta 20

<210> 388
<211> 28
<212> DNA
<213> Artificial Sequence

<220>
<223> probe

<400> 388
tgccttcttc ctccctcact tctcacct 28

<210> 389
<211> 24
<212> DNA
<213> Artificial Sequence

<220>
<223> reverse primer

<400> 389
caaagctgtc agctctagca aaag 24

<210> 390
<211> 19
<212> DNA
<213> Artificial sequence

<220>
<223> forward primer

<400> 390

gcccgaaacg ccgaatata 19

<210> 391
<211> 21
<212> DNA
<213> Artificial Sequence

<220>
<223> probe

<400> 391
taccgcagca aaccgcttgg g 21

<210> 392
<211> 23
<212> DNA
<213> Artificial Sequence

<220>
<223> reverse primer

<400> 392
cgtggctctc ttatcctcat gat 23

<210> 393
<211> 20
<212> DNA
<213> Artificial Sequence

<220>
<223> forward primer

<400> 393
ggtgtgccac agaccttcct 20

<210> 394
<211> 24
<212> DNA
<213> Artificial Sequence

<220>
<223> reverse primer

<400> 394
acggagttct tgacagagtt ttga 24

<210> 395
<211> 27
<212> DNA
<213> Artificial Sequence

<220>
<223> probe

<400> 395
ttggcctgta atcacctgtg cagcctt 27

<210> 396

<211> 19
<212> DNA
<213> Artificial Sequence

<220>
<223> forward primer

<400> 396
tccctgcggt cccagatag 19

<210> 397
<211> 20
<212> DNA
<213> Artificial Sequence

<220>
<223> reverse primer

<400> 397
gtgggaacag ggtggacact 20

<210> 398
<211> 25
<212> DNA
<213> Artificial Sequence

<220>
<223> probe

<400> 398
atcctgcccg gagtggaact gaagc 25

<210> 399
<211> 20
<212> DNA
<213> Artificial Sequence

<220>
<223> forward primer

<400> 399
aatccaaggg ggagagtgat 20

<210> 400
<211> 26
<212> DNA
<213> Artificial sequence

<220>
<223> probe

<400> 400
catatggact ttgactcagc tgtggc 26

<210> 401
<211> 20
<212> DNA
<213> Artificial Sequence

<220>
<223> reverse primer

<400> 401
gtacagattt tgcccgagga 20

<210> 402
<211> 21
<212> DNA
<213> Artificial Sequence

<220>
<223> forward primer

<400> 402
tgtggacatc ttcccctcag a 21

<210> 403
<211> 24
<212> DNA
<213> Artificial sequence

<220>
<223> probe

<400> 403
ttccctactg agccaccttc tctg 24

<210> 404
<211> 18
<212> DNA
<213> Artificial sequence

<220>
<223> reverse primer

<400> 404
ctagcccgac cggttcgt 18

<210> 405
<211> 24
<212> DNA
<213> Artificial Sequence

<220>
<223> forward primer

<400> 405
ctatatgcag ccagagatgt gaca 24

<210> 406
<211> 23
<212> DNA
<213> Artificial Sequence

<220>
<223> probe

<400> 406
acagcctgcc actcatcaca gcc 23

<210> 407
<211> 24
<212> DNA
<213> Artificial Sequence

<220>
<223> reverse primer

<400> 407
ccacgagttt cttactgaga atgg 24

<210> 408
<211> 19
<212> DNA
<213> Artificial Sequence

<220>
<223> forward primer

<400> 408
gggccaaata ttcagaagc 19

<210> 409
<211> 20
<212> DNA
<213> Artificial Sequence

<220>
<223> reverse primer

<400> 409
ggatgggtat gatgggacag 20

<210> 410
<211> 20
<212> DNA
<213> Artificial Sequence

<220>
<223> probe

<400> 410
ccaccatagc ggccatggag 20

<210> 411
<211> 22
<212> DNA
<213> Artificial Sequence

<220>
<223> forward primer

<400> 411
cttcacagtg ctcctgcagt ct 22

<210> 412
<211> 21
<212> DNA
<213> Artificial Sequence

<220>
<223> reverse primer

<400> 412
catctgcttc agctcgttgg t 21

<210> 413
<211> 26
<212> DNA
<213> Artificial sequence

<220>
<223> probe

<400> 413
aagtacacgt aagttacagc cacaca 26

<210> 414
<211> 18
<212> DNA
<213> Artificial Sequence

<220>
<223> forward primer

<400> 414
gcctcggtgt gcctttca 18

<210> 415
<211> 22
<212> DNA
<213> Artificial Sequence

<220>
<223> probe

<400> 415
catcgccagc tacgccctgc tc 22

<210> 416
<211> 19
<212> DNA
<213> Artificial Sequence

<220>
<223> reverse primer

<400> 416
cgtgatgtgc gcaatcatg 19

<210> 417
<211> 19
<212> DNA

<213> Artificial sequence

<220>
<223> forward primer

<400> 417
gtggatgtgc cctgaagga 19

<210> 418
<211> 20
<212> DNA
<213> Artificial sequence

<220>
<223> reverse primer

<400> 418
ctgcggatcc agggtaagaa 20

<210> 419
<211> 28
<212> DNA
<213> Artificial Sequence

<220>
<223> probe

<400> 419
aagccaggcg tctacacgag agtctcac 28

<210> 420
<211> 20
<212> DNA
<213> Artificial sequence

<220>
<223> forward primer

<400> 420
gccctggatt tcagaaagag 20

<210> 421
<211> 20
<212> DNA
<213> Artificial Sequence

<220>
<223> reverse primer

<400> 421
agttacaagc cagggaagga 20

<210> 422
<211> 25
<212> DNA
<213> Artificial Sequence

<220>

<223> probe

<400> 422
caagtctgga tctgggaccc tttcc 25

<210> 423
<211> 20
<212> DNA
<213> Artificial Sequence

<220>
<223> forward primer

<400> 423
ctgctgtctt gggtgcattg 20

<210> 424
<211> 18
<212> DNA
<213> Artificial sequence

<220>
<223> reverse primer

<400> 424
gcagcctggg accacttg 18

<210> 425
<211> 25
<212> DNA
<213> Artificial Sequence

<220>
<223> probe

<400> 425
ttgccttgct gctctacctc cacca 25

<210> 426
<211> 19
<212> DNA
<213> Artificial sequence

<220>
<223> forward primer

<400> 426
tgacgatggc ctggagtgt 19

<210> 427
<211> 22
<212> DNA
<213> Artificial Sequence

<220>
<223> reverse primer

<400> 427

ggtaccggat catgaggatc tg 22

<210> 428
<211> 21
<212> DNA
<213> Artificial Sequence

<220>
<223> probe

<400> 428
ctgggcagca ccaagtccgg a 21

<210> 429
<211> 22
<212> DNA
<213> Artificial Sequence

<220>
<223> forward primer

<400> 429
agaggcatcc atgaacttca ca 22

<210> 430
<211> 24
<212> DNA
<213> Artificial Sequence

<220>
<223> reverse primer

<400> 430
caaactccac agtacttggg ttga 24

<210> 431
<211> 20
<212> DNA
<213> Artificial Sequence

<220>
<223> probe

<400> 431
cgggctgcat cagcacacgc 20

<210> 432
<211> 23
<212> DNA
<213> Artificial sequence

<220>
<223> forward primer

<400> 432
gcagttggaa gacacaggaa agt 23

<210> 433

<211> 27
<212> DNA
<213> Artificial Sequence

<220>
<223> probe

<400> 433
tccccaaatt gcagatttat caacggc 27

<210> 434
<211> 21
<212> DNA
<213> Artificial Sequence

<220>
<223> reverse primer

<400> 434
tgcgtggcac tattttcaag a 21

<210> 435
<211> 25
<212> DNA
<213> Artificial Sequence

<220>
<223> forward primer

<400> 435
agactgtgga gtttgatgtt gttga 25

<210> 436
<211> 22
<212> DNA
<213> Artificial Sequence

<220>
<223> reverse primer

<400> 436
ggaacaccac caggacctgt aa 22

<210> 437
<211> 23
<212> DNA
<213> Artificial Sequence

<220>
<223> probe

<400> 437
ttgctgcctc cgcacccttt tct 23

<210> 438
<211> 20
<212> DNA
<213> Artificial Sequence

<220>
<223> forward primer

<400> 438
acccagtagc aaggagaagc 20

<210> 439
<211> 20
<212> DNA
<213> Artificial Sequence

<220>
<223> reverse primer

<400> 439
cagctggtgg taggttctga 20

<210> 440
<211> 21
<212> DNA
<213> Artificial Sequence

<220>
<223> probe

<400> 440
cactcactgc tccgagtgcg g 21

**Description**

Background of the Invention

Field of the Invention

The present invention provides genes and gene sets the expression of which is important in the diagnosis and/or prognosis of breast cancer.

Description of the Related Art

Oncologists have a number of treatment options available to them, including different combinations of chemotherapeutic drugs that are characterized as "standard of care," and a number of drugs that do not carry a label claim for particular cancer, but for which there is evidence of efficacy in that cancer. Best likelihood of good treatment outcome requires that patients be assigned to optimal available cancer treatment, and that this assignment be made as quickly as possible following diagnosis.

Currently, diagnostic tests used in clinical practice are single analyte, and therefore do not capture the potential value of knowing relationships between dozens of different markers. Moreover, diagnostic tests are frequently not quantitative, relying on immunohistochemistry. This method often yields different results in different laboratories, in part because the reagents are not standardized, and in part because the interpretations are subjective and cannot be easily quantified. RNA-based tests have not often been used because of the problem of RNA degradation over time and the fact that it is difficult to obtain fresh tissue samples from patients for analysis. Fixed paraffin-embedded tissue is more readily available and methods have been established to detect RNA in fixed tissue. However, these methods typically do not allow for the study of large numbers of genes (DNA or RNA) from small amounts of material. Thus, traditionally fixed tissue has been rarely used other than for immunohistochemistry detection of proteins.

Recently, several groups have published studies concerning the classification of various cancer types by microarray gene expression analysis (see, e.g. Golub et al., Science 286:531-537 (1999); Bhattacharjae et al., Proc. Natl. Acad. Sci. USA 98:13790-13795 (2001); Chen-Hsiang et al., Bioinformatics 17 (Suppl. 1):S316-S322 (2001); Ramaswamy et al., Proc. Natl. Acad. Sci. USA 98:15149-15154 (2001)). Certain classifications of human breast cancers based on gene expression patterns have also been reported (Martin et al., Cancer Res. 60:2232-2238 (2000); West et al., Proc. Natl. Acad. Sci. USA 98:11462-11467 (2001); Sorlie et al., Proc. Natl. Acad. Sci. USA 98:10869-10874 (2001); Yan et al., Cancer Res. 61:8375-8380 (2001)). However, these studies mostly focus on improving and refining the already established classification of various types of cancer, including breast cancer, and generally do not provide new insights into the relationships of the differentially expressed genes, and do not link the findings to treatment strategies in order to improve the clinical outcome of cancer therapy.

Although modem molecular biology and biochemistry have revealed hundreds of genes whose activities influence the behavior of tumor cells, state of their differentiation, and their sensitivity or resistance to certain therapeutic drugs, with a few exceptions, the status of these genes has not been exploited for the purpose of routinely making clinical decisions about drug treatments. One notable exception is the use of estrogen receptor (ER) protein expression in breast carcinomas to select patients to treatment with anti-estrogen drugs, such as tamoxifen. Another exceptional example is the use of ErbB2 (Her2) protein expression in breast carcinomas to select patients with the Her2 antagonist drug Herceptin® (Genentech, Inc., South San Francisco, CA).

Despite recent advances, the challenge of cancer treatment remains to target specific treatment regimens to pathogenically distinct tumor types, and ultimately personalize tumor treatment in order to maximize outcome. Hence, a need exists for tests that simultaneously provide predictive information about patient responses to the variety of treatment options. This is particularly true for breast cancer, the biology of which is poorly understood. It is clear that the classification of breast cancer into a few subgroups, such as $ErbB2^+$ subgroup, and subgroups characterized by low to absent gene expression of the estrogen receptor (ER) and a few additional transcriptional factors (Perou et al., Nature 406:747-752 (2000)) does not reflect the cellular and molecular heterogeneity of breast cancer, and does not allow the design of treatment strategies maximizing patient response.

WO02/10436 discloses a method for prognostic classification of breast cancer based on determining the expression level of one or more RNA transcripts selected from a list of 57 genes. These 57 genes are found to be preferentially expressed in tumors of poor prognosis vs. tumors of good prognosis. The long-term survival of patients without recurrence is measured through the mitotic activity index, which is an indicator of death, recurrence and metastases in breast cancer.

RASCHELLA GIUSEPPE ET AL ("Expression of B-myb in neuroblastoma tumors is a poor prognostic factor independent from MYCN amplification" CANCER RESEARCH, vol. 59, no. 14, pages 3365-3368) disclose MYBL2 as a marker of poor prognosis in neuroblastoma.

Summary of the Invention

The present disclosure provides a set of genes, the expression of which has prognostic value, specifically with respect to disease-free survival.

The present invention accommodates the use of archived paraffin-embedded biopsy material for assay of all markers in the set, and therefore is compatible with the most widely available type of biopsy material. It is also compatible with several different methods of tumour tissue harvest, for example, via core biopsy or fine needle aspiration. Further, for each member of the gene set, the invention specifies oligonucleotide sequences that can be used in the test.

According to an aspect of the present invention, there is provided a method of predicting the likelihood of long-term survival of a breast cancer patient without recurrence of breast cancer as specified in claim 1.

In a particular embodiment, the breast cancer is invasive breast carcinoma.

In a further embodiment, RNA is isolated from a fixed, wax-embedded breast cancer tissue specimen of the patient. Isolation may be performed by a technique known in the art, for example from core biopsy tissue or fine needle apirate cells.

The patient may be diagnosed with estrogen receptor (ER)-positive invasive breast cancer.

The method may include creating a report summarizing the data obtained by the gene expression analysis.

The report may, for example, include prediction of the likelihood of long term survival of the patient and/or recommendation for a treatment modality of said patient.

Brief Description of the Drawings

Table 1 is a list of genes, expression of which correlate with breast cancer survival. Results from a retrospective clinical trial. Binary statistical analysis.

Table 2 is a list of genes, expression of which correlates with breast cancer survival in estrogen receptor (ER) positive patients. Results from a retrospective clinical trial. Binary statistical analysis.

Table 3 is a list of genes, expression of which correlates with breast cancer survival in estrogen receptor (ER) negative patients. Results from a retrospective clinical trial. Binary statistical analysis.

Table 4 is a list of genes, expression of which correlates with breast cancer survival. Results from a retrospective clinical trial. Cox proportional hazards statistical analysis.

Tables 5A and B show a list of genes, expression of which correlate with breast cancer survival. Results from a retrospective clinical trial. The table includes accession numbers for the genes, and amplicon sequences used for PCR amplification.

Tables 6A-6F The table includes sequences for the forward and reverse primers (designated by "f" and "r", respectively) and probes (designated by "p") used for PCR amplification of the amplicons listed in Tables 5A-B.

Detailed Description of the Preferred Embodiment

A. Definitions

Unless defined otherwise, technical and scientific terms used herein have the same meaning as commonly understood by one of ordinary skill in the art to which this invention belongs. Singleton *et al.,* Dictionary of Microbiology and Molecular Biology 2nd ed., J. Wiley & Sons (New York, NY 1994), and March, Advanced Organic Chemistry Reactions, Mechanisms and Structure 4th ed., John Wiley & Sons (New York, NY 1992), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

The term "microarray" refers to an ordered arrangement of hybridizable array elements, preferably polynucleotide probes, on a substrate.

The term "polynucleotide," when used in singular or plural, generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules.

The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including *in vitro* recombinant DNA-mediated techniques and by expression of DNAs in cells and organisms.

The terms "differentially expressed gene," "differential gene expression" and their synonyms, which are used interchangeably, refer to a gene whose expression is activated to a higher or lower level in a subject suffering from a disease, specifically cancer, such as breast cancer, relative to its expression in a normal or control subject. The terms also include genes whose expression is activated to a higher or lower level at different stages of the same disease. It is also understood that a differentially expressed gene may be either activated or inhibited at the nucleic acid level or protein level, or may be subject to alternative splicing to result in a different polypeptide product. Such differences may be evidenced by a change in mRNA levels, surface expression, secretion or other partitioning of a polypeptide, for example. Differential gene expression may include a comparison of expression between two or more genes or their gene products, or a comparison of the ratios of the expression between two or more genes or their gene products, or even a comparison of two differently processed products of the same gene, which differ between normal subjects and subjects suffering from a disease, specifically cancer, or between various stages of the same disease. Differential expression includes both quantitative, as well as qualitative, differences in the temporal or cellular expression pattern in a gene or its expression products among, for example, normal and diseased cells, or among cells which have undergone different disease events or disease stages. For the purpose of this invention, "differential gene expression" is considered to be present when there is at least an about two-fold, preferably at least about four-fold, more preferably at least about six-fold, most preferably at least about ten-fold difference between the expression of a given gene in normal and diseased subjects, or in various stages of disease development in a diseased subject.

The phrase "gene amplification" refers to a process by which multiple copies of a gene or gene fragment are formed in a particular cell or cell line. The duplicated region (a stretch of amplified DNA) is often referred to as "amplicon." Usually, the amount of the messenger RNA (mRNA) produced, *i.e.,* the level of gene expression, also increases in the proportion of the number of copies made of the particular gene expressed.

The term "diagnosis" is used herein to refer to the identification of a molecular or pathological state, disease or condition, such as the identification of a molecular subtype of head and neck cancer, colon cancer, or other type of cancer.

The term "prognosis" is used herein to refer to the prediction of the likelihood of cancer-attributable death or progression, including recurrence, metastatic spread, and drug resistance, of a neoplastic disease, such as breast cancer.

The term "prediction" is used herein to refer to the likelihood that a patient will respond either favorably or unfavorably to a drug or set of drugs, and also the extent of those responses, or that a patient will survive, following surgical removal or the primary tumor and/or chemotherapy for a certain period of time without cancer recurrence. The predictive methods of the present invention can be used clinically to make treatment decisions by choosing the most appropriate treatment modalities for any particular patient. The predictive methods of the present invention are valuable tools in predicting if a patient is likely to respond favorably to a treatment regimen, such as surgical intervention, chemotherapy with a given drug or drug combination, and/or radiation therapy, or whether long-term survival of the patient, following sugery and/or termination of chemotherapy or other treatment modalities is likely.

The term "long-term" survival is used herein to refer to survival for at least 3 years, more preferably for at least 8 years, most preferably for at least 10 years following surgery or other treatment.

The term "tumor," as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to, breast cancer, colon cancer, lung cancer, prostate cancer, hepatocellular cancer, gastric cancer, pancreatic cancer, cervical cancer, ovarian cancer, liver cancer, bladder cancer, cancer of the urinary tract, thyroid cancer, renal cancer, carcinoma, melanoma, and brain cancer.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

In the context of the present invention, reference to "at least one," "at least two," "at least five," etc. of the genes listed in any particular gene set means any one or any and all combinations of the genes listed.

The terms "expression threshold," and "defined expression threshold" are used interchangeably and refer to the level of a gene or gene product in question above which the gene or gene product serves as a predictive marker for patient survival without cancer recurrence. The threshold is defined experimentally from clinical studies such as those described in the Example below. The expression threshold can be selected either for maximum sensitivity, or for maximum selectivity, or for minimum error. The determination of the expression threshold for any situation is well within the knowledge of those skilled in the art.

B. Detailed Description

The practice of the present invention will employ, unless otherwise indicated, conventional techniques of molecular biology (including recombinant techniques), microbiology, cell biology, and biochemistry, which are within the skill of the art. Such techniques are explained fully in the literature, such as, "Molecular Cloning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); and "PCR: The Polymerase Chain Reaction", (Mullis et al., eds., 1994).

*1. Gene Expression Profiling*

In general, methods of gene expression profiling can be divided into two large groups: methods based on hybridization analysis of polynucleotides, and methods based on sequencing of polynucleotides. The most commonly used methods known in the art for the quantification of mRNA expression in a sample include northern blotting and *in situ* hybridization (Parker & Barnes, Methods in Molecular Biology 106:247-283 (1999)); RNAse protection assays (Hod, Biotechniques 13:852-854 (1992)); and reverse transcription polymerase chain reaction (RT-PCR) (Weis et al., Trends in Genetics 8:263-264 (1992)). Alternatively, antibodies may be employed that can recognize specific duplexes, including DNA duplexes, RNA duplexes, and DNA-RNA hybrid duplexes or DNA-protein duplexes. Representative methods for sequencing-based gene expression analysis include Serial Analysis of Gene Expression (SAGE), and gene expression analysis by massively parallel signature sequencing (MPSS).

2. *Reverse Transcriptase PCR (RT-PCR)*

Of the techniques listed above, the most sensitive and most flexible quantitative method is RT-PCR, which can be used to compare mRNA levels in different sample populations, in normal and tumor tissues, with or without drug treatment, to characterize patterns of gene expression, to discriminate between closely related mRNAs, and to analyze RNA structure.

The first step is the isolation of mRNA from a target sample. The starting material is typically total RNA isolated from human tumors or tumor cell lines, and corresponding normal tissues or cell lines, respectively. Thus RNA can be isolated from a variety of primary tumors, including breast, lung, colon, prostate, brain, liver, kidney, pancreas, spleen, thymus, testis, ovary, uterus, etc., tumor, or tumor cell lines, with pooled DNA from healthy donors. If the source of mRNA is a primary tumor, mRNA can be extracted, for example, from frozen or archived paraffin-embedded and fixed (e.g. formalin-fixed) tissue samples.

General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MasterPure™ Complete DNA and RNA Purification Kit (EPICENTRE®, Madison, WI), and Paraffin Block RNA Isolation Kit (Ambion, Inc.). Total RNA from tissue samples can be isolated using RNA Stat-60 (Tel-Test). RNA prepared from tumor can be isolated, for example, by cesium chloride density gradient centrifugation.

As RNA cannot serve as a template for PCR, the first step in gene expression profiling by RT-PCR is the reverse transcription of the RNA template into cDNA, followed by its exponential amplification in a PCR reaction. The two most commonly used reverse transcriptases are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labelled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, ABI PRISM 7700™ Sequence Detection System™ (Perkin-Elmer-Applied Biosystems, Foster City, CA, USA), or Lightcycler (Roche Molecular Biochemicals, Mannheim, Germany). In a preferred embodiment, the 5' nuclease procedure is run on a real-time quantitative PCR device such as the ABI PRISM 7700™ Sequence Detection System™. The system consists of a thermocycler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle ($C_t$).

To minimize errors and the effect of sample-to-sample variation, RT-PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPDH) and β-actin.

A more recent variation of the RT-PCR technique is the real time quantitative PCR, which measures PCR product accumulation through a dual-labeled fluorigenic probe (i.e., TaqMan® probe). Real time PCR is compatible both with quantitative competitive PCR, where internal competitor for each target sequence is used for normalization, and with quantitative comparative PCR using a normalization gene contained within the sample, or a housekeeping gene for RT-PCR. For further details see, e.g. Held et al., Genome Research 6:986-994 (1996).

The steps of a representative protocol for profiling gene expression using fixed, paraffin-embedded tissues as the RNA source, including mRNA isolation, purification, primer extension and amplification are given in various published journal articles {for example: T.E. Godfrey et al,. J. Molec. Diagnostics 2: 84-91 [2000]; K. Specht et al., Am. J. Pathol. 158: 419-29 [2001]}. Briefly, a representative process starts with cutting about 10 μm thick sections of paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific promoters followed by RT-PCR.

According to one aspect of the present invention, PCR primers and probes are designed based upon intron sequences present in the gene to be amplified. In this embodiment, the first step in the primer/probe design is the delineation of intron sequences within the genes. This can be done by publicly available software, such as the DNA BLAT software developed by Kent, W.J., Genome Res. 12(4):656-64 (2002), or by the BLAST software including its variations. Subsequent steps follow well established methods of PCR primer and probe design.

In order to avoid non-specific signals, it is important to mask repetitive sequences within the introns when designing the primers and probes. This can be easily accomplished by using the Repeat Masker program available on-line through the Baylor College of Medicine, which screens DNA sequences against a library of repetitive elements and returns a query sequence in which the repetitive elements are masked. The masked intron sequences can then be used to design primer and probe sequences using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers. In: Krawetz S, Misener S (eds) Bioinformtics Methods and Protocols: Methods in Molecular Biology. Humana Press, Totowa, NJ, pp 365-386)

The most important factors considered in PCR primer design include primer length, melting temperature (Tm), and G/C content, specificity, complementary primer sequences, and 3'-end sequence. In general, optimal PCR primers are generally 17-30 bases in length, and contain about 20-80%, such as, for example, about 50-60% G+C bases. Tm's between 50 and 80 °C, e.g. about 50 to 70 °C are typically preferred.

For further guidelines for PCR primer and probe design see, e.g. Dieffenbach, C.W. et al., "General Concepts for PCR Primer Design" in: PCR Primer, A Laboratory Manual, Cold Spring Harbor Laboratory Press, New York, 1995, pp. 133-155; Innis and Gelfand, "Optimization of PCRs" in: PCR Protocols, A Guide to Methods and Applications, CRC Press, London, 1994, pp. 5-11; and Plasterer, T.N. Primerselect: Primer and probe design. Methods Mol. Biol. 70:520-527 (1997).

3. *Microarrays*

Differential gene expression can also be identified, or confirmed using the microarray technique. Thus, the expression profile of breast cancer-associated genes can be measured in either fresh or paraffin-embedded tumor tissue, using microarray technology. In this method, polynucleotide sequences of interest (including cDNAs and oligonucleotides) are plated, or arrayed, on a microchip substrate. The arrayed sequences are then hybridized with specific DNA probes from cells or tissues of interest. Just as in the RT-PCR method, the source of mRNA typically is total RNA isolated from human tumors or tumor cell lines, and corresponding normal tissues or cell lines. Thus RNA can be isolated from a variety of primary tumors or tumor cell lines. If the source of mRNA is a primary tumor, mRNA can be extracted, for example, from frozen or archived paraffin-embedded and fixed (e.g. formalin-fixed) tissue samples, which are routinely prepared and preserved in everyday clinical practice.

In a specific embodiment of the microarray technique, PCR amplified inserts of cDNA clones are applied to a substrate in a dense array. Preferably at least 10,000 nucleotide sequences are applied to the substrate. The microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pairwise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et al., Proc. Natl. Acad. Sci. USA 93(2):106-149 (1996)). Microarray analysis can be performed by commercially available equipment, following manufacturer's protocols, such as by using the Affymetrix GenChip technology, or Incyte's microarray technology.

The development of microarray methods for large-scale analysis of gene expression makes it possible to search systematically for molecular markers of cancer classification and outcome prediction in a variety of tumor types.

4. *Serial Analysis of Gene Expression (SAGE)*

Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculescu et al., Science 270:484-487 (1995); and Velculescu et al., Cell 88:243-51 (1997).

*5. MassARRAY Technology*

The MassARRAY (Sequenom, San Diego, California) technology is an automated, high-throughput method of gene expression analysis using mass spectrometry (MS) for detection. According to this method, following the isolation of RNA, reverse transcription and PCR amplification, the cDNAs are subjected to primer extension. The cDNA-derived primer extension products are purified, and dipensed on a chip array that is pre-loaded with the components needed for MALTI-TOF MS sample preparation. The various cDNAs present in the reaction are quantitated by analyzing the peak areas in the mass spectrum obtained.

6. *Gene Expression Analysis by Massively Parallel Signature Sequencing (MPSS)*

This method, described by Brenner et al., Nature Biotechnology 18:630-634 (2000), is a sequencing approach that combines non-gel-based signature sequencing with *in vitro* cloning of millions of templates on separate 5 $\mu$m diameter microbeads. First, a microbead library of DNA templates is constructed by *in vitro* cloning. This is followed by the assembly of a planar array of the template-containing microbeads in a flow cell at a high density (typically greater than 3 x $10^6$ microbeads/$cm^2$). The free ends of the cloned templates on each microbead are analyzed simultaneously, using a fluorescence-based signature sequencing method that does not require DNA fragment separation. This method has been shown to simultaneously and accurately provide, in a single operation, hundreds of thousands of gene signature sequences from a yeast cDNA library.

7. *Immunohistochemistry*

Immunohistochemistry methods are also suitable for detecting the expression levels of prognostic markers. Thus, antibodies or antisera, preferably polyclonal antisera, and most preferably monoclonal antibodies specific for each marker are used to detect expression. The antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

8. *Proteomics*

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. my mass spectrometry or N-terminal sequencing, and (3) analysis of the data using bioinformatics. Proteomics methods are valuable supplements to other methods of gene expression profiling, and can be used, alone or in combination with other methods, to detect products of prognostic markers.

9. *General Description of the mRNA Isolation, Purification and Amplification*

The steps of a representative protocol for profiling gene expression using fixed, paraffin-embedded tissues as the RNA source, including mRNA isolation, purification, primer extension and amplification are given in various published journal articles {for example: T.E. Godfrey et al. J. Molec. Diagnostics 2: 84-91 [2000]; K. specht et al., Am. J. Pathol. 158: 419-29 [2001]}. Briefly, a representative process starts with cutting about 10 μm thick sections of paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific promoters followed by RT-PCR. Finally, the data are analyzed to identify the best treatment option(s) available to the patient on the basis of the characteristic gene expression pattern identified in the tumor sample examined.

10. *Breast Cancer Gene Set, Assayed Gene Subsequences, and Clinical Application of Gene Expression Data*

An important aspect of the present invention is to use the measured expression of certain genes by breast cancer tissue to provide prognostic information. For this purpose it is necessary to correct for (normalize away) both differences in the amount of RNA assayed and variability in the quality of the RNA used. Therefore, the assay typically measures and incorporates the expression of certain normalizing genes, including well known housekeeping genes, such as GAPDH and Cyp1. Alternatively, normalization can be based on the mean or median signal (Ct) of all of the assayed genes or a large subset thereof (global normalization approach). On a gene-by-gene basis, measured normalized amount of a patient tumor mRNA is compared to the amount found in a breast cancer tissue reference set. The number (N) of breast cancer tissues in this reference set should be sufficiently high to ensure that different reference sets (as a whole) behave essentially the same way. If this condition is met, the identity of the individual breast cancer tissues present in a particular set will have no significant impact on the relative amounts of the genes assayed. Usually, the breast cancer tissue reference set consists of at least about 30, preferably at least about 40 different FPE breast cancer tissue specimens. Unless noted otherwise, normalized expression levels for each mRNA/tested tumor/patient will be expressed as a percentage of the expression level measured in the reference set. More specifically, the reference set of a sufficiently high number (e.g. 40) of tumors yields a distribution of normalized levels of each mRNA species. The level measured in a particular tumor sample to be analyzed falls at some percentile within this range, which can be determined by methods well known in the art. Below, unless noted otherwise, reference to expression levels of a gene assume normalized expression relative to the reference set although this is not always explicitly stated.

Further details of the invention will be described in the following non-limiting Example

Example

A Phase II Study of Gene Expression in 79 Malignant Breast Tumors

A gene expression study was designed and conducted with the primary goal to molecularly characterize gene expression in paraffin-embedded, fixed tissue samples of invasive breast ductal carcinoma, and to explore the correlation between such molecular profiles and disease-free survival.

Study design

Molecular assays were performed on paraffin-embedded, formalin-fixed primary breast tumor tissues obtained from 79 individual patients diagnosed with invasive breast cancer. All patients in the study had 10 or more positive nodes. Mean age was 57 years, and mean clinical tumor size was 4.4 cm. Patients were included in the study only if histopathologic assessment, performed as described in the Materials and Methods section, indicated adequate amounts of tumor tissue and homogeneous pathology.

Materials and Methods

Each representative tumor block was characterized by standard histopathology for diagnosis, semi-quantitative assessment of amount of tumor, and tumor grade. A total of 6 sections (10 microns in thickness each) were prepared and placed in two Costar Brand Microcentrifuge Tubes (Polypropylene, 1.7 mL tubes, clear; 3 sections in each tube). If the tumor constituted less than 30% of the total specimen area, the sample may have been crudely dissected by the pathologist, using gross microdissection, putting the tumor tissue directly into the Costar tube.

If more than one tumor block was obtained as part of the surgical procedure, the block most representative of the pathology was used for analysis.

Gene Expression Analysis

mRNA was extracted and purified from fixed, paraffin-embedded tissue samples, and prepared for gene expression analysis as described in section 9 above.

Molecular assays of quantitative gene expression were performed by RT-PCR, using the ABI PRISM 7900™ Sequence Detection System™ (Perkin-Elmer-Applied Biosystems, Foster City, CA, USA). ABI PRISM 7900™ consists of a thermocycler, laser, charge-coupled device (CCD), camera and computer. The system amplifies samples in a 384-well format on a thermocycler. During amplification, laser-induced fluorescent signal is collected in real-time through fiber optics cables for all 384 wells, and detected at the CCD. The system includes software for running the instrument and for analyzing the data.

Analysis and Results

Tumor tissue was analyzed for 185 cancer-related genes and 7 reference genes. The threshold cycle (CT) values for each patient were normalized based on the median of the 7 reference genes for that particular patient. Clinical outcome data were available for all patients from a review of registry data and selected patient charts.

Outcomes were classified as:

0 died due to breast cancer or to unknown cause or alive with breast cancer recurrence;
1 alive without breast cancer recurrence or died due to a cause other than breast cancer

Analysis was performed by:

1. Analysis of the relationship between normalized gene expression and the binary outcomes of 0 or 1.
2. Analysis of the relationship between normalized gene expression and the time to outcome (0 or 1 as defined above) where patients who were alive without breast cancer recurrence or who died due to a cause other than breast cancer were censored. This approach was used to evaluate the prognostic impact of individual genes and also sets of multiple genes.

*Analysis of patients with invasive breast carcinoma by binary approach*

In the first (binary) approach, analysis was performed on all 79 patients with invasive breast carcinoma. A t test was performed on the groups of patients classified as either no recurrence and no breast cancer related death at three years, versus recurrence, or breast cancer-related death at three years, and the p-values for the differences between the groups for each gene were calculated.

Table 1 lists the 47 genes for which the p-value for the differences between the groups was <0.10. The first column of mean expression values pertains to patients who neither had a metastatic recurrence of nor died from breast cancer. The second column of mean expression values pertains to patients who either had a metastatic recurrence of or died from breast cancer.

Table 1

| | Mean | Mean | t-value | df | p | Valid N | Valid N |
|---|---|---|---|---|---|---|---|
| Bcl2 | -0.15748 | -1.22816 | 4.00034 | 75 | 0.000147 | 35 | 42 |
| PR | -2.67225 | -5.49747 | 3.61540 | 75 | 0.000541 | 35 | 42 |
| IGF1R | -0.59390 | -1.71506 | 3.49158 | 75 | 0.000808 | 35 | 42 |
| BAG1 | 0.18844 | -0.68509 | 3.42973 | 75 | 0.000985 | 35 | 42 |
| CD68 | -0.52275 | 0.10983 | -3.41186 | 75 | 0.001043 | 35 | 42 |
| EstR1 | -0.35581 | -3.00699 | 3.32190 | 75 | 0.001384 | 35 | 42 |
| CTSL | -0.64894 | -0.09204 | -3.26781 | 75 | 0.001637 | 35 | 42 |
| IGFBP2 | -0.81181 | -1.78398 | 3.24158 | 75 | 0.001774 | 35 | 42 |
| GATA3 | 1.80525 | 0.57428 | 3.15608 | 75 | 0.002303 | 35 | 42 |
| TP53BP2 | -4.71118 | -6.09289 | 3.02888 | 75 | 0.003365 | 35 | 42 |

(continued)

| | Mean | Mean | t-value | df | p | Valid N | Valid N |
|---|---|---|---|---|---|---|---|
| EstR1 | 3.67801 | 1.64693 | 3.01073 | 75 | 0.003550 | 35 | 42 |
| CEGP1 | -2.02566 | -4.25537 | 2.85620 | 75 | 0.005544 | 35 | 42 |
| SURV | -3.67493 | -2.96982 | -2.70544 | 75 | 0.008439 | 35 | 42 |
| p27 | 0.80789 | 0.28807 | 2.55401 | 75 | 0.012678 | 35 | 42 |
| Chk1 | -3.37981 | -2.80389 | -2.46979 | 75 | 0.015793 | 35 | 42 |
| BBC3 | -4.71789 | -5.62957 | 2.46019 | 75 | 0.016189 | 35 | 42 |
| ZNF217 | 1.10038 | 0.62730 | 2.42282 | 75 | 0.017814 | 35 | 42 |
| EGFR | -2.88172 | -2.20556 | -2.34774 | 75 | 0.021527 | 35 | 42 |
| CD9 | 1.29955 | 0.91025 | 2.31439 | 75 | 0.023386 | 35 | 42 |
| MYBL2 | -3.77489 | -3.02193 | -2.29042 | 75 | 0.024809 | 35 | 42 |
| HIF1A | -0.44248 | 0.03740 | -2.25950 | 75 | 0.026757 | 35 | 42 |
| GRB7 | -1.96063 | -1.05007 | -2.25801 | 75 | 0.026854 | 35 | 42 |
| pS2 | -1.00691 | -3.13749 | 2.24070 | 75 | 0.028006 | 35 | 42 |
| RIZ1 | -7.62149 | -8.38750 | 2.20226 | 75 | 0.030720 | 35 | 42 |
| ErbB3 | -6.89508 | -7.44326 | 2.16127 | 75 | 0.033866 | 35 | 42 |
| TOP2B | 0.45122 | 0.12665 | 2.14616 | 75 | 0.035095 | 35 | 42 |
| MDM2 | 1.09049 | 0.69001 | 2.10967 | 75 | 0.038223 | 35 | 42 |
| PRAME | -6.40074 | -7.70424 | 2.08126 | 75 | 0.040823 | 35 | 42 |
| GUS | -1.51683 | -1.89280 | 2.05200 | 75 | 0.043661 | 35 | 42 |
| RAD51C | -5.85618 | -6.71334 | 2.04575 | 75 | 0.044288 | 35 | 42 |
| AIB1 | -3.08217 | -2.28784 | -2.00600 | 75 | 0.048462 | 35 | 42 |
| STK15 | -3.11307 | -2.59454 | -2.00321 | 75 | 0.048768 | 35 | 42 |
| GAPDH | -0.35829 | -0.02292 | -1.94326 | 75 | 0.055737 | 35 | 42 |
| FHIT | -3.00431 | -3.67175 | 1.86927 | 75 | 0.065489 | 35 | 42 |
| KRT19 | 2.52397 | 2.01694 | 1.85741 | 75 | 0.067179 | 35 | 42 |
| TS | -2.83607 | -2.29048 | -1.83712 | 75 | 0.070153 | 35 | 42 |
| GSTM1 | -3.69140 | -4.38623 | 1.83397 | 75 | 0.070625 | 35 | 42 |
| G-Catenin | 0.31875 | -0.15524 | 1.80823 | 75 | 0.074580 | 35 | 42 |
| AKT2 | 0.78858 | 0.46703 | 1.79276 | 75 | 0.077043 | 35 | 42 |
| CCNB1 | -4.26197 | -3.51628 | -1.78803 | 75 | 0.077810 | 35 | 42 |
| PI3KC2A | -2.27401 | -2.70265 | 1.76748 | 75 | 0.081215 | 35 | 42 |
| FBXO5 | -4.72107 | -4.24411 | -1.75935 | 75 | 0.082596 | 35 | 42 |
| DR5 | -5.80850 | -6.55501 | 1.74345 | 75 | 0.085353 | 35 | 42 |
| CIAP1 | -2.81825 | -3.09921 | 1.72480 | 75 | 0.088683 | 35 | 42 |
| MCM2 | -2.87541 | -2.50683 | -1.72061 | 75 | 0.089445 | 35 | 42 |
| CCND1 | 1.30995 | 0.80905 | 1.68794 | 75 | 0.095578 | 35 | 42 |
| EIF4E | -5.37657 | -6.47156 | 1.68169 | 75 | 0.096788 | 35 | 42 |

In the foregoing Table 1, negative t-values indicate higher expression, associated with worse outcomes, and, inversely, higher (positive) t-values indicate higher expression associated with better outcomes. Thus, for example, elevated expression of the CD68 gene (t-value = -3.41, CT mean alive< CT mean deceased) indicates a reduced likelihood of disease free survival. Similarly, elevated expression of the BCl2 gene (t-value = 4.00; CT mean alive> CT mean deceased) indicates an increased likelihood of disease free survival.

Based on the data set forth in Table 1, the expression of any of the following genes in breast cancer above a defined expression threshold indicates a reduced likelihood of survival without cancer recurrence following surgery: Grb7, CD68, CTSL, Chk1, Her2, STK15, AIB1, SURV, EGFR, MYBL2, HIF1$\alpha$.

Based on the data set forth in Table 1, the expression of any of the following genes in breast cancer above a defined expression threshold indicates a better prognosis for survival without cancer recurrence following surgery: TP53BP2, PR, Bcl2, KRT14, EstR1, IGFBP2, BAG1, CEGP1, KLK10, $\beta$ Catenin, GSTM1, FHIT, Riz1, IGF1, BBC3, IGFR1, TBP, p27, IRS1, IGF1R, GATA3, CEGP1, ZNF217, CD9, pS2, ErbB3, TOP2B, MDM2, RAD51, and KRT19.

*Analysis of ER positive patients by binary approach*

57 patients with normalized CT for estrogen receptor (ER) >0 (i.e., ER positive patients) were subjected to separate analysis. A t test was performed on the two groups of patients classified as either no recurrence and no breast cancer related death at three years, or recurrence or breast cancer-related death at three years, and the p-values for the differences between the groups for each gene were calculated. Table 2, below, lists the genes where the p-value for the differences between the groups was <0.105. The first column of mean expression values pertains to patients who neither had a metastatic recurrence nor died from breast cancer. The second column of mean expression values pertains to patients who either had a metastatic recurrence of or died from breast cancer.

Table 2

| | Mean | Mean | t-value | df | p | Valid N | Valid N |
|---|---|---|---|---|---|---|---|
| IGF1R | -0.13975 | -1.00435 | 3.65063 | 55 | 0.000584 | 30 | 27 |
| Bcl2 | 0.15345 | -0.70480 | 3.55488 | 55 | 0.000786 | 30 | 27 |
| CD68 | -0.54779 | 0.19427 | -3.41818 | 55 | 0.001193 | 30 | 27 |
| HNF3A | 0.39617 | -0.63802 | 3.20750 | 55 | 0.002233 | 30 | 27 |
| CTSL | -0.66726 | 0.00354 | -3.20692 | 55 | 0.002237 | 30 | 27 |
| TP53BP2 | -4.81858 | -6.44425 | 3.13698 | 55 | 0.002741 | 30 | 27 |
| GATA3 | 2.33386 | 1.40803 | 3.02958 | 55 | 0.003727 | 30 | 27 |
| BBC3 | -4.54979 | -5.72333 | 2.91943 | 55 | 0.005074 | 30 | 27 |
| RAD51C | -5.63363 | -6.94841 | 2.85475 | 55 | 0.006063 | 30 | 27 |
| BAG1 | 0.31087 | -0.50669 | 2.61524 | 55 | 0.011485 | 30 | 27 |
| IGFBP2 | -0.49300 | -1.30983 | 2.59121 | 55 | 0.012222 | 30 | 27 |
| FBXO5 | -4.86333 | -4.05564 | -2.56325 | 55 | 0.013135 | 30 | 27 |
| EstR1 | 0.68368 | -0.66555 | 2.56090 | 55 | 0.013214 | 30 | 27 |
| PR | -1.89094 | -3.86602 | 2.52803 | 55 | 0.014372 | 30 | 27 |
| SURV | -3.87857 | -3.10970 | -2.49622 | 55 | 0.015579 | 30 | 27 |
| CD9 | 1.41691 | 0.91725 | 2.43043 | 55 | 0.018370 | 30 | 27 |
| RB1 | -2.51662 | -2.97419 | 2.41221 | 55 | 0.019219 | 30 | 27 |
| EPHX1 | -3.91703 | -5.85097 | 2.29491 | 55 | 0.025578 | 30 | 27 |
| CEGP1 | -1.18600 | -2.95139 | 2.26608 | 55 | 0.027403 | 30 | 27 |
| CCNB1 | -4.44522 | -3.35763 | -2.25148 | 55 | 0.028370 | 30 | 27 |
| TRAIL | 0.34893 | -0.56574 | 2.20372 | 55 | 0.031749 | 30 | 27 |
| EstR1 | 4.60346 | 3.60340 | 2.20223 | 55 | 0.031860 | 30 | 27 |
| DR5 | -5.71827 | -6.79088 | 2.14548 | 55 | 0.036345 | 30 | 27 |
| MCM2 | -2.96800 | -2.48458 | -2.10518 | 55 | 0.039857 | 30 | 27 |
| Chk1 | -3.46968 | -2.85708 | -2.08597 | 55 | 0.041633 | 30 | 27 |
| p27 | 0.94714 | 0.49656 | 2.04313 | 55 | 0.045843 | 30 | 27 |
| MYBL2 | -3.97810 | -3.14837 | -2.02921 | 55 | 0.047288 | 30 | 27 |
| GUS | -1.42486 | -1.82900 | 1.99758 | 55 | 0.050718 | 30 | 27 |
| P53 | -1.08810 | -1.47193 | 1.92087 | 55 | 0.059938 | 30 | 27 |
| HIF1A | -0.40925 | 0.11688 | -1.91278 | 55 | 0.060989 | 30 | 27 |
| cMet | -6.36835 | -5.58479 | -1.88318 | 55 | 0.064969 | 30 | 27 |
| EGFR | -2.95785 | -2.28105 | -1.86840 | 55 | 0.067036 | 30 | 27 |
| MTA1 | -7.55365 | -8.13656 | 1.81479 | 55 | 0.075011 | 30 | 27 |
| RIZ1 | -7.52785 | -8.25903 | 1.79518 | 55 | 0.078119 | 30 | 27 |
| ErbB3 | -6.62488 | -7.10826 | 1.79255 | 55 | 0.078545 | 30 | 27 |
| TOP2B | 0.54974 | 0.27531 | 1.74888 | 55 | 0.085891 | 30 | 27 |
| EIF4E | -5.06603 | -6.31426 | 1.68030 | 55 | 0.098571 | 30 | 27 |
| TS | -2.95042 | -2.36167 | -1.67324 | 55 | 0.099959 | 30 | 27 |
| STK15 | -3.25010 | -2.72118 | -1.64822 | 55 | 0.105010 | 30 | 27 |

For each gene, a classification algorithm was utilized to identify the best threshold value (CT) for using each gene alone in predicting clinical outcome.

Based on the data set forth in Table 2, expression of the following genes in ER-positive cancer above a defined expression level is indicative of a reduced likelihood of survival without cancer recurrence following surgery: CD68; CTSL; FBXO5; SURV; CCNB1; MCM2; Chk1; MYBL2; HIF1A; cMET; EGFR; TS; STK15. Many of these genes (CD68, CTSL, SURV, CCNB1, MCM2, Chk1, MYBL2, EGFR, and STK15) were also identified as indicators of poor prognosis in the previous analysis, not limited to ER-positive breast cancer. Based on the data set forth in Table 2, expression of the following genes in ER-positive cancer above a defined expression level is indicative of a better prognosis for survival without cancer recurrence following surgery: IGFR1; BC12; HNF3A; TP53BP2; GATA3; BBC3; RAD51C; BAG1; IGFBP2; PR; CD9; RB1; EPHX1; CEGP1; TRAIL; DR5; p27; p53; MTA; RIZ1; ErbB3; TOP2B; EIF4E. Of the latter genes, IGFR1; BC12; TP53BP2; GATA3; BBC3; RAD51C; BAG1; IGFBP2; PR; CD9; CEGP1; DR5; p27; RIZ1; ErbB3; TOP2B; EIF4E have also been identified as indicators of good prognosis in the previous analysis, not limited to ER-positive breast cancer.

*Analysis of ER negative patients by Binary approach*

Twenty patients with normalized CT for estrogen receptor (ER) <1.6 (i.e., ER negative patients) were subjected to separate analysis. A t test was performed on the two groups of patients classified as either no recurrence and no breast cancer related death at three years, or recurrence or breast cancer-related death at three years, and the p-values for the differences between the groups for each gene were calculated. Table 3 lists the genes where the p-value for the differences between the groups was <0.118. The first column of mean expression values pertains to patients who neither had a metastatic recurrence nor died from breast cancer. The second column of mean expression values pertains to patients who either had a metastatic recurrence of or died from breast cancer.

Table 3

| | Mean | Mean | t-value | df | p | Valid N | Valid N |
|---|---|---|---|---|---|---|---|
| KRT14 | -1.95323 | -6.69231 | 4.03303 | 18 | 0.000780 | 5 | 15 |
| KLK10 | -2.68043 | -7.11288 | 3.10321 | 18 | 0.006136 | 5 | 15 |
| CCND1 | -1.02285 | 0.03732 | -2.77992 | 18 | 0.012357 | 5 | 15 |
| Upa | -0.91272 | -0.04773 | -2.49460 | 18 | 0.022560 | 5 | 15 |
| HNF3A | -6.04780 | -2.36469 | -2.43148 | 18 | 0.025707 | 5 | 15 |
| Maspin | -3.56145 | -6.18678 | 2.40169 | 18 | 0.027332 | 5 | 15 |
| CDH1 | -3.54450 | -2.34984 | -2.38755 | 18 | 0.028136 | 5 | 15 |
| HER2 | -1.48973 | 1.53108 | -2.35826 | 18 | 0.029873 | 5 | 15 |
| GRB7 | -2.55289 | 0.00036 | -2.32890 | 18 | 0.031714 | 5 | 15 |
| AKT1 | -0.36849 | 0.46222 | -2.29737 | 18 | 0.033807 | 5 | 15 |
| TGFA | -4.03137 | -5.67225 | 2.28546 | 18 | 0.034632 | 5 | 15 |
| FRP1 | 1.45776 | -1.39459 | 2.27884 | 18 | 0.035097 | 5 | 15 |
| STMY3 | -1.59610 | -0.26305 | -2.23191 | 18 | 0.038570 | 5 | 15 |
| Contig 27882 | -4.27585 | -7.34338 | 2.18700 | 18 | 0.042187 | 5 | 15 |
| A-Catenin | -1.19790 | -0.39085 | -2.15624 | 18 | 0.044840 | 5 | 15 |
| VDR | -4.37823 | -2.37167 | -2.15620 | 18 | 0.044844 | 5 | 15 |
| GRO1 | -3.65034 | -5.97002 | 2.12286 | 18 | 0.047893 | 5 | 15 |
| MCM3 | -3.86041 | -5.55078 | 2.10030 | 18 | 0.050061 | 5 | 15 |
| B-actin | 4.69672 | 5.19190 | -2.04951 | 18 | 0.055273 | 5 | 15 |
| HIF1A | -0.64183 | -0.10566 | -2.02301 | 18 | 0.058183 | 5 | 15 |
| MMP9 | -8.90613 | -7.35163 | -1.88747 | 18 | 0.075329 | 5 | 15 |
| VEGF | 0.37904 | 1.10778 | -1.87451 | 18 | 0.077183 | 5 | 15 |
| PRAME | -4.95855 | -7.41973 | 1.86668 | 18 | 0.078322 | 5 | 15 |
| AIB1 | -3.12245 | -1.92934 | -1.86324 | 18 | 0.078829 | 5 | 15 |
| KRT5 | -1.32418 | -3.62027 | 1.85919 | 18 | 0.079428 | 5 | 15 |
| KRT18 | 1.08383 | 2.25369 | -1.83831 | 18 | 0.082577 | 5 | 15 |
| KRT17 | -0.69073 | -3.56536 | 1.78449 | 18 | 0.091209 | 5 | 15 |
| P14ARF | -1.87104 | -3.36534 | 1.63923 | 18 | 0.118525 | 5 | 15 |

Based on the data set forth in Table 3, expression of the following genes in ER-negative cancer above a defined expression level is indicative of a reduced likelihood of survival without cancer recurrence ($p<0.05$): CCND1; UPA; HNF3A; CDH1; Her2; GRB7; AKT1; STMY3; α-Catenin; VDR; GRO1. Only 2 of these genes (Her2 and Grb7) were also identified as indicators of poor prognosis in the previous analysis, not limited to ER-negative breast cancer. Based on the data set forth in Table 3, expression of the following genes in ER-negative cancer above a defined expression level is indicative of a better prognosis for survival without cancer recurrence (KT14; KLK10; Maspin, TGFα, and FRP1. Of the latter genes, only KLK10 has been identified as an indicator of good prognosis in the previous analysis, not limited to ER-negative breast cancer.

*Analysis of multiple genes and indicators of outcome*

Two approaches were taken in order to determine whether using multiple genes would provide better discrimination between outcomes.

First, a discrimination analysis was performed using a forward stepwise approach. Models were generated that classified outcome with greater discrimination than was obtained with any single gene alone.

According to a second approach (time-to-event approach), for each gene a Cox Proportional Hazards model (see, e.g. Cox, D. R., and Oakes, D. (1984), Analysis of Survival Data, Chapman and Hall, London, New York) was defined with time to recurrence or death as the dependent variable, and the expression level of the gene as the independent variable. The genes that have a p-value < 0.10 in the Cox model were identified. For each gene, the Cox model provides the relative risk (RR) of recurrence or death for a unit change in the expression of the gene. One can choose to partition the patients into subgroups at any threshold value of the measured expression (on the CT scale), where all patients with expression values above the threshold have higher risk, and all patients with expression values below the threshold have lower risk, or vice versa, depending on whether the gene is an indicator of bad (RR>1.01) or good (RR<1.01) prognosis. Thus, any threshold value will define subgroups of patients with respectively increased or decreased risk. The results are summarized in Table 4. The third column, with the heading: exp(coef), shows RR values.

Table 4

| Gene | coef | exp(coef) | se(coef) | z | p |
|---|---|---|---|---|---|
| TP53BP2 | -0.21892 | 0.803386 | 0.068279 | -3.20625 | 0.00134 |
| GRB7 | 0.235697 | 1.265791 | 0.073541 | 3.204992 | 0.00135 |
| PR | -0.10258 | 0.90251 | 0.035864 | -2.86018 | 0.00423 |
| CD68 | 0.465623 | 1.593006 | 0.167785 | 2.775115 | 0.00552 |
| Bcl2 | -0.26769 | 0.765146 | 0.100785 | -2.65603 | 0.00791 |
| KRT14 | -0.11892 | 0.887877 | 0.046938 | -2.53359 | 0.0113 |
| PRAME | -0.13707 | 0.871912 | 0.054904 | -2.49649 | 0.0125 |
| CTSL | 0.431499 | 1.539564 | 0.185237 | 2.329444 | 0.0198 |
| EstR1 | -0.07686 | 0.926018 | 0.034848 | -2.20561 | 0.0274 |
| Chk1 | 0.284466 | 1.329053 | 0.130823 | 2.174441 | 0.0297 |
| IGFBP2 | -0.2152 | 0.806376 | 0.099324 | -2.16669 | 0.0303 |
| HER2 | 0.155303 | 1.168011 | 0.072633 | 2.13818 | 0.0325 |
| BAG1 | -0.22695 | 0.796959 | 0.106377 | -2.13346 | 0.0329 |
| CEGP1 | -0.07879 | 0.924236 | 0.036959 | -2.13177 | 0.033 |
| STK15 | 0.27947 | 1.322428 | 0.132762 | 2.105039 | 0.0353 |
| KLK10 | -0.11028 | 0.895588 | 0.05245 | -2.10248 | 0.0355 |
| B.Catenin | -0.16536 | 0.847586 | 0.084796 | -1.95013 | 0.0512 |
| EstR1 | -0.0803 | 0.922842 | 0.042212 | -1.90226 | 0.0571 |
| GSTM1 | -0.13209 | 0.876266 | 0.072211 | -1.82915 | 0.0674 |
| TOP2A | -0.11148 | 0.894512 | 0.061855 | -1.80222 | 0.0715 |
| AIB1 | 0.152968 | 1.165288 | 0.086332 | 1.771861 | 0.0764 |
| FHIT | -0.15572 | 0.855802 | 0.088205 | -1.7654 | 0.0775 |
| RIZ1 | -0.17467 | 0.839736 | 0.099464 | -1.75609 | 0.0791 |
| SURV | 0.185784 | 1.204162 | 0.106625 | 1.742399 | 0.0814 |
| IGF1 | -0.10499 | 0.900338 | 0.060482 | -1.73581 | 0.0826 |
| BBC3 | -0.1344 | 0.874243 | 0.077613 | -1.73163 | 0.0833 |
| IGF1R | -0.13484 | 0.873858 | 0.077889 | -1.73115 | 0.0834 |

(continued)

| Gene | coef | exp(coef) | se(coef) | z | p |
|---|---|---|---|---|---|
| DIABLO | 0.284336 | 1.32888 | 0.166556 | 1.707148 | 0.0878 |
| TBP | -0.34404 | 0.7089 | 0.20564 | -1.67303 | 0.0943 |
| p27 | -0.26002 | 0.771033 | 0.1564 | -1.66256 | 0.0964 |
| IRS1 | -0.07585 | 0.926957 | 0.046096 | -1.64542 | 0.0999 |

The binary and time-to-event analyses, with few exceptions, identified the same genes as prognostic markers. For example, comparison of Tables 1 and 4 shows that 10 genes were represented in the top 15 genes in both lists. Furthermore, when both analyses identified the same gene at [p<0.10], which happened for 21 genes, they were always concordant with respect to the direction (positive or negative sign) of the correlation with survival/recurrence. Overall, these results strengthen the conclusion that the identified markers have significant prognostic value.

For Cox models comprising more than two genes (multivariate models), stepwise entry of each individual gene into the model is performed, where the first gene entered is preselected from among those genes having significant univariate p-values, and the gene selected for entry into the model at each subsequent step is the gene that best improves the fit of the model to the data. This analysis can be performed with any total number of genes. In the analysis the results of which are shown below, stepwise entry was performed for up to 10 genes.

Multivariate analysis is performed using the following equation:

$$RR=\exp[\text{coef(geneA)} \times \text{Ct(geneA)} + \text{coef(geneB)} \times \text{Ct(geneB)} + \text{coef(geneC)} \times \text{Ct(geneC)} + \ldots\ldots\ldots\ldots\ldots].$$

In this equation, coefficients for genes that are predictors of beneficial outcome are positive numbers and coefficients for genes that are predictors of unfavorable outcome are negative numbers. The "Ct" values in the equation are ΔCts, i.e. reflect the difference between the average normalized Ct value for a population and the normalized Ct measured for the patient in question. The convention used in the present analysis has been that ΔCts below and above the population average have positive signs and negative signs, respectively (reflecting greater or lesser mRNA abundance). The relative risk (RR) calculated by solving this equation will indicate if the patient has an enhanced or reduced chance of long-term survival without cancer recurrence.

*<u>Multivariate gene analysis of 79 patients with invasive breast carcinoma</u>*

A multivariate stepwise analysis, using the Cox Proportional Hazards Model, was performed on the gene expression data obtained for all 79 patients with invasive breast carcinoma. The following ten-gene sets have been identified by this analysis as having particularly strong predictive value of patient survival:

(a) TP53BP2, Bc12, BAD, EPHX1, PDGFRβ, DIABLO, XIAP, YB1, CA9, and KRT8.
(b) GRB7, CD68, TOP2A, Bc12, DIABLO, CD3, ID1, PPM1D, MCM6, and WISP1.
(c) PR, TP53BP2, PRAME, DIABLO, CTSL, IGFBP2, TIMP1, CA9, MMP9, and COX2.
(d) CD68, GRB7, TOP2A, Bcl2, DIABLO, CD3, ID1, PPM1D, MCM6, and WISP1.
(e) Bcl2, TP53BP2, BAD, EPHX1, PDGFRβ, DIABLO, XIAP, YB1, CA9, and KRT8.
(f) KRT14, KRT5, PRAME, TP53BP2, GUS1, AIB1, MCM3, CCNE1, MCM6, and ID1.
(g) PRAME, TP53BP2, EstR1, DIABLO, CTSL, PPM1D, GRB7, DAPK1, BBC3, and VEGFB.
(h) CTSL2, GRB7, TOP2A, CCNB1, Bcl2, DIABLO, PRAME, EMS1, CA9, and EpCAM.
(i) EstR1, TP53BP2, PRAME, DIABLO, CTSL, PPM1D, GRB7, DAPK1, BBC3, and VEGFB.
(k) Chk1, PRAME, p53BP2, GRB7, CA9, CTSL, CCNB1, TOP2A, tumor size, and IGFBP2.
(1) IGFBP2, GRB7, PRAME, DIABLO, CTSL, β-Catenin, PPM1D, Chk1, WISP1, and LOT1.
(m) HER2, TP53BP2, Bcl2, DIABLO, TIMP1, EPHX1, TOP2A, TRAIL, CA9, and AREG.
(n) BAG1, TP53BP2; PRAME, IL6, CCNB1, PAI1, AREG, tumor size, CA9, and Ki67.
(o) CEGP1, TP53BP2, PRAME, DIABLO, Bcl2, COX2, CCNE1, STK15, and AKT2, and FGF18.
(p) STK15, TP53BP2, PRAME, IL6, CCNE1, AKT2, DIABLO, cMet, CCNE2, and COX2.
(q) KLK10, EstR1, TP53BP2, PRAME, DIABLO, CTSL, PPM1D, GRB7, DAPK1, and BBC3.
(r) AIB1, TP53BP2, Bcl2, DIABLO, TIMP1, CD3, p53, CA9, GRB7, and EPHX1
(s) BBC3, GRB7, CD68, PRAME, TOP2A, CCNB1, EPHX1, CTSL GSTM1, and APC.

(t) CD9, GRB7, CD68, TOP2A, Bcl2, CCNB1, CD3, DIABLO, ID1, and PPM1D.
(w) EGFR, KRT14, GRB7, TOP2A, CCNB1, CTSL, Bcl2, TP, KLK10, and CA9.
(x) HIF1α, PR, DIABLO, PRAME, Chk1, AKT2, GRB7, CCNE1, TOP2A, and CCNB1.
(y) MDM2, TP53BP2, DIABLO, Bcl2, AIB1, TIMP1, CD3, p53, CA9, and HER2.
(z) MYBL2, TP53BP2, PRAME, IL6, Bcl2, DIABLO, CCNE1, EPHX1, TIMP1, and CA9.
(aa) p27, TP53BP2, PRAME, DIABLO, Bcl2, COX2, CCNE1, STK15, AKT2, and ID1.
(ab) RAD51, GRB7, CD68, TOP2A, CIAP2, CCNB1, BAG1, IL6, FGFR1, and TP53BP2.
(ac) SURV, GRB7, TOP2A, PRAME, CTSL, GSTM1, CCNB1, VDR, CA9, and CCNE2.
(ad) TOP2B, TP53BP2, DIABLO, Bcl2, TIMP1, AIB1, CA9, p53, KRT8, and BAD.
(ae) ZNF217, GRB7, p53BP2, PRAME, DIABLO, Bcl2, COX2, CCNE1, APC4, and β-Catenin.

While the present invention has been described with reference to what are considered to be the specific embodiments, it is to be understood that the invention is not limited to such embodiments.

| Gene | Accession | Seq |
|---|---|---|
| AIB1 | NM_006534 | GCGGCGAGTTTCCGATTTAAAGCTGAGCTGCGAGGAAAATGGCGGCGGGAGGATCAAAATACTTGCTGGATGGTGGACTCA |
| AKT1 | NM_005163 | CGCTTCTATGGCGCTGAGATTGTGTCAGCCCTGGACTACCTGCACTCGGAGAAGAACGTGGTGTACCGGGA |
| AKT2 | NM_001626 | TCCTGCCACCCTTCAAACCTCAGGTCACGTCCGAGGTCGACACAAGGTACTTCGATGATGAATTTACCGCC |
| APC | NM_000038 | GGACAGCAGGAATGTGTTTCTCCATACAGGTCACGGGGAGCCAATGGTTCAGAAACAAATCGAGTGGGT |
| AREG | NM_001657 | TGTGAGTGAAATGCCTTCTAGTAGTGAACCGTCCTCGGGAGCCGACTATGACTACTCAGAAGAGTATGATAACGAACCACAA |
| B-actin | NM_001101 | CAGCAGATGTGGATCAGCAAGCAGGAGTATGACGAGTCCGGCCCCTCCATCGTCCACCGCAAA TGC |
| B-Catenin | NM_001904 | GGCTCTTGTGCGTACTGTCCTTCGGGCTGGTGACAGGGAAGACATCACTGAGCCTGCCATCTGTGCTCTTCGTCATCTGA |
| BAD | NM_032989 | GGGTCAGGTGCCTCGAGATCGGGCTTGGGCCCAGAGCATGTTCCAGATCCCAGAGTTTGAGCCGAGTGAGCAG |
| BAG1 | NM_004323 | CGTTGTCAGCACTTGGAATACAAGATGGTTGCCGGGTCATGTTAATTGGGAAAAAGAACAGTCCACAGGAAGAGGTTGAAC |
| BBC3 | NM_014417 | CCTGGAGGGTCCTGTACAATCTCATCATGGGACTCCTCCTGCCCTTACCCAGGGCCACAGAGCCCCGAGATGGAGCCCAATTAG |
| Bcl2 | NM_000633 | CAGATGGACCTAGTACCCACTGAGATTTCCACGCCGAAGGACAGCGATGGGAAAAATGCCCTTAAATCATAGG |
| CA9 | NM_001216 | ATCCTAGCCCTGGTTTTTGGCCTCCTTTTTGCTGTCACCAGCGTCGCGTTCCTTGTGCAGATGAGAAGGCAG |
| CCNB1 | NM_031966 | TTCAGGTTGTTGCAGGAGACCATGTACATGACTGTCTCCATTATTGATCGGTTCATGCAGAATAATTGTGTGCCCAAGAAGATG |
| CCND1 | NM_001758 | GCATGTTCGTGGCCTCTAAGATGAAGGAGACCATCCCCCTGACGGCCGAGAAGCTGTGCATCTACACCG |
| CCNE1 | NM_001238 | AAAGAAGATGATGACCGGGTTTACCCAAACTCAACGTGCAAGCCTCGGATTATTGCACCATCCAGAGGCTC |
| CCNE2 | NM_057749 | ATGCTGTGGCTCCTTCCTAACTGGGGCTTTCTTGACATGTAGGTTGCTTGGTAATAACCTTTTTGTATATCACAATTTGGGT |
| CD3z | NM_000734 | AGATGAAGTGGAAGGCGCTTTTCACCGCGGCCATCCTGCAGGCACAGTTGCCGATTACAGAGGCA |
| CD68 | NM_001251 | TGGTTCCCAGCCCTGTGTCCACCTCCAAGCCCAGATTCAGATTCGAGTCATGTACACAACCCAGGGTGGAGGAG |
| CD9 | NM_001769 | GGGCGTGGAACAGTTTATCTCAGACATCTGCCCCAAGAAGGACGTACTCGAAACCTTCACCGTG |
| CDH1 | NM_004360 | TGAGTGTCCCCCGGTATCTTCCCCGCCCTGCCAATCCCGATGAAATTGGAAATTTTATTGATGAAAATCTGAAAGCGGCTG |
| CEGP1 | NM_020974 | TGACAATCAGCACACCTGCATTCACCGCTCGGAAGAGGGCCTGAGCTGCATGAATAAGGATCACGGCTGTAGTCACA |
| Chk1 | NM_001274 | GATAAATTGGTACAAGGGATCAGCTTTTCCCAGCCCACATGTCCTGATCATATGCTTTTGAATAGTCAGTTACTTGGCACCC |
| CIAP1 | NM_001166 | TGCCTGTGGTGGGAAGCTCAGTAACTGGGAACCAAAGGATGATGCTATGTCAGAACACCGGAGGCATTTTCC |
| cIAP2 | NM_001165 | GGATATTTCCGTGGCTCTTATTCAAACTCTCCATCAAATCCTGTAAACTCCAGAGCAAATCAAGATTTTTCTGCCTTGATGAGAAG |
| cMet | NM_000245 | GACATTTCCAGTCCTGCAGTCAATGCCTCTCTGCCCCACCCTTTGTTCAGTGTGGCTGGTGCCACGACAAATGTGTGCGATCGGAG |
| Contig 278 | AK000618 | GGCATCCTGGCCCAAAGTTTCCCAAATCCAGGCGGCTAGAGGCCCACTGCTTCCCAACTACCAGCTGAGGGGGTC |
| COX2 | NM_000963 | TCTGCAGAGTTGGAAGCACTCTATGGTGACATCGATGGTGTGGAGCTGTATCCTGCCCTTCTGGTAGAAAAGCCTCGGC |
| CTSL | NM_001912 | GGGAGGCTTATCTCACTGAGTGAGCAGAATCTGGTAGACTGCTCTGGGCCTCAAGGCAATGAAGGCTGCAATGG |
| CTSL2 | NM_001333 | TGTCTCACTGAGCGAGCAGAATCTGGTGGACTGTTCGCGTCCTCAAGGCAATCAGGGCTGCAATGGT |
| DAPK1 | NM_004938 | CGCTGACATCATGAATGTTCCTCGACCGGCTGGAGGCGAGTTTGGATATGACAAAGACACATCGTTGCTGAAAGAGA |
| DIABLO | NM_019887 | CACAATGGCGGCTCTGAAGAGTTGGCTGTCGCGCAGCGTAACTTCATTCTTCAGGTACAGACAGTGTTTGTGT |
| DRS | NM_003842 | CTCTGAGACAGTGCTTCGATGACTTTGCAGACTTGGTGCCCTTTGACTCCTGGGAGCCGCTCATGAGGAAGTTGGGCCTCATGG |
| EGFR | NM_005228 | TGTCGATGGACTTCCAGAACCACCTGGGCAGCTGCCAAAAGTGTGATCCAAGCTGTCCCAAT |
| EIF4E | NM_001968 | GATCTAAGATGGCGACTGTCGAACCGGAAACCACCCCTACTCCTAATCCCCCGACTACAGAAGAGGAGAAAACGGAATCTAA |

(continued)

| | | |
|---|---|---|
| EMS1 | NM_005231 | GGCAGTGTCACTGAGTCCTTGAAATCCTCCCCTGCCCCGCGGGTCTCTGGATTGGGACGCACAGTGCA |
| EpCAM | NM_002354 | GGGCCCTCCAGAACAATGATGGGCTTTATGATCCTGACTGCGATGAGAGCGGGCTCTTTAAGGCCAAGGCCAAGCAGTGCA |
| EPHX1 | NM_000120 | ACCGTAGGCTCTGCTCTGAATGACTCTCCTGTGGGTCTGGCTGCCTATATTCTAGAGAAGTTTTCCACCTGGACCA |
| ErbB3 | NM_001982 | CGGTTATGTCATGCCAGATACACACCTCAAAGGTACTCCCTCCTCCCGGGAAGGCACCCTTTCTTCAGTGGGTCTCAGTTC |
| EstR1 | NM_00125 | CGTGGTGCCCCTCTATGACCTGCTGCTGGAGATGCTGGACGCCCACCGCCTACATGCGCCCACTAGCC |
| FBXO5 | NM_012177 | GGCTATTCCTCATTTTCTCTACAAAGTGGCCTCAGTGAACATGAAGAAGGTAGCCTCCTGGAGGAGAATTTCGGTGACAGTCTACAATCC |
| FGF18 | NM_003862 | CGGTAGTCAAGTCCGGATCAAGGGCAAGGAGACGGAATTCTACCTGTGCATGAACCGCAAAGGCAAGC |
| FGFR1 | NM_023109 | CACGGGACATTCACCACATCGACTACTATAAAAAGACAACCAACGGCCGACTGCCTGTGAAGTGGATGGCACCC |
| FHIT | NM_002012 | CCAGTGGAGCGCTTCCATGACCTGCGTCCTGATGAAGTGGCCGATTTGTTTCAGACGACCCAGAGAG |
| FRP1 | NM_003012 | TTGGTACCTGTGGGTTAGCATCAAGTTCTCCCCAGGGTAGAATTCAATCAGAGCTCCAGTTTGCATTTGGATGTG |
| G-Catenin | NM_002230 | TCAGCAGCAAGGGCATCATGGAGGAGGATGAGGCCTGCGGGCGCCAGTACACGCTCAAGAAAACCACC |
| GAPDH | NM_002046 | ATTCCACCCATGGCAAATTCCATGGCACCGTCAAGGCTGAGAACGGGAAGCTTGTCATCAATGGAAATCCCATC |
| GATA3 | NM_002051 | CAAAGGAGCTCACTGTGGTGTCTGTGTTCCAACCACTGAATCTGGACCCCATCTGTGAATAAGCCATTCTGACTC |
| GRB7 | NM 005310 | CCATCTGCATCCATCTTGTTTGGGCTCCCCACCCTTGAGAAGTGCCTCAGATAATACCCTGGTGGCC |
| GRO1 | NM_001511 | CGAAAAGATGCTGAACAGTGACAAATCCAACTGACCAGAAGGGAGGAGGAAGCTCACTGGTGGCTGTTCCTGA |
| GSTM1 | NM_000561 | AAGCTATGAGGAAAAGAAGTACACGATGGGGGACGCTCCTGATTATGACAGAAGCCAGTGGCTGAATGAAAAATTCAAGCTGGGCC |
| GUS | NM_000181 | CCCACTCAGTAGCCAAGTCACAATGTTTGGAAAACAGCCCGTTTACTTGAGCAAGACTGATACCACCTGCGTG |
| HER2 | NM_00444B | CGGTGTGAGAAGTGCAGCAAGCCCTGTGCCCGAGTGTGCTATGGTCTGGGCATGGAGCACTTGCGAGAGG |
| HIF1A | NM_001530 | TGAACATAAAGTCTGCAACATGGAAGGTATTGCACTGCACAGGCCACATTCACGTATATGATACCAACAGTAACCAACCTCA |
| HNF3A | NM_004496 | TCCAGGATGTTAGGAACTGTGAAGATGGAAGGGCATGAAACCAGCGACTGGAACAGCTACTACGCAGACACGC |
| ID1 | NM_002165 | AGAACCGCAAGGTGAGCAAGGTGGAGATTCTCCAGCACGTCATCGACTACATCAGGGACCTrCAGTTGGA |
| IGF1 | NM_000618 | TCCGGAGCTGTGATCTAAGGAGGCTGGAGATGTATTGCGCACCCCTCAAGCCTGCCAAGTCAGCTCGCTCTGTCCG |
| IGF1R | NM_000875 | GCATGGTAGCCGAAGATTTCACAGTCAAAATCGGAGATTTTGGTATGACGCGAGATATCTATGAGACAGACTATTACCGGAAA |
| IGFBP2 | NM_000597 | GTGGACAGCACCATGAACATGTTGGGCGGGGGAGGCAGTGCTGGCCGGAAGCCCCTCAAGTCGGGTATGAAGG |
| IL6 | NM_000600 | CCTGAACCTTCCAAAGATGGCTGAAAAAGATGGATGCTTCCAATCTGGATTCAATGAGGAGACTTGCCTGGT |
| IRS1 | NM_005544 | CCACAGCTCACCTTCTGTCAGGTGTCCATCCCAGCTCCAGCCAGCTCCCAGAGAGGAAGAGACTGGCACTGAGG |
| KI-67 | NM_002417 | CGGACTTTGGGTGCGACTTGACGAGCGGTGGTTCGACAAGTGGCCTTGCGGGCCGGATCGTCCCAGTGGAAGAGTTGTAA |
| KLK10 | NM_002776 | GCCCAGAGGCTCCATCGTCCATCCTCTTCCTCCCCAGTCGGCTGAACTCTCCCCTTGTCTGCACTGTTCAAACCTCTG |
| KRT14 | NM_000526 | GGCCTGCTGAGATCAAAGACTACAGTCCCTACTTCAAGACCATTGAGGACCTGAGGAACAAGATTCTCACAGCCACAGTGGAC |
| KRT17 | NM_000422 | CGAGGATTGGTTCTTCAGCAAGACAGAGGAACTGAACCGCGAGGTGGCCACCAACAGTGAGCTGGTGCAGAGT |
| KRT18 | NM_000224 | AGAGATCGAGGCTCTCAAGGAGGAGCTGCTCTTCATGAAGAAGAACCACGAAGAGGAAGTAAAAGGCC |
| KRT19 | NM_002276 | TGAGCGGCAGAATCAGGAGTACCAGCGGCTCATGGACATCAAGTCGCGGCTGGAGCAGGAGA TTGCCACCTACCGCA |
| KRT5 | NM_000424 | TCAGTGGAGAAGGAGTTGGACCAGTCAACATCTCTGTTGTCACAAGCAGTGTTTCCTCTGGATATGGCA |
| KRT8 | NM_002273 | GGATGAAGCTTACATGAACAAGGTAGAGCTGGAGTCTCGCCTGGAAGGGCTGACCGACGAGATCAACTTCCTCAGGCAGCTATATG |

(continued)

| | | |
|---|---|---|
| LOT1 varia | NM_002656 | GGAAAGACCACCTGAAAAACCACCTCCAGACCCACGACCCCAACAAAATGGCCTTTGGGTGTGAGGAGTGTGGGAAGAAGTAC |
| Maspin | NM_002639 | CAGATGGCCACTTTGAGAACATTTTAGCTGACAACAGTGTGAACGACCAGACCAAAATCCTTGTGGTTAATGCTGCC |
| MCM2 | NM_004526 | GACTTTTGCCCGCTACCTTTCATTCCGGCGTGACAACAATGAGCTGTTGCTCTTCATACTGAAGCAGTTAGTGGC |
| MCM3 | NM_002388 | GGAGAACAATCCCCTTGAGACAGAATATGGCCTTTCTGTCTACAAGGATCACCAGACCATCACCATCCAGGAGAT |
| MCM6 | NM_005915 | TGATGGTCCTATGTGTCACATTCATCACAGGTTTCATACCAACACAGGCTTCAGCACTTCCTTTGGTGTGTTTCCTGTCCCA |
| MDM2 | NM_002392 | CTACAGGGACGCCATCGAATCCGGATCTTGATGCTGGTGTAAGTGAACATTCAGGTGATTGGTTGGAT |
| MMP9 | NM_004994 | GAGAACCAATCTCACCGACAGGCAGCTGGCAGAGGAATACCTGTACCGCTATGGTTACACTCGGGTG |
| MTA1 | NM_004689 | CCGCCCTCACCTGAAGAGAAACGCGCTCCTTGGCGGACACTGGGGGAGGAGAGGAAGAAGCGCGGCTAACTTATTCC |
| MYBL2 | NM_002466 | GCCGAGATCGCCAAGATGTTGCCAGGGAGGACAGACAATGCTGTGAAGAATCACTGGAACTCTACCATCAAAAG |
| P14ARF | S79535 | CCCTCGTGCTGATGCTACTGAGGAGCCAGCGTCTAGGGCAGCAGCCGCTTCCTAGAAGACCAGGTCATGATG |
| p27 | NM_004064 | CGGTGGACCACGAAGAGTTAACCCGGGACTTGGAGAAGCACTGCAGAGACA TGGAAGAGGCGAGCC |
| P53 | NM_000546 | CTTTGAACCCTTGCTTGCAATAGGTGTGCGTCAGAAGCACCCAGGACTTCCATTTGCTTTGTCCCGGG |
| PAI1 | NM_000602 | CCGCAACGTGGTTTTCTCACCCTATGGGGTGGCCTCGGTGTTGGCCATGCTCCAGCTGACAACAGGAGGAGAAACCCAGCA |
| PDGFRb | NM_002609 | CCAGCTCTCCTTCCAGCTACAGATCAATGTCCCTGTCCGAGTGCTGGAGCTAAGTGAGAGCCACCC |
| PI3KC2A | NM_002645 | ATACCAATCACCGCACAAACCCAGGCTATTTGTTAAGTCCAGTCACAGCGCAAAGAAACATA TGCGGAGAAAATGCTAGTGTG |
| PPM1D | NM_003620 | GCCATCCGCAAAGGCTTTCTCGCTTGTCACCTTGCCATGTGGAAGAAACTGGCGGAATGGCC |
| PR | NM_000926 | GCATCAGGCTGTCATTATGGTGTCCTTACCTGTGGGAGCTGTAAGGTCTTCTTTAAGAGGGCAATGGAAGGGCAGCACAACTACT |
| PRAME | NM_006115 | TCTCCATATCTGCCTTGCAGAGTCTCCTGCAGCACCTCATCGGGCTGAGCAATCTGACCCACGTGC |
| pS2 | NM_003225 | GCCCTCCCAGTGTGCAAATAAGGGCTGCTGTTTCGACGACACCGTTCGTGGGGTCCCCTGGTGCTTCTATCCTAATACCATCGACG |
| RAD51C | NM_058216 | GAACTTCTTGAGCAGGAGCA TACCCAGGGCTTCATAATCACCTTCTGTICAGCACTAGATGATATTCTIGGGGGTGGA |
| R81 | NM_000321 | CGAAGCCCTTACAAGTTTCCTAGTTCACCCTTACGGATTCCTGGAGGGAACATCTATATTTCACCCCTGAAGAGTCC |
| RIZ1 | NM_012231 | CCAGACGAGCGATTAGAAGCGGCAGCTTGTGAGGTGAATGA TTTGGGGGAAGAGGAGGAGGAGGAAGAGGAGGA |
| STK15 | NM_003600 | CATCTTCCAGGAGGACCACTCTCTGTGGCACCCTGGACTACCTGCCCCCTGAAATGATTGAAGGTCGGA |
| STMY3 | NM_005940 | CCTGGAGGCTGCAACATACCTCAATCCTGTCCCAGGCCGGATCCTCCTGAAGCCCTTTTCGCAGCACTGCTATCCTCCAAAGCCATTGTA |

Table 5B

| | | |
|---|---|---|
| SURV | NM_001168 | TGTTTTGATTCCCGGGCTTACCAGGTGAGAAGTGAGGGAGGAAGAAGGCAGTGTCCCTTTTGCTAGAGCTGACAGCTTTG |
| TBP | NM_003194 | GCCCGAAACGCCGAATATAATCCCAAGCGGTTTGCTGCGGTAATCATGAGGATAAGAGAGCCACG |
| TGFA | NM_003236 | GGTGTGCCACAGACCTTCCTACTTGGCCTGTAATCACCTGTGCAGCCTTTTGTGGGCCTTCAAAACTCTGTCAAGAACTCCGT |
| TIMP1 | NM_003254 | TCCCTGCGGTCCCAGATAGCCTGAATCCTGCCCGGAGTGGAACTGAAGCCTGCACAGTGTCCACCCTGTTCCCAC |
| TOP2A | NM_001067 | AATCCAAGGGGGAGAGTGATGACTTCCATATGGACTTTGACTCAGCTGTGGCTCCTCGGGCAAAATCTGTAC |
| TOP2B | NM_001068 | TGTGGACATCTTCCCCTCAGACTTCCCTACTGAGCCACCTTCTCTGCCACGAACCGGTCGGGCTAG |
| TP | NM_001953 | CTATATGCAGCCAGAGATGTGACAGCCACCGTGGACAGCCTGCCACTCATCACAGCCTCCATTCTCAGTAAGAAACTCGTGG |
| TP53BP2 | NM_005426 | GGGCCAAATATTCAGAAGCTTTTATATCAGAGGACCACCATAGCGGCCATGGAGACCATCTCTGTCCCATCATACCCATCC |
| TRAIL | NM_003810 | CTTCACAGTGCTCCTGCAGTCTCTCTGTGTGGCTGTAACTTACGTGTACTTTACCAACGAGCTGAAGCAGATG |
| TS | NM_001071 | GCCTCGGTGTGCCTTTCAACATCGCCAGCTACGCCCTGCTCACGTACATGATTGCGCACATCACG |
| upa | NM_002658 | GTGGATGTGCCCTGAAGGACAAGCCAGGCGTCTACACGAGAGTCTCACACTTCTTACCCTGGATCCGCAG |
| VDR | NM_000376 | GCCCTGGATTTCAGAAAGAGCCAAGTCTGGATCTGGGACCCTTTCCTTCCTTGCCTGGCTTGTAACT |
| VEGF | NM_003376 | CTGCTGTCTTGGGTGCATTGGAGCCTTGCCTTGCTGCTCTACCTCCACCATGCCAAGTGGTCCCAGGCTGC |
| VEGFB | NM_003377 | TGACGATGGCCTGGAGTGTGTGCCCACTGGGCAGCACCAAGTCCGGATGCAGATCCTCATGATCCGGTACC |
| WISP1 | NM_003882 | AGAGGCATCCATGAACTTCACACTTGCGGGCTGCATCAGCACACGCTCCTATCAACCCAAGTACTGTGGAGTTTG . |
| XIAP | NM_001167 | GCAGTTGGAAGACACAGGAAAGTATCCCCAAATTGCAGATTTATCAACGGCTTTTATCTTGAAAATAGTGCCACGCA |
| YB-1 | NM_004559 | AGACTGTGGAGTTTGATGTTGTTGAAGGAGAAAAGGGTGCGGAGGCAGCAAATGTTACAGGTCCTGGTGGTGTTCC |
| ZNF217 | NM_006526 | ACCCAGTAGCAAGGAGAAGCCCACTCACTGCTCCGAGTGCGGCAAAGCTTTCAGAACCTACCACCAGCTG |

| Gene | Accession | Probe Name | Seq | Len |
|---|---|---|---|---|
| AIB1 | NM_006534 | S1994/AIB1.f3 | GCGGCGAGTTTCCGATTTA | 19 |
| AIB1 | NM_006534 | S1995/AI81.r3 | TGAGTCCACCATCCAGCAAGT | 21 |
| AIB1 | NM_006534 | S5055/AIB1.p3 | ATGGCGGCGGGAGGATCAAAA | 21 |
| AKT1 | NM_005163 | S0010/AKT1.f3 | CGCTTCTATGGCGCTGAGAT | 20 |
| AKT1 | NM_005163 | S0012/AKT1.r3 | TCCCGGTACACCACGTTCTT | 20 |
| AKT1 | NM_005163 | S4776/AKT1.p3 | CAGCCCTGGACTACCTGCACTCGG | 24 |
| AKT2 | NM_001626 | S0828/AKT2.f3 | TCCTGCCACCCTTCAAACC | 19 |
| AKT2 | NM_001626 | S0829/AKT2.r3 | GGCGGTAAATTCATCATCGAA | 21 |
| AKT2 | NM_001626 | S4727/AKT2.p3 | CAGGTCACGTCCGAGGTCGACACA | 24 |
| APC | NM_000038 | S0022/APC.f4 | GGACAGCAGGAATGTGTTTC | 20 |
| APC | NM_000038 | S0024/APC.r4 | ACCCACTCGATTTGTTTCTG | 20 |
| APC | NM_000038 | S4888/APC.p4 | CATTGGCTCCCCGTGACCTGTA | 22 |
| AREG | NM_001657 | S0025/AREG.f2 | TGTGAGTGAAATGCCTTCTAGTAGTGA | 27 |
| AREG | NM_001657 | S0027/AREG.r2 | TTGTGGTTCGTTATCATACTCTTCTGA | 27 |
| AREG | NM_001657 | S4889/AREG.p2 | CCGTCCTCGGGAGCCGACTATGA | 23 |
| B-actin | NM_001101 | S0034/B-acti.f2 | CAGCAGATGTGGATCAGCAAG | 21 |
| B-actin | NM_001101 | S0036/B-acti.r2 | GCATTTGCGGTGGACGAT | 18 |
| B-actin | NM_001101 | S4730/B-acti.p2 | AGGAGTATGACGAGTCCGGCCCC | 23 |
| B-Catenin | NM_001904 | S2150/B-Cate.f3 | GGCTCTTGTGCGTACTGTCCTT | 22 |
| B-Catenin | NM_001904 | S2151/B-Cate.r3 | TCAGATGACGAAGAGCACAGATG | 23 |
| B-Catenin | NM_001904 | S5046/B-Cate.p3 | AGGCTCAGTGATGTCTTCCCTGTCACCAG | 29 |
| BAD | NM_032989 | S2011/BAD.f1 | GGGTCAGGTGCCTCGAGAT | 19 |
| BAD | NM_032989 | S2012/BAD.r1 | CTGCTCACTCGGCTCAAACTC | 21 |
| BAD | NM_032989 | S5058/BAD.p1 | TGGGCCCAGAGCATGTTCCAGATC | 24 |
| BAG1 | NM_004323 | S1386/BAG1.f2 | CGTTGTCAGCACTTGGAATACAA | 23 |
| BAG1 | NM_004323 | S1387/BAG1.r2 | GTTCAACCTCTTCCTGTGGACTGT | 24 |
| BAG1 | NM_004323 | S4731/BAG1.p2 | CCCAATTAACATGACCCGGCAACCAT | 26 |
| BAG1 | NM_014417 | S1584/BBC3.f2 | CCTGGAGGGTCCTGTACAAT | 20 |
| BBC3 | NM_014417 | S1585/BBC3.r2 | CTAATTGGGCTCCATCTCG | 19 |
| BBC3 | NM_014417 | S4890/BBC3.p2 | CATCATGGGACTCCTGCCCTTACC | 24 |
| Bcl2 | NM_000633 | S0043/Bcl2.f2 | CAGATGGACCTAGTACCCACTGAGA | 25 |
| Bcl2 | NM_000633 | S0045/Bcl2.r2 | CCTATGATTTAAGGGCATTTTTCC | 24 |
| Bcl2 | NM_000633 | S4732/Bcl2.p2 | TTCCACGCCGAAGGACAGCGAT | 22 |
| CA9 | NM_001216 | S1398/CA9.f3 | ATCCTAGCCCTGGTTTTTGG | 20 |
| CA9 | NM_001216 | S1399/CA9.r3 | CTGCCTTCTCATCTGCACAA | 20 |
| CA9 | NM_001216 | S4938/CA9.p3 | TTTGCTGTCACCAGCGTCGC | 20 |
| CCNB1 | NM_031966 | S1720/CCNB1.f2 | TTCAGGTTGTTGCAGGAGAC | 20 |
| CCNB1 | NM_031966 | S1721/CCNB1.r2 | CATCTTCTTGGGCACACAAT | 20 |
| CCNB1 | NM_031966 | S4733/CCNB1.p2 | TGTCTCCATTATTGATCGGTTCATGCA | 27 |
| CCND1 | NM_001758 | S0058/CCND1.f3 | GCATGTTCGTGGCCTCTAAGA | 21 |
| CCND1 | NM_001758 | S0060/CCND1.r3 | CGGTGTAGATGCACAGCTTCTC | 22 |
| CCND1 | NM_001758 | S4986/CCND1.p3 | AAGGAGACCATCCCCCTGACGGC | 23 |
| CCNE1 | NM_001238 | S1446/CCNE1.f1 | AAAGAAGATGATGACCGGGTTTAC | 24 |
| CCNE1 | NM_001238 | S1447/CCNE1.r1 | GAGCCTCTGGATGGTGCAAT | 20 |
| CCNE1 | NM_001238 | S4944/CCNE1.p1 | CAAACTCAACGTGCAAGCCTCGGA | 24 |
| CCNE2 | NM_057749 | S1458/CCNE2.f2 | ATGCTGTGGCTCCTTCCTAACT | 22 |
| CCNE2 | NM_057749 | S1459/CCNE2.r2 | ACCCAAATTGTGATATACAAAAAGGTT | 27 |
| CCNE2 | NM_057749 | S4945/CCNE2.p2 | TACCAAGCAACCTACATGTCAAGAAAGCCC | 30 |
| CD3z | NM_000734 | S0064/CD3z.f1 | AGATGAAGTGGAAGGCGCTT | 20 |
| CD3z | NM_000734 | S0066/CD3z.r1 | TGCCTCTGTAATCGGCAACTG | 21 |

(continued)

| Gene | Accession | Probe Name | Seq | Len |
|---|---|---|---|---|
| CD3z | NM_000734 | S4988/CD3z.p1 | CACCGCGGCCATCCTGCA | 18 |
| CD68 | MM_001251 | S0067/CD68.f2 | TGGTTCCCAGCCCTGTGT | 18 |
| CD68 | NM_001251 | S0069/CD68.r2 | CTCCTCCACCCTGGGTTGT | 19 |
| CD68 | NM_001251 | S4734/CD68.p2 | CTCCAAGCCCAGATTCAGATTCGAGTCA | 28 |
| CD9 | NM_001769 | S0686/CD9.f1 | GGGCGTGGAACAGTTTATCT | 20 |
| CD9 | NM_001769 | S0687/CD9.r1 | CACGGTGAAGGTTTCGAGT | 19 |
| CD9 | NM_001769 | S4792/CD9.p1 | AGACATCTGCCCCAAGAAGGACGT | 24 |
| CDH1 | NM_004360 | S0073/CDH1.f3 | TGAGTGTCCCCCGGTATCTTC | 21 |
| CDH1 | NM_004360 | S0075/CDH1.r3 | CAGCCGCTTTCAGATTTTCAT | 21 |
| CDH1 | NM_004360 | S4990/CDH1.p3 | TGCCAATCCCGATGAAATTGGAAATTT | 27 |
| CEGP1 | NM_020974 | S1494/CEGP1.f2 | TGACAATCAGCACACCTGCAT | 21 |
| CEGP1 | NM_020974 | S1495/CEGP1.r2 | TGTGACTACAGCCGTGATCCTTA | 23 |
| CEGP1 | NM_020974 | S4735/CEGP1.p2 | CAGGCCCTCTTCCGAGCGGT | 20 |
| Chk1 | NM_001274 | S1422/Chk1.f2 | GATAAATTGGTACAAGGGATCAGCTT | 26 |
| Chk1 | NM_001274 | S1423/Chk1.r2 | GGGTGCCAAGTAACTGACTATTCA | 24 |
| Chk1 | NM_001274 | S4941/Chk1.p2 | CCAGCCCACATGTCCTGATCATATGC | 26 |
| CIAP1 | NM_001166 | S0764/CIAP1.f2 | TGCCTGTGGTGGGAAGCT | 18 |
| CIAP1 | NM_001166 | S0765/CIAP1.r2 | GGAAAATGCCTCCGGTGTT | 19 |
| CIAP1 | NM_001166 | S4802/CIAP1.p2 | TGACATAGCATCATCCTTTGGTTCCCAGTT | 30 |
| cIAP2 | NM_001165 | S0076/cIAP2.f2 | GGATATTTCCGTGGCTCTTATTCA | 24 |
| cIAP2 | NM_001165 | S0078/cIAP2.r2 | CTTCTCATCAAGGCAGAAAAATCTT | 25 |
| cIAP2 | NM_001165 | S4991/cIAP2.p2 | TCTCCATCAAATCCTGTAAACTCCAGAGCA | 30 |
| cMet | NM_000245 | S0082/cMet.f2 | GACATTTCCAGTCCTGCAGTCA | 22 |
| cMet | NM_000245 | S0084/cMet.r2 | CTCCGATCGCACACATTTGT | 20 |
| cMet | NM_000245 | S4993/cMet.p2 | TGCCTCTCTGCCCCACCCTTTGT | 23 |
| Contig 27882 | AK000618 | S2633/Contig.f3 | GGCATCCTGGCCCAAAGT | 18 |
| Contig 27882 | A000618 | S2634/Contig.r3 | GACCCCCTCAGCTGGTAGTTG | 21 |
| Contig 27882 | AK000618 | S4977/Contig.p3 | CCCAAATCCAGGCGGCTAGAGGC | 23 |
| COX2 | NM_000963 | S0088/COX2.f1 | TCTGCAGAGTTGGAAGCACTCTA | 23 |
| COX2 | NM_000963 | S0090/COX2.r1 | GCCGAGGCTTTTCTACCAGAA | 21 |
| COX2 | NM_000963 | S4995/COX2.p1 | CAGGATACAGCTCCACAGCATCGATGTC | 28 |
| CTSL | NM_001912 | S1303/CTSL.f2 | GGGAGGCTTATCTCACTGAGTGA | 23 |
| CTSL | NM_001912 | S1304/CTSL.r2 | CCATTGCAGCCTTCATTGC | 19 |
| CTSL | NM_001912 | S4899/CTSL.p2 | TTGAGGCCCAGAGCAGTCTACCAGATTCT | 29 |
| CTSL2 | MM_001333 | S4354/CTSL2.f1 | TGTCTCACTGAGCGAGCAGAA | 21 |
| CTSL2 | NM_001333 | S4355/CTSL2.r1 | ACCATTGCAGCCCTGATTG | 19 |
| CTSL2 | MM_001333 | S4356/CTSL2.p1 | CTTGAGGACGCGAACAGTCCACCA | 24 |
| DAPK1 | NM_004938 | S1768/DAPK1.f3 | CGCTGACATCATGAATGTTCCT | 22 |
| DAPK1 | NM_004938 | S1769/DAPK1.r3 | TCTCTTTCAGCAACGATGTGTCTT | 24 |
| DAPK1 | NM_004938 | S4927/DAPK1.p3 | TCATATCCAAACTCGCCTCCAGCCG | 25 |
| DIABLO | NM_019887 | S0808/DIABLO.f1 | CACAATGGCGGCTCTGAAG | 19 |
| DIABLO | NM_019887 | S0809/DIABLO.r1 | ACACAAACACTGTCTGTACCTGAAGA | 26 |
| DIABLO | NM_019887 | S4813/DIABLO.p1 | AAGTTACGCTGCGCGACAGCCAA | 23 |
| DR5 | NM_003842 | S2551/DR5.f2 | CTCTGAGACAGTGCTTCGATGACT | 24 |
| DR5 | NM_003842 | S2552/DR5.r2 | CCATGAGGCCCAACTTCCT | 19 |
| DR5 | NM_003842 | S4979/DR5.p2 | CAGACTTGGTGCCCTTTGACTCC | 23 |
| EGFR | NM_005228 | S0103/EGFR.f2 | TGTCGATGGACTTCCAGAAC | 20 |
| EGFR | NM_005228 | S0105/EGFR.r2 | ATTGGGACAGCTTGGATCA | 19 |
| EGFR | NM_005228 | S4999/EGFR.p2 | CACCTGGGCAGCTGCCAA | 18 |
| EIF4E | NM_001968 | S0106/EIF4E.f1 | GATCTAAGATGGCGACTGTCGAA | 23 |

(continued)

| Gene | Accession | Probe Name | Seq | Len |
|---|---|---|---|---|
| EIF4E | NM_001968 | S0108/EIF4E.r1 | TTAGATTCCGTTTTCTCCTCTTCTG | 25 |
| EIF4E | NM_001968 | S5000/EIF4E.p1 | ACCACCCCTACTCCTAATCCCCCGACT | 27 |
| EMS1 | NM_005231 | S2663/EMS1.f1 | GGCAGTGTCACTGAGTCCTTGA | 22 |
| EMS1 | NM_005231 | S2664/EMS1.r1 | TGCACTGTGCGTCCCAAT | 18 |
| ELMS1 | NM_005231 | S4956/EMS1.p1 | ATCCTCCCCTGCCCCGCG | 18 |
| EpCAM | NM_002354 | S1807/EpCAM.f1 | GGGCCCTCCAGAACAATGAT | 20 |
| EpCAM | NM_002354 | S1808/EpCAM.r1 | TGCACTGCTTGGCCTTAAAGA | 21 |
| EpCAM | NM_002354 | S4984/EpCAM.p1 | CCGCTCTCATCGCAGTCAGGATCAT | 25 |
| EPHX1 | NM_000120 | S1865/EPHX1.f2 | ACCGTAGGCTCTGCTCTGAA | 20 |
| EPHX1 | NM_000120 | S1866/EPHX1.r2 | TGGTCCAGGTGGAAAACTTC | 20 |
| EPHX1 | NM_000120 | S4754/EPHX1.p2 | AGGCAGCCAGACCCACAGGA | 20 |
| ErbB3 | NM_001982 | S0112/ErbB3.f1 | CGGTTATGTCATGCCAGATACAC | 23 |
| ErbB3 | NM_001982 | S0114/ErbB3.r1 | GAACTGAGACCCACTGAAGAAAGG | 24 |
| ErbB3 | NM_001982 | S5002/ErbB3.p1 | CCTCAAAGGTACTCCCTCCTCCCGG | 25 |
| EstR1 | NM_000125 | S0115/EstR1.f1 | CGTGGTGCCCCTCTATGAC | 19 |
| EstR1 | NM_000125 | S0117/EstR1.r1 | GGCTAGTGGGCGCATGTAG | 19 |
| EstR1 | NM_000125 | S4737/EstR1.p1 | CTGGAGATGCTGGACGCCC | 19 |
| FBXO5 | NM_012177 | S2017/FBXO5.r1 | GGATTGTAGACTGTCACCGAAATTC | 25 |
| FBXO5 | NM_012177 | S2018/FBXO5.f1 | GGCTATTCCTCATTTTCTCTACAAAGTG | 28 |
| FBXO5 | NM_012177 | S5061/FBXO5.p1 | CCTCCAGGAGGCTACCTTCTTCATGTTCAC | 30 |
| FGF18 | NM_003862 | S1665/FGF18.f2 | CGGTAGTCAAGTCCGGATCAA | 21 |
| FGF18 | NM_003862 | S1666/FGF18.r2 | GCTTGCCTTTGCGGTTCA | 18 |
| FGF18 | NM_003862 | S4914/FGF18.p2 | CAAGGAGACGGAATTCTACCTGTGC | 25 |
| FGFR1 | NM_023109 | S0818/FGFR1.f3 | CACGGGACATTCACCACATC | 20 |
| FGFR1 | NM_023109 | S0819/FGFR1.r3 | GGGTGCCATCCACTTCACA | 19 |
| FGFR1 | NM_023109 | S4816/FGFR1.p3 | ATAAAAAGACAACCAACGGCCGACTGC | 27 |
| FHIT | NM_002012 | S2443/FHIT.f1 | CCAGTGGAGCGCTTCCAT | 18 |
| FHIT | NM_002012 | S2444/FHIT.r1 | CTCTCTGGGTCGTCTGAAACAA | 22 |
| FHIT | NM_002012 | S2445/FHIT.p1 | TCGGCCACTTCATCAGGACGCAG | 23 |
| FHIT | NM_002012 | S4921/FHIT.p1 | TCGGCCACTTCATCAGGACGCAG | 23 |
| FRP1 | NM_003012 | S1804/FRP1.f3 | TTGGTACCTGTGGGTTAGCA | 20 |
| FRP1 | NM_003012 | S1805/FRP1.r3 | CACATCCAAATGCAAACTGG | 20 |
| FRP1 | NM_003012 | S4983/FRP1.p3 | TCCCCAGGGTAGAATTCAATCAGAGC | 26 |
| G-Catenin | NM_002230 | S2153/G-Cate.f1 | TCAGCAGCAAGGGCATCAT | 19 |
| G-Catenin | NM_002230 | S2154/G-Cate.r1 | GGTGGTTTTCTTGAGCGTGTACT | 23 |
| G-Catenin | NM_002230 | S5044/G-Cate.p1 | CGCCCGCAGGCCTCATCCT | 19 |
| GAPDH | NM_002046 | S0374/GAPDH.f1 | ATTCCACCCATGGCAAATTC | 20 |
| GAPDH | NM_002046 | S0375/GAPDH.r1 | GATGGGATTTCCATTGATGACA | 22 |
| GAPDH | NM_002046 | S4738/GAPDH.p1 | CCGTTCTCAGCCTTGACGGTGC | 22 |
| GATA3 | NM_002051 | S0127/GATA3.f3 | CAAAGGAGCTCACTGTGGTGTCT | 23 |
| GATA3 | NM_002051 | S0129/GATA3.r3 | GAGTCAGAATGGCTTATTCACAGATG | 26 |
| GATA3 | NM_002051 | S5005/GATA3.p3 | TGTTCCAACCACTGAATCTGGACC | 24 |
| GRB7 | NM_005310 | S0130/GRB7.f2 | CCATCTGCATCCATCTTGTT | 20 |
| GRB7 | NM_005310 | S0132/GR87.r2 | GGCCACCAGGGTATTATCTG | 20 |
| GRB7 | NM_005310 | S4726/GRB7.p2 | CTCCCCACCCTTGAGAAGTGCCT | 23 |
| GRO1 | NM_001511 | S0133/GRO1.f2 | CGAAAAGATGCTGAACAGTGACA | 23 |
| GRO1 | NM_001511 | S0135/GRO1.r2 | TCAGGAACAGCCACCAGTGA | 20 |
| GRO1 | NM_001511 | S5006/GRO1.p2 | CTTCCTCCTCCCTTCTGGTCAGTTGGAT | 28 |
| GSTM1 | NM_000561 | S2026/GSTM1.r1 | GGCCCAGCTTGAATTTTTCA | 20 |
| GSTM1 | NM_000561 | S2027/GSTM1.f1 | AAGCTATGAGGAAAAGAAGTACACGAT | 27 |

(continued)

| Gene | Accession | Probe Name | Seq | Len |
|---|---|---|---|---|
| GSTM1 | NM_000561 | S4739/GSTM1.p1 | TCAGCCACTGGCTTCTGTCATAATCAGGAG | 30 |
| GUS | NM_000181 | S0139/GUS.f1 | CCCACTCAGTAGCCAAGTCA | 20 |
| GUS | NM_000181 | S0141/GUS.r1 | CACGCAGGTGGTATCAGTCT | 20 |
| GUS | NM_000181 | S4740/GUS.p1 | TCAAGTAAACGGGCTGTTTTCCAAACA | 27 |
| HER2 | NM_004448 | S0142/HER2.f3 | CGGTGTGAGAAGTGCAGCAA | 20 |
| HER2 | NM_004448 | S0144/HER2.r3 | CCTCTCGCAAGTGCTCCAT | 19 |
| HER2 | NM_004448 | S4729/HER2.p3 | CCAGACCATAGCACACTCGGGCAC | 24 |
| HIF1A | NM_001530 | S1207/HIF1A.f3 | TGAACATAAAGTCTGCAACATGGA | 24 |
| HIF1A | NM_001530 | S1208/HIF1A.r3 | TGAGGTTGGTTACTGTTGGTATCATATA | 28 |
| HIF1A | NM_001530 | S4753/HIF1A.p3 | TTGCACTGCACAGGCCACATTCAC | 24 |
| HNF3A | NM_004496 | S0148/HNF3A.f1 | TCCAGGATGTTAGGAACTGTGAAG | 24 |
| HNF3A | NM_004496 | S0150/HNF3A.r1 | GCGTGTCTGCGTAGTAGCTGTT | 22 |
| HNF3A | NM_004496 | S5008/HNF3A.p1 | AGTCGCTGGTTTCATGCCCTTCCA | 24 |
| ID1 | NM_002165 | S0820/ID1.f1 | AGAACCGCAAGGTGAGCAA | 19 |
| IDI | NM_002165 | S0821/ID1.r1 | TCCAACTGAAGGTCCCTGATG | 21 |
| ID1 | NM_002165 | S4832/ID1.p1 | TGGAGATTCTCCAGCACGTCATCGAC | 26 |
| IGF1 | NM_000618 | S0154/IGF1.f2 | TCCGGAGCTGTGATCTAAGGA | 21 |
| IGF1 | NM_000618 | S0156/IGF1.r2 | CGGACAGAGCGAGCTGACTT | 20 |
| IGF1 | NM_000618 | S5010/IGF1.p2 | TGTATTGCGCACCCCTCAAGCCTG | 24 |
| IGF1R | NM_000875 | S1249/IGF1R.f3 | GCATGGTAGCCGAAGATTTCA’ | 21 |
| IGF1 R | NM_000875 | S1250/IGF1R.r3 | TTTCCGGTAATAGTCTGTCTCATAGATATC | 30 |
| IGF1R | NM_000875 | S4895/IGF1R.p3 | CGCGTCATACCAAAATCTCCGATTTTGA | 28 |
| IGFBP2 | NM_000597 | S1128/IGFBP2.f1 | GTGGACAGCACCATGAACA | 19 |
| IGFBP2 | NM_000597 | S1129/IGFBP2.r1 | CCTTCATACCCGACTTGAGG | 20 |
| IGFBP2 | NM_000597 | S4837/IGFBP2.p1 | CTTCCGGCCAGCACTGCCTC | 20 |
| IL6 | NM_000600 | S0760/IL6.f3 | CCTGAACCTTCCAAAGATGG | 20 |
| IL6 | NM_000600 | S0761/IL6.r3 | ACCAGGCAAGTCTCCTCATT | 20 |
| IL6 | NM_000600 | S4800/IL6.p3 | CCAGATTGGAAGCATCCATCTTTTTCA | 27 |
| IRS1 | NM_005544 | S1943/IRS1.f3 | CCACAGCTCACCTTCTGTCA | 20 |
| IRS1 | NM_005544 | S1944/IRS1.r3 | CCTCAGTGCCAGTCTCTTCC | 20 |
| IRS1 | NM_005544 | S5050/IRS1.p3 | TCCATCCCAGCTCCAGCCAG | 20 |
| Ki-67 | NM_002417 | S0436/Ki-67.f2 | CGGACTTTGGGTGCGACTT | 19 |
| Ki-67 | NM_002417 | S0437/Ki-67.r2 | TTACAACTCTTCCACTGGGACGAT | 24 |
| Ki-67 | NM_002417 | S4741/Ki-67.p2 | CCACTTGTCGAACCACCGCTCGT | 23 |
| KLK10 | NM_002776 | S2624/KLK10.f3 | GCCCAGAGGCTCCATCGT | 18 |
| KLK10 | NM_002776 | S2625/KLK10.r3 | CAGAGGTTTGAACAGTGCAGACA | 23 |
| KLK10 | NM_002776 | S4978/KLK10.p3 | CCTCTTCCTCCCCAGTCGGCTGA | 23 |
| KRT14 | NM_000526 | S1853/KRT14.f1 | GGCCTGCTGAGATCAAAGAC | 20 |
| KRT14 | NM_000526 | S1854/KRT14.r1 | GTCCACTGTGGCTGTGAGAA | 20 |
| KRT14 | NM_000526 | S5037/KRT14.p1 | TGTTCCTCAGGTCCTCAATGGTCTTG | 26 |
| KRT17 | NM_000422 | S0172/KRT17.f2 | CGAGGATTGGTTCTTCAGCAA | 21 |
| KRT17 | NM_000422 | S0174/KRT17.r2 | ATCTGCACCAGCTCACTGTTG | 22 |
| KRT17 | NM_000422 | S5013/KRT17.p2 | CACCTCGCGGTTCAGTTCCTCTGT | 24 |
| KRT18 | NM_000224 | S1710/KRT18.f2 | AGAGATCGAGGCTCTCAAGG | 20 |
| KRT18 | NM_000224 | S1711/KRT18.r2 | GGCCTTTTACTTCCTCTTCG | 20 |
| KRT18 | NM_000224 | S4762/KRT18.p2 | TGGTTCTTCTTCATGAAGAGCAGCTCC | 27 |
| KRT19 | NM_002276 | S1515/KRT19.f3 | TGAGCGGCAGAATCAGGAGTA | 21 |
| KRT19 | NM_002276 | S1516/KRT19.r3 | TGCGGTAGGTGGCAATCTC | 19 |
| KRT19 | NM_002276 | S4866/KRT19.p3 | CTCATGGACATCAAGTCGCGGCTG | 24 |
| KRT5 | NM_000424 | S0175/KRT5.f3 | TCAGTGGAGAAGGAGTTGGA | 20 |

(continued)

| Gene | Accession | Probe Name | Seq | Len |
|---|---|---|---|---|
| KRT5 | NM_000424 | S0177/KRT5.r3 | TGCCATATCCAGAGGAAACA | 20 |
| KRT5 | NM_000424 | S5015/KRT5.p3 | CCAGTCAACATCTCTGTTGTCACAAGCA | 28 |
| KRT8 | NM_002273 | S2588/KRT8.f3 | GGATGAAGCTTACATGAACAAGGTAGA | 27 |
| KRT8 | NM_002273 | S2589/KRT8.r3 | CATATAGCTGCCTGAGGAAGTTGAT | 25 |
| KRT8 | NM_002273 | S4952/KRT8.p3 | CGTCGGTCAGCCCTT'CCAGGC | 21 |
| LOT1 variant 1 | NM_002656 | S0692/LOT1 v.f2 | GGAAAGACCACCTGAAAAACCA | 22 |
| LOT1 variant 1 | NM_002656 | S0693/LOT1 v.r2 | GTACTTCTTCCCACACTCCTCACA | 24 |
| LOT1 variant 1 | NM_002656 | S4793/LOT1 v.p2 | ACCCACGACCCCAACAAAATGGC | 23 |
| Maspin | NM_002639 | S0836/Maspin.f2 | CAGATGGCCACTTTGAGAACATT | 23 |
| Maspin | NM_002639 | S0837/Maspin.r2 | GGCAGCATTAACCACAAGGATT | 22 |
| Maspin | NM_002639 | S4835/Maspin.p2 | AGCTGACAACAGTGTGAACGACCAGACC | 28 |
| MCM2 | NM_004526 | S1602/MCM2.f2 | GACTTTTGCCCGCTACCTTTC | 21 |
| MCM2 | NM_004526 | S1603/MCM2.r2 | GCCACTAACTGCTTCAGTATGAAGAG | 26 |
| MCM2 | NM_004526 | S4900/MCM2.p2 | ACAGCTCATTGTTGTCACGCCGGA | 24 |
| MCM3 | NM_002388 | S1524/MCM3.f3 | GGAGAACAATCCCCTTGAGA | 20 |
| MCM3 | NM_002388 | S1525/MCM3.r3 | ATCTCCTGGATGGTGATGGT | 20 |
| MCM3 | NM_002388 | S4870/MCM3.p3 | TGGCCTTTCTGTCTACAAGGATCACCA | 27 |
| MCM6 | NM_005915 | S1704/MCM6.f3 | TGATGGTCCTATGTGTCACATTCA | 24 |
| MCM6 | NM_005915 | S1705/MCM6.r3 | TGGGACAGGAAACACACCAA | 20 |
| MCM6 | NM_005915 | S4919/MCM6.p3 | CAGGTTTCATACCAACACAGGCTTCAGCAC | 30 |
| MDM2 | NM_002392 | S0830/MDM2.f1 | CTACAGGGACGCCATCGAA | 19 |
| MDM2 | NM_002392 | S0831/MDM2.r1 | ATCCAACCAATCACCTGAATGTT | 23 |
| MDM2 | NM_002392 | S4834/MDM2.p1 | CTTACACCAGCATCAAGATCCGG | 23 |
| MMP9 | NM_004994 | S0656/MMP9.f1 | GAGAACCAATCTCACCGACA | 20 |
| MMP9 | NM_004994 | S0657/MMP9.r1 | CACCCGAGTGTAACCATAGC | 20 |
| MMP9 | NM_004994 | S4760/MMP9.p1 | ACAGGTATTCCTCTGCCAGCTGCC | 24 |
| MTA1 | NM_004689 | S2369/MTA1.f1 | CCGCCCTCACCTGAAGAGA | 19 |
| MTA1 | NM_004689 | S2370/MTA1.r1 | GGAATAAGTTAGCCGCGCTTCT | 22 |
| MTA1 | NM_004689 | S4855/MTA1.p1 | CCCAGTGTCCGCCAAGGAGCG | 21 |
| MYBL2 | NM_002466 | S3270/MYBL2.f1 | GCCGAGATCGCCAAGATG | 18 |
| MYBL2 | NM_002466 | S3271/MYBL2.r1 | CTTTTGATGGTAGAGTTCCAGTGATTC | 27 |
| MYBL2 | NM_002466 | S4742/MYBL2.p1 | CAGCATTGTCTGTCCTCCCTGGCA | 24 |
| P14ARF | S78535 | S2842/P14ARF.f1 | CCCTCGTGCTGATGCTACT | 19 |
| P14ARF | S78535 | S2843/P14ARF.r1 | CATCATGACCTGGTCTTCTAGG | 22 |
| P14ARF | S78535 | S4971/P14ARF.p1 | CTGCCCTAGACGCTGGCTCCTC | 22 |
| p27 | NM_004064 | S0205/p27.f3 | CGGTGGACCACGAAGAGTTAA | 21 |
| p27 | NM_004064 | S0207/p27.r3 | GGCTCGCCTCTTCCATGTC | 19 |
| p27 | NM_004064 | S4750/p27.p3 | CCGGGACTTGGAGAAGCACTGCA | 23 |
| P53 | NM_000546 | S0208/P53.f2 | CTTTGAACCCTTGCTTGCAA | 20 |
| P53 | NM_000546 | S0210/P53.r2 | CCCGGGACAAAGCAAATG | 18 |
| P53 | NM_000546 | S5065/P53.p2 | AAGTCCTGGGTGCTTCTGACGCACA | 25 |
| PAI1 | NM_000602 | S0211/PAI.1.f3 | CCGCAACGTGGTTTTCTCA | 19 |
| PAI1 | NM_000602 | S0213/PAI1.r3 | TGCTGGGTTTCTCCTCCTGTT | 21 |
| PAI1 | NM_000602 | S5066/PAI1.p3 | CTCGGTGTTGGCCATGCTCCAG | 22 |
| PDGFRb | NM_002609 | S1346/PDGFRb.f3 | CCAGCTCTCCTTCCAGCTAC | 20 |
| PDGFRb | NM_002609 | S1347/PDGFRb.r3 | GGGTGGCTCTCACTTAGCTC | 20 |
| PDGFRb | NM_002609 | S4931/PDGFRb.p3 | ATCAATGTCCCTGTCCGAGTGCTG | 24 |
| PI3KC2A | NM_002645 | S2020/PI3KC2.r1 | CACACTAGCATTTTCTCCGCATA | 23 |
| PI3KC2A | NM_002645 | S2021/PI3KC2.f1 | ATACCAATCACCGCACAAACC | 21 |
| PI3KC2A | NM_002645 | S5062/PI3KC2.p1 | TGCGCTGTGACTGGACTTAACAAATAGCCT | 30 |

(continued)

| Gene | Accession | Probe Name | Seq | Len |
|---|---|---|---|---|
| PPM1D | NM_003620 | S3159/PPM1D.f1 | GCCATCCGCAAAGGCTTT | 18 |
| PPM1D | NM_003620 | S3160/PPM1D.r1 | GGCCATTCCGCCAGTTTC | 18 |
| PPM1D | NM_003620 | S4856/PPM1D.p1 | TCGCTTGTCACCTTGCCATGTGG | 23 |
| PR | NM_000926 | S1336/PR.f6 | GCATCAGGCTGTCATTATGG | 20 |
| PR | NM_000926 | S1337/PR.r6 | AGTAGTTGTGCTGCCCTTCC | 20 |
| PR | NM_000926 | S4743/PR.p6 | TGTCCTTACCTGTGGGAGCTGTAAGGTC | 28 |
| PRAME | NM_006115 | S1985/PRAME.f3 | TCTCCATATCTGCCTTGCAGAGT | 23 |
| PRAME | NM_006115 | S1986/PRAME.r3 | GCACGTGGGTCAGATTGCT | 19 |
| PRAME | NM_006115 | S4756/PRAME.p3 | TCCTGCAGCACCTCATCGGGCT | 22 |
| pS2 | NM_003225 | S0241/pS2.f2 | GCCCTCCCAGTGTGCAAAT | 19 |
| pS2 | NM_003225 | S0243/pS2.r2 | CGTCGATGGTATTAGGATAGAAGCA | 25 |
| pS2 | NM_003225 | S5026/pS2.p2 | TGCTGTTTCGACGACACCGTTCG | 23 |
| RAD51C | NM_058216 | S2606/RAD51C.f3 | GAACTTCTTGAGCAGGAGCATACC | 24 |
| RAD51C | NM_058216 | S2607/RAD51C.r3 | TCCACCCCCAAGAATATCATCTAGT | 25 |
| RAD51C | NM_058216 | S4764/RAD51C.p3 | AGGGCTTCATAATCACCTTCTGTTC | 25 |
| RB1 | NM_000321 | S2700/RB1.f1 | CGAAGCCCTTACAAGTTTCC | 20 |
| RB1 | NM_000321 | S2701/RB1.r1 | GGACTCTTCAGGGGTGAAAT | 20 |
| RB1 | NM_000321 | S4765/RB1.p1 | CCCTTACGGATTCCTGGAGGGAAC | 24 |
| RIZ1 | NM_012231 | S1320/RIZ1.f2 | CCAGACGAGCGATTAGAAGC | 20 |
| RIZ1 | NM_012231 | S1321/RIZ1.r2 | TCCTCCTCTTCCTCCTCCTC | 20 |
| RIZ1 | NM_012231 | S4761/RIZ1.p2 | TGTGAGGTGAATGATTTGGGGGA | 23 |
| STK15 | NM_003600 | S0794/STK15.f2 | CATCTTCCAGGAGGACCACT | 20 |
| STK15 | NM_003600 | S0795/STK15.r2 | TCCGACCTTCAATCATTTCA | 20 |
| STK15 | NM_003600 | S4745/STK15.p2 | CTCTGTGGCACCCTGGACTACCTG | 24 |
| STMY3 | NM_005940 | S2067/STMY3.f3 | CCTGGAGGCTGCAACATACC | 20 |
| STMY3 | NM_005940 | S2068/STMY3.r3 | TACAATGGCTTTGGAGGATAGCA | 23 |
| STMY3 | NM_005940 | S4746/STMY3.p3 | ATCCTCCTGAAGCCCTTTTCGCAGC | 25 |
| SURV | NM_001168 | S0259/SURV.f2 | TGTTTTGATTCCCGGGCTTA | 20 |
| SURV | NM_001168 | S0261/SURV.r2 | CAAAGCTGTCAGCTCTAGCAAAAG | 24 |
| SURV | NM_001168 | S4747/SURV.p2 | TGCCTTCTTCCTCCCTCACTTCTCACCT | 28 |
| TBP | NM_003194 | S0262/TBP.f1 | GCCCGAAACGCCGAATATA | 19 |
| TBP | NM_003194 | S0264/TBP.r1 | CGTGGCTCTCTTATCCTCATGAT | 23 |
| TBP | NM_003194 | S4751/TBP.p1 | TACCGCAGCAAACCGCTTGGG | 21 |
| TGFA | NM_003236 | S0489/TGFA.f2 | GGTGTGCCACAGACCTTCCT | 20 |
| TGFA | NM_003236 | S0490/TGFA.r2 | ACGGAGTTCTTGACAGAGTTTTGA | 24 |
| TGFA | NM_003236 | S4768/TGFA.p2 | TTGGCCTGTAATCACCTGTGCAGCCTT | 27 |
| TIMP1 | NM_003254 | S1695/TIMP1.f3 | TCCCTGCGGTCCCAGATAG | 19 |
| TIMP1 | NM_003254 | S1696/TIMP1.r3 | GTGGGAACAGGGTGGACACT | 20 |
| TIMP1 | NM_003254 | S4918/TIMP1.p3 | ATCCTGCCCGGAGTGGAACTGAAGC | 25 |
| TOP2A | NM_001067 | S0271/TOP2A.f4 | AATCCAAGGGGGAGAGTGAT | 20 |
| TOP2A | NM_001067 | S0273/TOP2A.r4 | GTACAGATTTTGCCCGAGGA | 20 |
| TOP2A | NM_001067 | S4777/TOP2A.p4 | CATATGGACTTTGACTCAGCTGTGGC | 26 |
| TOP2B | NM_001068 | S0274/TOP2B.f2 | TGTGGACATCTTCCCCTCAGA | 21 |
| TOP28 | NM_001068 | S0276/TOP2B.r2 | CTAGCCCGACCGGTTCGT | 18 |
| TOP2B | M_001068 | S4778/TOP2B.p2 | TTCCCTACTGAGCCACCTTCTCTG | 24 |
| TP | NM_001953 | S0277/TP.f3 | CTATATGCAGCCAGAGATGTGACA | 24 |
| TP | NM_001953 | S0279/TP.r3 | CCACGAGTTTCTTACTGAGAATGG | 24 |
| TP | NM_001953 | S4779/TP.p3 | ACAGCCTGCCACTCATCACAGCC | 23 |
| TP53BP2 | NM_005426 | S1931/TP53BP.f2 | GGGCCAAATATTCAGAAGC | 19 |
| TP53BP2 | NM_005426 | S1932/TP53BP.r2 | GGATGGGTATGATGGGACAG | 20 |

(continued)

| Gene | Accession | Probe Name | Seq | Len |
|---|---|---|---|---|
| TP53BP2 | NM_005426 | S5049/TP53BP.p2 | CCACCATAGCGGCCATGGAG | 20 |
| TRAIL | NM_003810 | S2539/TRAIL.f1 | CTTCACAGTGCTCCTGCAGTCT | 22 |
| TRAIL | NM_003810 | S2540/TRAIL.r1 | CATCTGCTTCAGCTCGTTGGT | 21 |
| TRAIL | NM_003810 | S4980/TRAIL.p1 | AAGTACACGTAAGTTACAGCCACACA | 26 |
| TS | NM_001071 | S0280/TS.f1 | GCCTCGGTGTGCCTTTCA | 18 |
| TS | NM_001071 | S0282/TS.r1 | CGTGATGTGCGCAATCATG | 19 |
| TS | NM_001071 | S4780/TS.p1 | CATCGCCAGCTACGCCCTGCTC | 22 |
| upa | NM_002658 | S0283/upa.f3 | GTGGATGTGCCCTGAAGGA | 19 |
| upa | NM_002658 | S0285/upa.r3 | CTGCGGATCCAGGGTAAGAA | 20 |

Table 6F

| | | | | |
|---|---|---|---|---|
| upa | NM_002658 | S4769/upa.p3 | AAGCCAGGCGTCTACACGAGAGTCTCAC | 28 |
| VDR | NM_000376 | S2745/VDR.f2 | GCCCTGGATTTCAGAAAGAG | 20 |
| VDR | NM_000376 | S2746/VDR.r2 | AGTTACAAGCCAGGGAAGGA | 20 |
| VDR | NM_000376 | S4962/VDR.p2 | CAAGTCTGGATCTGGGACCCTTTCC | 25 |
| VEGF | NM_003376 | S0286/VEGF.f1 | CTGCTGTCTTGGGTGCATTG | 20 |
| VEGF | NM_003376 | S0288/VEGF.r1 | GCAGCCTGGGACCACTTG | 18 |
| VEGF | NM_003376 | S4782/VEGF.p1 | TTGCCTTGCTGCTCTACCTCCACCA | 25 |
| VEGFB | NM_003377 | S2724/VEGFB.f1 | TGACGATGGCCTGGAGTGT | 19 |
| VEGFB | NM_003377 | S2725/VEGFB.r1 | GGTACCGGATCATGAGGATCTG | 22 |
| VEGFB | NM_003377 | S4960/VEGFB.p1 | CTGGGCAGCACCAAGTCCGGA | 21 |
| WISP1 | NM_003882 | S1671/WISP1.f1 | AGAGGCATCCATGAACTTCACA | 22 |
| WISP1 | NM_003882 | S1672/WISP1.r1 | CAAACTCCACAGTACTTGGGTTGA | 24 |
| WISP1 | NM_003882 | S4915/WISP1.p1 | CGGGCTGCATCAGCACACGC | 20 |
| XIAP | NM_001167 | S0289/XIAP.f1 | GCAGTTGGAAGACACAGGAAAGT | 23 |
| XIAP | NM_001167 | S0291/XIAP.r1 | TGCGTGGCACTATTTTCAAGA | 21 |
| XIAP | NM_001167 | S4752/XIAP.p1 | TCCCCAAATTGCAGATTTATCAACGGC | 27 |
| YB-1 | NM_004559 | S1194/YB-1.f2 | AGACTGTGGAGTTTGATGTTGTTGA | 25 |
| YB-1 | NM_004559 | S1195/YB-1.r2 | GGAACACCACCAGGACCTGTAA | 22 |
| YB-1 | NM_004559 | S4843/YB-1.p2 | TTGCTGCCTCCGCACCCTTTTCT | 23 |
| ZNF217 | NM_006526 | S2739/ZNF217.f3 | ACCCAGTAGCAAGGAGAAGC | 20 |
| ZNF217 | NM_006526 | S2740/ZNF217.r3 | CAGCTGGTGGTAGGTTCTGA | 20 |
| ZNF217 | NM_006526 | S4961/ZNF217.p3 | CACTCACTGCTCCGAGTGCGG | 21 |

## Claims

1. A method of predicting the likelihood of long-term survival of a breast cancer patient without recurrence of breast cancer, comprising
determining a level of an RNA transcript of MYBL2 in a breast cancer tumour sample obtained from said patient;
normalizing said level of the RNA transcript to obtain a normalized expression level of MYBL2;
wherein increased normalized expression level of MYBL2 compared to the normalized level of the RNA transcript of MYBL2 in a breast cancer tissue reference set indicates a decreased likelihood of long-term survival without breast cancer recurrence.

2. A method as claimed in claim 1, further comprising
determining a normalized expression level of an RNA transcript of at least one gene selected from GRB7, CD68, CTSL, Chkl, AIB1, CCNB1, MCM2, FBXO5, Her2, STK15, SURV, EGFR, HIF1$\alpha$, and TS, or their expression products;
wherein increased normalized expression levels of the at least one gene indicates a decreased likelihood of long-term survival without breast cancer recurrence.

3. A method as claimed in claim 1, wherein the breast cancer is invasive breast carcinoma.

4. A method as claimed in claim 1, wherein the breast cancer tumour sample is a fixed, wax-embedded tissue specimen.

5. A method as claimed in claim 1, wherein the breast cancer is estrogen-receptor positive breast cancer.

6. A method as claimed in claim 1, wherein the MYBL2 RNA transcript level is determined by quantitative reverse-transcription polymerase chain reaction (qRT-PCR).

7. A method as claimed in claim 1, further comprises providing a report based on the normalized expression level of MYBL2.

8. A method as claimed claim 7, wherein the report includes a prediction of the likelihood of long-term survival of the patient without breast cancer recurrence.

## Patentansprüche

1. Verfahren zur Prognose der Wahrscheinlichkeit des langfristigen Überlebens eines Brustkrebspatienten ohne ein Wiederauftreten von Brustkrebs, umfassend
Bestimmen eines Niveaus eines RNA-Transkripts von MYBL2 in einer dem besagten Patienten entnommenen Brustkrebstumorprobe;
Normalisieren des besagten Niveaus des RNA-Transkripts, um ein normalisiertes Expressionsniveau von MYBL2 zu erhalten;
worin ein erhöhtes normalisiertes Expressionsniveau von MYBL2 im Vergleich zum normalisierten Niveau des RNA-Transkripts von MYBL2 in einem Brustkrebsgewebe-Referenzsatz auf eine verringerte Wahrscheinlichkeit des langfristigen Überlebens ohne ein Wiederauftreten von Brustkrebs hindeutet.

2. Verfahren nach Anspruch 1, ferner umfassend
Bestimmen eines normalisierten Expressionsniveaus eines RNA-Transkripts von mindestens einem Gen, das aus GRB7, CD68, CTSL, Chk1, AIB1, CCNB1, MCM2, FBXO5, Her2, STK15, SURV, EGFR, HIF1$\alpha$ und TS oder deren Expressionsprodukten ausgewählt wird;
worin erhöhte normalisierte Expressionsniveaus des mindestens einen Gens auf eine verringerte Wahrscheinlichkeit des langfristigen Überlebens ohne ein Wiederauftreten von Brustkrebs hindeuten.

3. Verfahren nach Anspruch 1, worin der Brustkrebs ein invasives Brustkarzinom ist.

4. Verfahren nach Anspruch 1, worin die Brustkrebstumorprobe eine fixierte, in Wachs eingebettete Gewebeprobe ist.

5. Verfahren nach Anspruch 1, worin der Brustkrebs ein Östrogenrezeptor-positiver Brustkrebs ist.

6. Verfahren nach Anspruch 1, worin das Niveau des RNA-Transkripts von MYBL2 durch quantitative Reverse-Transcription-Polymerase-Kettenreaktion (qRT-PCR) bestimmt wird.

7. Verfahren nach Anspruch 1, ferner umfassend das Bereitstellen eines auf dem normalisierten Expressionsniveau von MYBL2 beruhenden Berichts.

8. Verfahren nach Anspruch 7, worin der Bericht eine Prognose der Wahrscheinlichkeit des langfristigen Überlebens eines Brustkrebspatienten ohne ein Wiederauftreten von Brustkrebs beinhaltet.

## Revendications

1. Procédé de prédiction de la probabilité de survie à long terme d'un patient qui a été atteint du cancer du sein sans récurrence du cancer du sein, comprenant
la détermination du taux d'une transcription d'ARN de MYBL2 dans un prélèvement de tumeur de cancer du sein obtenu dudit patient ;
la normalisation dudit taux de la transcription d'ARN pour obtenir un taux d'expression normalisé de MYBL2 ;
dans lequel un taux d'expression normalisé augmenté de MYBL2 comparé au taux normalisé de la transcription d'ARN de MYBL2 dans un tissu de cancer du sein de référence indique une diminution de la probabilité de survie à long terme sans récurrence du cancer du sein.

2. Procédé selon la revendication 1, comprenant en outre la détermination d'un taux d'expression normalisé d'une transcription d'ARN d'au moins un gène sélectionné parmi GRB7, CD68, CTSL, Chkl, AIB1, CCNB1, MCM2, FBX05, Her2, STK15, SURV, EGFR, HIF1 a et TS, ou leurs produits d'expression ;
dans lequel des taux d'expression normalisés augmentés d'au moins un gène indique une diminution de la probabilité de survie à long terme sans récurrence du cancer du sein.

3. Procédé selon la revendication 1, dans lequel le cancer du sein est un carcinome invasif du sein.

**4.** Procédé selon la revendication 1, dans lequel le prélèvement de tumeur du cancer du sein est un échantillon de tissu fixé, inclus dans de la cire.

**5.** Procédé selon la revendication 1, dans lequel le cancer du sein est un cancer du sein positif au récepteur de l'oestrogène.

**6.** Procédé selon la revendication 1, dans lequel le taux de la transcription d'ARN de MYBL2 est déterminé par une réaction en chaîne par polymérase de transcription inverse quantitative (qRT-PCR).

**7.** Procédé selon la revendication 1, qui comprend en outre la fourniture d'un rapport basé sur le taux d'expression normalisé de MYBL2.

**8.** Procédé selon la revendication 7, dans lequel le rapport inclut une prédiction de la probabilité de survie à long terme du patient sans récurrence du cancer du sein.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0210436 A
- US 60440861 B


### Non-patent literature cited in the description


- **GOLUB et al.** *Science,* 1999, vol. 286, 531-537
- **BHATTACHARJAE et al.** *Proc. Natl. Acad. Sci. USA,* 2001, vol. 98, 13790-13795
- **CHEN-HSIANG et al.** *Bioinformatics,* 2001, vol. 17 (1), S316-S322
- **RAMASWAMY et al.** *Proc. Natl. Acad. Sci. USA,* 2001, vol. 98, 15149-15154
- **MARTIN et al.** *Cancer Res.,* 2000, vol. 60, 2232-2238
- **WEST et al.** *Proc. Natl. Acad. Sci. USA,* 2001, vol. 98, 11462-11467
- **SORLIE et al.** *Proc. Natl. Acad. Sci. USA,* 2001, vol. 98, 10869-10874
- **YAN et al.** *Cancer Res.,* 2001, vol. 61, 8375-8380
- **PEROU et al.** *Nature,* 2000, vol. 406, 747-752
- **RASCHELLA GIUSEPPE et al.** Expression of B-myb in neuroblastoma tumors is a poor prognostic factor independent from MYCN amplification. *CANCER RESEARCH,* vol. 59 (14), 3365-3368
- **AUSUBEL et al.** Current Protocols in Molecular Biology. Wiley Interscience Publishers, 1995
- **SAMBROOK et al.** Molecular Cloning: A Laboratory Manual, New York. Cold Spring Harbor Press, 1989
- **SAMBROOK et al.** Molecular Cloning: A Laboratory Manual. 1989
- Oligonucleotide Synthesis. 1984
- Animal Cell Culture. 1987
- Methods in Enzymology. Academic Press, Inc
- Handbook of Experimental Immunology. Blackwell Science Inc, 1987
- Gene Transfer Vectors for Mammalian Cells. 1987
- Current Protocols in Molecular Biology. 1987
- PCR: The Polymerase Chain Reaction. 1994
- **PARKER ; BARNES.** *Methods in Molecular Biology,* 1999, vol. 106, 247-283
- **HOD.** *Biotechniques,* 1992, vol. 13, 852-854
- **WEIS et al.** *Trends in Genetics,* 1992, vol. 8, 263-264
- **AUSUBEL et al.** Current Protocols of Molecular Biology. John Wiley and Sons, 1997
- **RUPP ; LOCKER.** *Lab Invest.,* 1987, vol. 56, A67
- **DE ANDRÉS et al.** *BioTechniques,* 1995, vol. 18, 42044
- **HELD et al.** *Genome Research,* 1996, vol. 6, 986-994
- **T.E. GODFREY et al.** *J. Molec. Diagnostics,* 2000, vol. 2, 84-91
- **K. SPECHT et al.** *Am. J. Pathol.,* 2001, vol. 158, 419-29
- **KENT, W.J.** *Genome Res.,* 2002, vol. 12 (4), 656-64
- Primer3 on the WWW for general users and for biologist programmers. **STEVE ROZEN ; HELEN J. SKALETSKY.** Bioinformtics Methods and Protocols: Methods in Molecular Biology. Humana Press, 2000, 365-386
- General Concepts for PCR Primer Design. **DIEFFENBACH, C.W. et al.** PCR Primer, A Laboratory Manual. Cold Spring Harbor Laboratory Press, 1995, 133-155
- Optimization of PCRs. **INNIS ; GELFAND.** PCR Protocols, A Guide to Methods and Applications. CRC Press, 1994, 5-11
- **PLASTERER, T.N.** Primerselect: Primer and probe design. *Methods Mol. Biol.,* 1997, vol. 70, 520-527
- **SCHENA et al.** *Proc. Natl. Acad. Sci. USA,* 1996, vol. 93 (2), 106-149
- **VELCULESCU et al.** *Science,* 1995, vol. 270, 484-487
- **VELCULESCU et al.** *Cell,* 1997, vol. 88, 243-51
- **BRENNER et al.** *Nature Biotechnology,* 2000, vol. 18, 630-634
- **COX, D. R. ; OAKES, D.** Analysis of Survival Data. Chapman and Hall, 1984